(19) Europäisches Patentamt
European Patent Office
Office européen des brevets

(11) EP 3 431 484 A1

(12) # EUROPEAN PATENT APPLICATION

(43) Date of publication:
**23.01.2019 Bulletin 2019/04**

(21) Application number: **17182689.4**

(22) Date of filing: **21.07.2017**

(51) Int Cl.:
***C07H 21/02*** *(2006.01)* ***C07H 21/04*** *(2006.01)*
***G01N 33/52*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Ludwig-Maximilians-Universität München**
**80539 München (DE)**

(72) Inventors:
- **HOPFNER, Karl-Peter**
**81377 München (DE)**
- **ANDREEVA, Liudmila**
**81377 München (DE)**
- **DREXLER, David**
**81377 München (DE)**

(74) Representative: **Grünecker Patent- und Rechtsanwälte**
**PartG mbB**
**Leopoldstraße 4**
**80802 München (DE)**

(54) **A FLUORESCENT CYCLIC DINUCLEOTIDE AND ITS USE IN METHODS OF IDENTIFYING SUBSTANCES HAVING AN ABILITY TO MODULATE THE CGAS/STING PATHWAY**

(57) The present invention relates to a non-canonical cyclic di-nucleotide containing an asymmetric 2'-5' and 3'-5' phosphodiester linkage and its use in a method for identifying a substance having an ability to modulate cGAS activity or bind to STING. Furthermore, the cyclic dinucleotide is used in a method of identifying a substance having an ability to modulate the activity of a 2'-5' phosphodiesterase.

EP 3 431 484 A1

SEQUENCE LISTING

<110> Ludwig-Maximilians-Universität München

<120> A fluorescent cyclic dinucleotide and its use in methods of identifying substances having an ability to modulate the cGAS/STING pathway

<130> EP111308SZpau

<140> not yet assigned
<141> herewith

<160> 12

<170> PatentIn version 3.5

<210> 1
<211> 1140
<212> DNA
<213> Homo sapiens

<400> 1
atgccccact ccagcctgca tccatccatc ccgtgtccca ggggtcacgg ggcccagaag 60

gcagccttgg ttctgctgag tgcctgcctg gtgacccttt gggggctagg agagccacca 120

gagcacactc tccggtacct ggtgctccac ctagcctccc tgcagctggg actgctgtta 180

aacggggtct gcagcctggc tgaggagctg cgccacatcc actccaggta ccggggcagc 240

tactggagga ctgtgcgggc ctgcctgggc tgcccctcc gccgtggggc cctgttgctg 300

ctgtccatct atttctacta ctccctccca aatgcggtcg gcccgccctt cacttggatg 360

cttgccctcc tgggcctctc gcaggcactg aacatcctcc tgggcctcaa gggcctggcc 420

ccagctgaga tctctgcagt gtgtgaaaaa gggaatttca acgtggccca tgggctggca 480

tggtcatatt acatcggata tctgcggctg atcctgccag agctccaggc ccggattcga 540

acttacaatc agcattacaa caacctgcta cggggtgcag tgagccagcg gctgtatatt 600

ctcctcccat tggactgtgg ggtgcctgat aacctgagta tggctgaccc caacattcgc 660

ttcctggata aactgcccca gcagaccggt gaccatgctg gcatcaagga tcgggtttac 720

agcaacagca tctatgagct tctggagaac gggcagcggg cgggcacctg tgtcctggag 780

tacgccaccc ccttgcagac tttgtttgcc atgtcacaat acagtcaagc tggctttagc 840

cgggaggata ggcttgagca ggccaaactc ttctgccgga cacttgagga catcctggca 900

gatgcccctg agtctcagaa caactgccgc ctcattgcct accaggaacc tgcagatgac 960

agcagcttct cgctgtccca ggaggttctc cggcacctgc ggcaggagga aaaggaagag 1020

gttactgtgg gcagcttgaa gacctcagcg gtgcccagta cctccacgat gtcccaagag 1080

cctgagctcc tcatcagtgg aatggaaaag cccctccctc tccgcacgga tttctcttga 1140

<210> 2

<211> 379
<212> PRT
<213> Homo sapiens

<400> 2

```
Met Pro His Ser Ser Leu His Pro Ser Ile Pro Cys Pro Arg Gly His
1               5                   10                  15

Gly Ala Gln Lys Ala Ala Leu Val Leu Leu Ser Ala Cys Leu Val Thr
            20                  25                  30

Leu Trp Gly Leu Gly Glu Pro Pro Glu His Thr Leu Arg Tyr Leu Val
        35                  40                  45

Leu His Leu Ala Ser Leu Gln Leu Gly Leu Leu Leu Asn Gly Val Cys
    50                  55                  60

Ser Leu Ala Glu Glu Leu Arg His Ile His Ser Arg Tyr Arg Gly Ser
65                  70                  75                  80

Tyr Trp Arg Thr Val Arg Ala Cys Leu Gly Cys Pro Leu Arg Arg Gly
                85                  90                  95

Ala Leu Leu Leu Leu Ser Ile Tyr Phe Tyr Tyr Ser Leu Pro Asn Ala
            100                 105                 110

Val Gly Pro Pro Phe Thr Trp Met Leu Ala Leu Leu Gly Leu Ser Gln
        115                 120                 125

Ala Leu Asn Ile Leu Leu Gly Leu Lys Gly Leu Ala Pro Ala Glu Ile
    130                 135                 140

Ser Ala Val Cys Glu Lys Gly Asn Phe Asn Val Ala His Gly Leu Ala
145                 150                 155                 160

Trp Ser Tyr Tyr Ile Gly Tyr Leu Arg Leu Ile Leu Pro Glu Leu Gln
                165                 170                 175

Ala Arg Ile Arg Thr Tyr Asn Gln His Tyr Asn Asn Leu Leu Arg Gly
            180                 185                 190

Ala Val Ser Gln Arg Leu Tyr Ile Leu Leu Pro Leu Asp Cys Gly Val
        195                 200                 205

Pro Asp Asn Leu Ser Met Ala Asp Pro Asn Ile Arg Phe Leu Asp Lys
    210                 215                 220

Leu Pro Gln Gln Thr Gly Asp His Ala Gly Ile Lys Asp Arg Val Tyr
```

225 230 235 240

Ser Asn Ser Ile Tyr Glu Leu Leu Glu Asn Gly Gln Arg Ala Gly Thr
245 250 255

Cys Val Leu Glu Tyr Ala Thr Pro Leu Gln Thr Leu Phe Ala Met Ser
260 265 270

Gln Tyr Ser Gln Ala Gly Phe Ser Arg Glu Asp Arg Leu Glu Gln Ala
275 280 285

Lys Leu Phe Cys Arg Thr Leu Glu Asp Ile Leu Ala Asp Ala Pro Glu
290 295 300

Ser Gln Asn Asn Cys Arg Leu Ile Ala Tyr Gln Glu Pro Ala Asp Asp
305 310 315 320

Ser Ser Phe Ser Leu Ser Gln Glu Val Leu Arg His Leu Arg Gln Glu
325 330 335

Glu Lys Glu Glu Val Thr Val Gly Ser Leu Lys Thr Ser Ala Val Pro
340 345 350

Ser Thr Ser Thr Met Ser Gln Glu Pro Glu Leu Leu Ile Ser Gly Met
355 360 365

Glu Lys Pro Leu Pro Leu Arg Thr Asp Phe Ser
370 375

<210> 3
<211> 1140
<212> DNA
<213> Homo sapiens

<400> 3
atgccccact ccagcctgca tccatccatc ccgtgtccca ggggtcacgg ggcccagaag 60

gcagccttgg ttctgctgag tgcctgcctg gtgacccttt gggggctagg agagccacca 120

gagcacactc tccggtacct ggtgctccac ctagcctccc tgcagctggg actgctgtta 180

aacggggtct gcagcctggc tgaggagctg cgccacatcc actccaggta ccggggcagc 240

tactggagga ctgtgcgggc ctgcctgggc tgcccccctcc gccgtggggc cctgttgctg 300

ctgtccatct atttctacta ctccctccca aatgcggtcg gcccgccctt cacttggatg 360

cttgccctcc tgggcctctc gcaggcactg aacatcctcc tgggcctcaa gggcctggcc 420

ccagctgaga tctctgcagt gtgtgaaaaa gggaatttca acgtggccca tgggctggca 480

tggtcatatt acatcggata tctgcggctg atcctgccag agctccaggc ccggattcga 540

```
acttacaatc agcattacaa caacctgcta cggggtgcag tgagccagcg gctgtatatt      600

ctcctcccat tggactgtgg ggtgcctgat aacctgagta tggctgaccc caacattcac      660

ttcctggata aactgcccca gcagaccggt gaccgtgctg gcatcaagga tcgggtttac      720

agcaacagca tctatgagct tctggagaac gggcagcggg cgggcacctg tgtcctggag      780

tacgccaccc ccttgcagac tttgtttgcc atgtcacaat acagtcaagc tggctttagc      840

cgggaggata ggcttgagca ggccaaactc ttctgccgga cacttgagga catcctggca      900

gatgcccctg agtctcagaa caactgccgc ctcattgcct accaggaacc tgcagatgac      960

agcagcttct cgctgtccca ggaggttctc cggcacctgc ggcaggagga aaaggaagag     1020

gttactgtgg gcagcttgaa gacctcagcg gtgcccagta cctccacgat gtcccaagag     1080

cctgagctcc tcatcagtgg aatggaaaag cccctccctc tccgcacgga tttctcttga     1140


<210>  4
<211>  379
<212>  PRT
<213>  Homo sapiens

<400>  4

Met Pro His Ser Ser Leu His Pro Ser Ile Pro Cys Pro Arg Gly His
1               5                   10                  15

Gly Ala Gln Lys Ala Ala Leu Val Leu Leu Ser Ala Cys Leu Val Thr
            20                  25                  30

Leu Trp Gly Leu Gly Glu Pro Pro Glu His Thr Leu Arg Tyr Leu Val
        35                  40                  45

Leu His Leu Ala Ser Leu Gln Leu Gly Leu Leu Leu Asn Gly Val Cys
    50                  55                  60

Ser Leu Ala Glu Glu Leu Arg His Ile His Ser Arg Tyr Arg Gly Ser
65                  70                  75                  80

Tyr Trp Arg Thr Val Arg Ala Cys Leu Gly Cys Pro Leu Arg Arg Gly
                85                  90                  95

Ala Leu Leu Leu Leu Ser Ile Tyr Phe Tyr Tyr Ser Leu Pro Asn Ala
            100                 105                 110

Val Gly Pro Pro Phe Thr Trp Met Leu Ala Leu Leu Gly Leu Ser Gln
        115                 120                 125

Ala Leu Asn Ile Leu Leu Gly Leu Lys Gly Leu Ala Pro Ala Glu Ile
    130                 135                 140
```

```
Ser Ala Val Cys Glu Lys Gly Asn Phe Asn Val Ala His Gly Leu Ala
145                 150                 155                 160

Trp Ser Tyr Tyr Ile Gly Tyr Leu Arg Leu Ile Leu Pro Glu Leu Gln
                165                 170                 175

Ala Arg Ile Arg Thr Tyr Asn Gln His Tyr Asn Asn Leu Leu Arg Gly
            180                 185                 190

Ala Val Ser Gln Arg Leu Tyr Ile Leu Leu Pro Leu Asp Cys Gly Val
        195                 200                 205

Pro Asp Asn Leu Ser Met Ala Asp Pro Asn Ile His Phe Leu Asp Lys
    210                 215                 220

Leu Pro Gln Gln Thr Gly Asp Arg Ala Gly Ile Lys Asp Arg Val Tyr
225                 230                 235                 240

Ser Asn Ser Ile Tyr Glu Leu Leu Glu Asn Gly Gln Arg Ala Gly Thr
                245                 250                 255

Cys Val Leu Glu Tyr Ala Thr Pro Leu Gln Thr Leu Phe Ala Met Ser
            260                 265                 270

Gln Tyr Ser Gln Ala Gly Phe Ser Arg Glu Asp Arg Leu Glu Gln Ala
        275                 280                 285

Lys Leu Phe Cys Arg Thr Leu Glu Asp Ile Leu Ala Asp Ala Pro Glu
    290                 295                 300

Ser Gln Asn Asn Cys Arg Leu Ile Ala Tyr Gln Glu Pro Ala Asp Asp
305                 310                 315                 320

Ser Ser Phe Ser Leu Ser Gln Glu Val Leu Arg His Leu Arg Gln Glu
                325                 330                 335

Glu Lys Glu Glu Val Thr Val Gly Ser Leu Lys Thr Ser Ala Val Pro
            340                 345                 350

Ser Thr Ser Thr Met Ser Gln Glu Pro Glu Leu Leu Ile Ser Gly Met
        355                 360                 365

Glu Lys Pro Leu Pro Leu Arg Thr Asp Phe Ser
    370                 375
```

<210> 5
<211> 618

<212> DNA
<213> Homo sapiens

<400> 5
ctggccccag ctgagatctc tgcagtgtgt gaaaaaggga atttcaacgt ggcccatggg 60

ctggcatggt catattacat cggatatctg cggctgatcc tgccagagct ccaggcccgg 120

attcgaactt acaatcagca ttacaacaac ctgctacggg gtgcagtgag ccagcggctg 180

tatattctcc tcccattgga ctgtggggtg cctgataacc tgagtatggc tgaccccaac 240

attcgcttcc tggataaact gccccagcag accggtgacc atgctggcat caaggatcgg 300

gtttacagca acagcatcta tgagcttctg gagaacgggc agcgggcggg cacctgtgtc 360

ctggagtacg ccaccccctt gcagactttg tttgccatgt cacaatacag tcaagctggc 420

tttagccggg aggataggct tgagcaggcc aaactcttct gccggacact tgaggacatc 480

ctggcagatg cccctgagtc tcagaacaac tgccgcctca ttgcctacca ggaacctgca 540

gatgacagca gcttctcgct gtcccaggag gttctccggc acctgcggca ggaggaaaag 600

gaagaggtta ctgtgggc 618

<210> 6
<211> 206
<212> PRT
<213> Homo sapiens

<400> 6

Leu Ala Pro Ala Glu Ile Ser Ala Val Cys Glu Lys Gly Asn Phe Asn
1 5 10 15

Val Ala His Gly Leu Ala Trp Ser Tyr Tyr Ile Gly Tyr Leu Arg Leu
20 25 30

Ile Leu Pro Glu Leu Gln Ala Arg Ile Arg Thr Tyr Asn Gln His Tyr
35 40 45

Asn Asn Leu Leu Arg Gly Ala Val Ser Gln Arg Leu Tyr Ile Leu Leu
50 55 60

Pro Leu Asp Cys Gly Val Pro Asp Asn Leu Ser Met Ala Asp Pro Asn
65 70 75 80

Ile Arg Phe Leu Asp Lys Leu Pro Gln Gln Thr Gly Asp His Ala Gly
85 90 95

Ile Lys Asp Arg Val Tyr Ser Asn Ser Ile Tyr Glu Leu Leu Glu Asn
100 105 110

Gly Gln Arg Ala Gly Thr Cys Val Leu Glu Tyr Ala Thr Pro Leu Gln

115 120 125

Thr Leu Phe Ala Met Ser Gln Tyr Ser Gln Ala Gly Phe Ser Arg Glu
130 135 140

Asp Arg Leu Glu Gln Ala Lys Leu Phe Cys Arg Thr Leu Glu Asp Ile
145 150 155 160

Leu Ala Asp Ala Pro Glu Ser Gln Asn Asn Cys Arg Leu Ile Ala Tyr
165 170 175

Gln Glu Pro Ala Asp Asp Ser Ser Phe Ser Leu Ser Gln Glu Val Leu
180 185 190

Arg His Leu Arg Gln Glu Glu Lys Glu Glu Val Thr Val Gly
195 200 205

<210> 7
<211> 618
<212> DNA
<213> Homo sapiens

<400> 7
ctggccccag ctgagatctc tgcagtgtgt gaaaaaggga atttcaacgt ggcccatggg 60

ctggcatggt catattacat cggatatctg cggctgatcc tgccagagct ccaggcccgg 120

attcgaactt acaatcagca ttacaacaac ctgctacggg gtgcagtgag ccagcggctg 180

tatattctcc tcccattgga ctgtggggtg cctgataacc tgagtatggc tgaccccaac 240

attcacttcc tggataaact gccccagcag accggtgacc gtgctggcat caaggatcgg 300

gtttacagca acagcatcta tgagcttctg gagaacgggc agcgggcggg cacctgtgtc 360

ctggagtacg ccacccccct gcagactttg tttgccatgt cacaatacag tcaagctggc 420

tttagccggg aggataggct tgagcaggcc aaactcttct gccggacact tgaggacatc 480

ctggcagatg cccctgagtc tcagaacaac tgccgcctca ttgcctacca ggaacctgca 540

gatgacagca gcttctcgct gtcccaggag gttctccggc acctgcggca ggaggaaaag 600

gaagaggtta ctgtgggc 618

<210> 8
<211> 206
<212> PRT
<213> Homo sapiens

<400> 8

Leu Ala Pro Ala Glu Ile Ser Ala Val Cys Glu Lys Gly Asn Phe Asn
1 5 10 15

```
Val Ala His Gly Leu Ala Trp Ser Tyr Tyr Ile Gly Tyr Leu Arg Leu
            20                  25                  30

Ile Leu Pro Glu Leu Gln Ala Arg Ile Arg Thr Tyr Asn Gln His Tyr
        35                  40                  45

Asn Asn Leu Leu Arg Gly Ala Val Ser Gln Arg Leu Tyr Ile Leu Leu
    50                  55                  60

Pro Leu Asp Cys Gly Val Pro Asp Asn Leu Ser Met Ala Asp Pro Asn
65                  70                  75                  80

Ile His Phe Leu Asp Lys Leu Pro Gln Gln Thr Gly Asp Arg Ala Gly
                85                  90                  95

Ile Lys Asp Arg Val Tyr Ser Asn Ser Ile Tyr Glu Leu Leu Glu Asn
            100                 105                 110

Gly Gln Arg Ala Gly Thr Cys Val Leu Glu Tyr Ala Thr Pro Leu Gln
        115                 120                 125

Thr Leu Phe Ala Met Ser Gln Tyr Ser Gln Ala Gly Phe Ser Arg Glu
    130                 135                 140

Asp Arg Leu Glu Gln Ala Lys Leu Phe Cys Arg Thr Leu Glu Asp Ile
145                 150                 155                 160

Leu Ala Asp Ala Pro Glu Ser Gln Asn Asn Cys Arg Leu Ile Ala Tyr
                165                 170                 175

Gln Glu Pro Ala Asp Asp Ser Ser Phe Ser Leu Ser Gln Glu Val Leu
            180                 185                 190

Arg His Leu Arg Gln Glu Glu Lys Glu Glu Val Thr Val Gly
        195                 200                 205


<210>  9
<211>  1569
<212>  DNA
<213>  Homo sapiens

<400>  9
atgcagcctt ggcacggaaa ggccatgcag agagcttccg aggccggagc cactgccccc        60

aaggcttccg cacggaatgc caggggcgcc ccgatggatc ccaccgagtc tccggctgcc       120

cccgaggccg ccctgcctaa ggcgggaaag ttcggccccg ccaggaagtc gggatcccgg       180

cagaaaaaga gcgccccgga cacccaggag aggccgcccg tccgcgcaac tggggcccgc       240

gccaaaaagg cccctcagcg cgcccaggac acgcagccgt ctgacgccac cagcgcccct       300
```

```
ggggcagagg ggctggagcc tcctgcggct cgggagccgg ctctttccag ggctggttct      360

tgccgccaga ggggcgcgcg ctgctccacg aagccaagac ctccgcccgg gccctgggac      420

gtgcccagcc ccggcctgcc ggtctcggcc cccattctcg tacggaggga tgcggcgcct      480

ggggcctcga agctccgggc ggttttggag aagttgaagc tcagccgcga tgatatctcc      540

acggcggcgg ggatggtgaa aggggttgtg gaccacctgc tgctcagact gaagtgcgac      600

tccgcgttca gaggcgtcgg gctgctgaac accgggagct actatgagca cgtgaagatt      660

tctgcaccta atgaatttga tgtcatgttt aaactggaag tccccagaat tcaactagaa      720

gaatattcca acactcgtgc atattacttt gtgaaattta aaagaaatcc gaaagaaaat      780

cctctgagtc agtttttaga aggtgaaata ttatcagctt ctaagatgct gtcaaagttt      840

aggaaaatca ttaaggaaga aattaacgac attaaagata cagatgtcat catgaagagg      900

aaaagaggag ggagccctgc tgtaacactt cttattagtg aaaaaatatc tgtggatata      960

accctggctt tggaatcaaa aagtagctgg cctgctagca cccaagaagg cctgcgcatt     1020

caaaactggc tttcagcaaa agttaggaag caactacgac taaagccatt ttaccttgta     1080

cccaagcatg caaaggaagg aaatggtttc caagaagaaa catggcggct atccttctct     1140

cacatcgaaa aggaaatttt gaacaatcat ggaaaatcta aaacgtgctg tgaaaacaaa     1200

gaagagaaat gttgcaggaa agattgttta aaactaatga aataccttttt agaacagctg    1260

aaagaaaggt ttaaagacaa aaaacatctg gataaattct cttcttatca tgtgaaaact     1320

gccttctttc acgtatgtac ccagaaccct caagacagtc agtgggaccg caaagacctg     1380

ggcctctgct ttgataactg cgtgacatac tttcttcagt gcctcaggac agaaaaactt     1440

gagaattatt ttattcctga attcaatcta ttctctagca acttaattga caaaagaagt     1500

aaagaatttc tgacaaagca aattgaatat gaaagaaaca atgagtttcc agtttttgat     1560

gaattttga                                                             1569


<210>  10
<211>  522
<212>  PRT
<213>  Homo sapiens

<400>  10

Met Gln Pro Trp His Gly Lys Ala Met Gln Arg Ala Ser Glu Ala Gly
1               5                   10                  15


Ala Thr Ala Pro Lys Ala Ser Ala Arg Asn Ala Arg Gly Ala Pro Met
            20                  25                  30


Asp Pro Thr Glu Ser Pro Ala Ala Pro Glu Ala Ala Leu Pro Lys Ala
        35                  40                  45
```

```
Gly Lys Phe Gly Pro Ala Arg Lys Ser Gly Ser Arg Gln Lys Lys Ser
    50                  55                  60

Ala Pro Asp Thr Gln Glu Arg Pro Pro Val Arg Ala Thr Gly Ala Arg
65                  70                  75                  80

Ala Lys Lys Ala Pro Gln Arg Ala Gln Asp Thr Gln Pro Ser Asp Ala
                85                  90                  95

Thr Ser Ala Pro Gly Ala Glu Gly Leu Glu Pro Pro Ala Ala Arg Glu
            100                 105                 110

Pro Ala Leu Ser Arg Ala Gly Ser Cys Arg Gln Arg Gly Ala Arg Cys
        115                 120                 125

Ser Thr Lys Pro Arg Pro Pro Pro Gly Pro Trp Asp Val Pro Ser Pro
    130                 135                 140

Gly Leu Pro Val Ser Ala Pro Ile Leu Val Arg Arg Asp Ala Ala Pro
145                 150                 155                 160

Gly Ala Ser Lys Leu Arg Ala Val Leu Glu Lys Leu Lys Leu Ser Arg
                165                 170                 175

Asp Asp Ile Ser Thr Ala Ala Gly Met Val Lys Gly Val Val Asp His
            180                 185                 190

Leu Leu Leu Arg Leu Lys Cys Asp Ser Ala Phe Arg Gly Val Gly Leu
        195                 200                 205

Leu Asn Thr Gly Ser Tyr Tyr Glu His Val Lys Ile Ser Ala Pro Asn
    210                 215                 220

Glu Phe Asp Val Met Phe Lys Leu Glu Val Pro Arg Ile Gln Leu Glu
225                 230                 235                 240

Glu Tyr Ser Asn Thr Arg Ala Tyr Tyr Phe Val Lys Phe Lys Arg Asn
                245                 250                 255

Pro Lys Glu Asn Pro Leu Ser Gln Phe Leu Glu Gly Glu Ile Leu Ser
            260                 265                 270

Ala Ser Lys Met Leu Ser Lys Phe Arg Lys Ile Ile Lys Glu Glu Ile
        275                 280                 285

Asn Asp Ile Lys Asp Thr Asp Val Ile Met Lys Arg Lys Arg Gly Gly
```

```
    290                 295                 300

Ser Pro Ala Val Thr Leu Leu Ile Ser Glu Lys Ile Ser Val Asp Ile
305                 310                 315                 320

Thr Leu Ala Leu Glu Ser Lys Ser Ser Trp Pro Ala Ser Thr Gln Glu
                325                 330                 335

Gly Leu Arg Ile Gln Asn Trp Leu Ser Ala Lys Val Arg Lys Gln Leu
            340                 345                 350

Arg Leu Lys Pro Phe Tyr Leu Val Pro Lys His Ala Lys Glu Gly Asn
        355                 360                 365

Gly Phe Gln Glu Glu Thr Trp Arg Leu Ser Phe Ser His Ile Glu Lys
    370                 375                 380

Glu Ile Leu Asn Asn His Gly Lys Ser Lys Thr Cys Cys Glu Asn Lys
385                 390                 395                 400

Glu Glu Lys Cys Cys Arg Lys Asp Cys Leu Lys Leu Met Lys Tyr Leu
                405                 410                 415

Leu Glu Gln Leu Lys Glu Arg Phe Lys Asp Lys Lys His Leu Asp Lys
            420                 425                 430

Phe Ser Ser Tyr His Val Lys Thr Ala Phe Phe His Val Cys Thr Gln
        435                 440                 445

Asn Pro Gln Asp Ser Gln Trp Asp Arg Lys Asp Leu Gly Leu Cys Phe
    450                 455                 460

Asp Asn Cys Val Thr Tyr Phe Leu Gln Cys Leu Arg Thr Glu Lys Leu
465                 470                 475                 480

Glu Asn Tyr Phe Ile Pro Glu Phe Asn Leu Phe Ser Ser Asn Leu Ile
                485                 490                 495

Asp Lys Arg Ser Lys Glu Phe Leu Thr Lys Gln Ile Glu Tyr Glu Arg
            500                 505                 510

Asn Asn Glu Phe Pro Val Phe Asp Glu Phe
        515                 520
```

<210>  11
<211>  1107
<212>  DNA
<213>  Homo sapiens

<400> 11
cggagggatg cggcgcctgg ggcctcgaag ctccgggcgg ttttggagaa gttgaagctc 60

agccgcgatg atatctccac ggcggcgggg atggtgaaag gggttgtgga ccacctgctg 120

ctcagactga agtgcgactc cgcgttcaga ggcgtcgggc tgctgaacac cgggagctac 180

tatgagcacg tgaagatttc tgcacctaat gaatttgatg tcatgtttaa actggaagtc 240

cccagaattc aactagaaga atattccaac actcgtgcat attactttgt gaaatttaaa 300

agaaatccga aagaaaatcc tctgagtcag tttttagaag gtgaaatatt atcagcttct 360

aagatgctgt caaagtttag gaaaatcatt aaggaagaaa ttaacgacat taaagataca 420

gatgtcatca tgaagaggaa aagaggaggg agccctgctg taacacttct tattagtgaa 480

aaaatatctg tggatataac cctggctttg gaatcaaaaa gtagctggcc tgctagcacc 540

caagaaggcc tgcgcattca aaactggctt tcagcaaaag ttaggaagca actacgacta 600

aagccatttt accttgtacc caagcatgca aaggaaggaa atggtttcca agaagaaaca 660

tggcggctat ccttctctca catcgaaaag gaaattttga acaatcatgg aaaatctaaa 720

acgtgctgtg aaaacaaaga agagaaatgt tgcaggaaag attgtttaaa actaatgaaa 780

tacctttttag aacagctgaa agaaaggttt aaagacaaaa aacatctgga taaattctct 840

tcttatcatg tgaaaactgc cttctttcac gtatgtaccc agaaccctca agacagtcag 900

tgggaccgca aagacctggg cctctgcttt gataactgcg tgacatactt tcttcagtgc 960

ctcaggacag aaaaacttga gaattatttt attcctgaat tcaatctatt ctctagcaac 1020

ttaattgaca aaagaagtaa agaatttctg acaaagcaaa ttgaatatga aagaaacaat 1080

gagtttccag tttttgatga attttga 1107

<210> 12
<211> 368
<212> PRT
<213> Homo sapiens

<400> 12
Arg Arg Asp Ala Ala Pro Gly Ala Ser Lys Leu Arg Ala Val Leu Glu
1 5 10 15

Lys Leu Lys Leu Ser Arg Asp Asp Ile Ser Thr Ala Ala Gly Met Val
20 25 30

Lys Gly Val Val Asp His Leu Leu Leu Arg Leu Lys Cys Asp Ser Ala
35 40 45

Phe Arg Gly Val Gly Leu Leu Asn Thr Gly Ser Tyr Tyr Glu His Val
50 55 60

Lys Ile Ser Ala Pro Asn Glu Phe Asp Val Met Phe Lys Leu Glu Val
65                  70                  75                  80

Pro Arg Ile Gln Leu Glu Glu Tyr Ser Asn Thr Arg Ala Tyr Tyr Phe
                85                  90                  95

Val Lys Phe Lys Arg Asn Pro Lys Glu Asn Pro Leu Ser Gln Phe Leu
            100                 105                 110

Glu Gly Glu Ile Leu Ser Ala Ser Lys Met Leu Ser Lys Phe Arg Lys
        115                 120                 125

Ile Ile Lys Glu Glu Ile Asn Asp Ile Lys Asp Thr Asp Val Ile Met
    130                 135                 140

Lys Arg Lys Arg Gly Gly Ser Pro Ala Val Thr Leu Leu Ile Ser Glu
145                 150                 155                 160

Lys Ile Ser Val Asp Ile Thr Leu Ala Leu Glu Ser Lys Ser Ser Trp
                165                 170                 175

Pro Ala Ser Thr Gln Glu Gly Leu Arg Ile Gln Asn Trp Leu Ser Ala
            180                 185                 190

Lys Val Arg Lys Gln Leu Arg Leu Lys Pro Phe Tyr Leu Val Pro Lys
        195                 200                 205

His Ala Lys Glu Gly Asn Gly Phe Gln Glu Glu Thr Trp Arg Leu Ser
    210                 215                 220

Phe Ser His Ile Glu Lys Glu Ile Leu Asn Asn His Gly Lys Ser Lys
225                 230                 235                 240

Thr Cys Cys Glu Asn Lys Glu Glu Lys Cys Cys Arg Lys Asp Cys Leu
                245                 250                 255

Lys Leu Met Lys Tyr Leu Leu Glu Gln Leu Lys Glu Arg Phe Lys Asp
            260                 265                 270

Lys Lys His Leu Asp Lys Phe Ser Ser Tyr His Val Lys Thr Ala Phe
        275                 280                 285

Phe His Val Cys Thr Gln Asn Pro Gln Asp Ser Gln Trp Asp Arg Lys
    290                 295                 300

Asp Leu Gly Leu Cys Phe Asp Asn Cys Val Thr Tyr Phe Leu Gln Cys
305                 310                 315                 320

```
Leu Arg Thr Glu Lys Leu Glu Asn Tyr Phe Ile Pro Glu Phe Asn Leu
                325                 330                 335

Phe Ser Ser Asn Leu Ile Asp Lys Arg Ser Lys Glu Phe Leu Thr Lys
            340                 345                 350

Gln Ile Glu Tyr Glu Arg Asn Asn Glu Phe Pro Val Phe Asp Glu Phe
        355                 360                 365
```

## Description

### Field of the invention

The present invention relates to a non-canonical cyclic di-nucleotide containing an asymmetric 2'-5' and 3'-5' phosphodiester linkage and its use in a method for identifying a substance having an ability to modulate cGAS activity or bind to STING. Furthermore, the cyclic dinucleotide is used in a method of identifying a substance having an ability to modulate the activity of a 2'-5' phosphodiesterase.

### Background of the invention

The innate immune system relies on germline-encoded recognition receptors (PRRs) to survey the extracellular, endosomal, and cytosolic milieu for signs of pathogen invasion or cellular damage. Following ligand binding, PRRs trigger downstream signaling to elicit innate immune responses and to activate the adaptive immune system. Since nucleic acids are central to the replication and propagation of most if not all pathogens, the detection of aberrant DNA and RNA has evolved as a fundamental mechanism of host defense.

DNA is usually confined within the nucleus and mitochondria of eukaryotic cells. The presence of cytosolic DNA either through infections or cellular damage triggers robust immune responses including type I interferon induction. The cyclic GMP-AMP synthase (cGAS) is a key sensor required for DNA recognition in the cytosol. Beside endogenous DNA, cGAS senses DNA from cytosolic bacteria, HIV (cDNA) and herpes viruses. In its inactive state cGAS exists in a monomeric apo-form. Upon recognition of cytosolic DNA, cGAS undergoes a conformational change resulting in the formation of an active $cGAS_2$:$DNA_2$ tetramer. Activated cGAS synthesizes cyclic dinucleotides comprising a conventional 3'-5' phosphodiester linkage and an unconventional 2'-5' phosphodiester linkage between a first nucleoside monophosphate and a second nucleoside monophosphate (2'3' cyclic dinucleotide CDNs). These CDNs serve as second messengers which then bind to the transmembrane adaptor protein stimulator of interferon genes (STING) associated with the endoplasmic reticulum. Upon binding to STING the adapter protein is activated, interacts with TBK 1 (TANK-binding kinase 1) and traffics through the Golgi to perinuclear endosomal compartments, where TBK 1 phosphorylates IRF 3 and IRF 7 to trigger type I interferon production.

While under normal conditions DNA sensing by the cGAS/STING axis protects the organism from harmful infections by DNA viruses, retroviruses and bacteria, unsolicited self-DNA sensing can result in autoimmune diseases such as systemic lupus erythematosus (SLE) and Aicardi-Goutières syndrome (AGS). AGS is a rare but generally fatal childhood inflammatory condition with neurological dysfunction which is caused by loss-of-function mutations in nucleic acid metabolizing enzymes resulting in accumulation of endogenous DNA in the cytosol.

Many genes have been identified as being involved in the development of autoimmune diseases. For instance, TREX1 encodes a 3'-5' DNA exonuclease, and it has been found that a loss of function mutation in this gene results in AGS and SLE (Crow, Hayward et al. "Mutations in the gene encoding the 3[prime]-5[prime] DNA exonuclease TREX1 cause Aicardi-Goutieres syndrome at the AGS1 locus". Nat Genet 38, 917-920, 2006; Lee-Kirsch, Gong et al. "Mutations in the gene encoding the 3[prime]-5[prime] DNA exonuclease TREX1 are associated with systemic lupus erythematosus". Nat Genet 39, 1065-1067, 2007). Interestingly, crossing TREX1-deficient mice with mice lacking either STING or the IFN$\alpha$/$\beta$ receptor resulted in a rescue of the autoinflammatory phenotypes (Gall, Treuting et al. "Autoimmunity Initiates in Nonhematopoietic Cells and Progresses via Lymphocytes in an Interferon-Dependent Autoimmune Disease". Immunity 36, 120-131, 2012; Ahn, Ruiz et al. "Intrinsic Self-DNA Triggers Inflammatory Disease Dependent on STING". The Journal of Immunology 2014), placing STING signaling central to these interferonopathies. Mutations in RNASEH2A, RNASH2B and RNASEH2C have also been linked to AGS (Crow, Hayward et al. "Mutations in the gene encoding the 3[prime]-5[prime] DNA exonuclease TREX1 cause Aicardi-Goutieres syndrome at the AGS1 locus". Nat Genet 38, 917-920, 2006; Crow, Chase et al. "Characterization of Human Disease Phenotypes Associated with Mutations in TREX1, RNASEH2A, RNASEH2B, RNASEH2C, SAMHD1, ADAR, and IFIH1". American journal of medical genetics. Part A 0, 296-312, 2015). The proteins encoded by these genes form the RNase H2 complex and function to degrade cellular RNA/DNA hybrids. In a mouse model of the disease due to a homozygous A174T knock-in-mutation, the cGAS/STING pathway was central to the autoinflammatory pathology, since the loss of STING in these mice reduced levels of inflammatory cytokines. Taken together, there is increasing evidence that STING plays a central role in autoimmune interferonopathies.

Several studies indicate that cGAS activation by cytosolic self-DNA is also responsible for the lethal phenotypes associated with SLE and ALS (Gao, Li et al. "Activation of cyclic GMP-AMP synthase by self-DNA causes autoimmune diseases". Proceedings of the National Academy of Sciences 112, E5699-E5705, 2015). Overall, these studies provide evidence that the inhibition of the cGAS/STING axis may be an effective therapeutic tool for the treatment of autoimmune diseases such as AGS and SLE.

Chronic inflammatory signaling may not only initiate autoinflammatory diseases but can also contribute to the

development of cancer, probably through cytokines, chemokines and growth factors that stimulate cellular proliferation and survival, as well as by promoting angiogenesis (Nowarski, Gagliani et al. "Innate Immune Cells in Inflammation and Cancer". Cancer Immunology Research 1, 77-84, 2013). Adaptive T cell responses are important for the control and eradication of tumor cells. Numerous immunotherapeutic strategies involving stimulating the adaptive immune response against cancers through checkpoint blockade are presently under evaluation in clinical trials. It has been shown that type I interferon production is involved in tumor antigen specific T cell activation and that the cGAS/STING pathway plays a key role in stimulating adaptive T cell responses against tumor cells (Woo, Fuertes et al. "STING-Dependent Cytosolic DNA Sensing Mediates Innate Immune Recognition of Immunogenic Tumors". Immunity 41, 830-842, 2014). Overall, the targeted inhibition or activation of the cGAS/STING pathway by small molecule compounds or therapeutic nucleic acids provide a promising tool for the pharmaceutical industry to develop strategies for the treatment of cancer, inflammatory diseases, and autoimmune disorders. In view of the numerous promising therapeutic approaches to modulate the cGAS/STING pathway intensive efforts are currently underway to identify small molecule compounds which are capable to modulate and fine-tune the innate immune system via the cGAS/STING pathway. However, large-scale screening assays for the identification of such modulators are currently hampered by the general lack of sensitive and scalable assays that allow a direct readout of the activity. Measuring the activity of cGAS is usually done via thin-layer chromatography of the produced 2'3'CDNs, using radioactive nucleoside triphosphates as substrates, which is impractical in high-throughput assays.

Alternatively, 2'3'CDN can be directly detected by mass spectrometry. The latter approach is sensitive and to some extent quantitative, however, it is difficult to measure reactions in parallel at high-throughput or at different time points to analyze steady-state rates. One approach of detecting CDNs is described by Roembke et al. (Roembke, Zhou et al. "A cyclic dinucleotide containing 2-aminopurine is a general fluorescent sensor for c-di-GMP and 3',3'-cGAMP". Molecular BioSystems 10, 1568-1575, 2014). In this article the authors suggest using the properties of fluorescent 3'3'-cGAMP analogue - 3'3'-cG(d2AP)MP. The method is based on $Mn^{2+}$-induced 3'3'-cG(d2AP)MP association with c-diGMP or 3'3'-cGAMP resulting in 3'3'-cG(d2AP)MP quenching. However, the method failed to detect 2'3'-cGAMP and c-di-AMP due to the lack of its association with 3'3'-cG(d2AP)MP. Hence, direct determination of human STING activating CDNs produced by cGAS was not successful with this assay. Furthermore, there is currently no robust and simple-to-implement high-throughput assay that directly measures ligand binding to STING in a quantitative manner, because STING is a receptor protein and not an enzyme. Very elaborate FRET 3'3'-cG(d2AP)MP (Roembke, Zhou et al. "A cyclic dinucleotide containing 2-aminopurine is a general fluorescent sensor for c-di-GMP and 3',3'-cGAMP". Molecular BioSystems 10, 1568-1575, 2014) methods have been proposed to measure the conformational change of STING, but these require very specialized equipment and also require modification of STING itself.

In view of all of the above, there is still a need for a sensitive and scalable biochemical assay that allows a direct readout of cGAS/STING activity, thereby providing a tool for identifying, on industrial scale, small molecule compounds that could modulate the cGAS/STING pathway and which could therefore be promising candidates in anti-inflammatory, anti-autoimmune, and anti-cancer therapy. The object of the present invention is to provide such means.

## Summary of the invention

The present invention relates to a fluorescent cyclic dinucleotide and its preparation. Furthermore, the present invention relates to a method for measuring cGAS activity. The present invention further relates to a method of identifying a substance having an ability to modulate cGAS activity. The present invention also relates to a method of identifying a substance having an ability to bind to STING. The present invention further relates to a method of indentifying a substance having an ability to modulate the activity of a 2'-5' phosphodiesterase. Finally, the present invention relates to a fluorescent cyclic dinucleotide and its use to label cGAS and STING and/or to identify substances having an ability to bind to STING and to complexes of the fluorescent cyclic dinucleotide with cGAS and STING.

According to one aspect, the invention relates to a cyclic dinucleotide comprising a 3'-5' phosphodiester linkage and a 2'-5' phosphodiester linkage between a first nucleoside monophosphate and a second nucleoside monophosphate, wherein at least one of the first and second nucleoside monophosphates is a fluorescent nucleoside monophosphate.

In a further aspect, the invention relates to a method of measuring cGAS activity, wherein the method comprises the steps of: (i) providing an aqueous solution comprising cGAS, cGAS-activating nucleic acid, a first nucleoside triphosphate, a second nucleoside triphosphate, and one or more divalent cation, wherein at least one of the first and second nucleoside triphosphates is a fluorescent nucleoside triphosphate, and wherein one of the first and second nucleoside triphosphates has a free 2' hydroxyl group and the other one has a free 3' hydroxyl group; and (ii) measuring the change of the fluorescence signal of the aqueous solution over time.

In another aspect, the invention provides a method of identifying a substance having an ability to modulate the activity of cyclic GMP-AMP synthase (cGAS), wherein the method comprises the steps of: (i) providing an aqueous solution comprising cGAS, cGAS-activating nucleic acid, a first nucleoside triphosphate, a second nucleoside triphosphate, and one or more divalent cation, wherein at least one of the first and second nucleoside triphosphates is a

fluorescent nucleoside triphosphate, and wherein one of the first and second nucleoside triphosphates has a free 2' hydroxyl group and the other one has a free 3' hydroxyl group; (ii) measuring the fluorescence signal of the aqueous solution; (iii) repeating steps (i) and (ii), wherein upon said repetition the aqueous solution further comprises the substance, and wherein the fluorescence signal is measured under identical or substantially identical conditions following the provision of the respective aqueous solution; and (iv) comparing the fluorescence signal measured in the presence of the substance with the fluorescence signal measured in the absence of the substance; wherein a measured fluorescence signal which is higher in the presence of the substance than in the absence of the substance indicates that the substance is a cGAS antagonist, while a measured fluorescence signal which is lower in the presence of the substance than in the absence of the substance indicates that the substance is a cGAS agonist.

A further aspect of the invention relates to a method of preparing a cyclic dinucleotide, wherein the method comprises the steps of (i) providing an aqueous solution comprising cGAS, cGAS-activating nucleic acid, a first nucleoside triphosphate, a second nucleoside triphosphate, and one or more divalent cation, wherein at least one of the first and second nucleoside triphosphates is a fluorescent nucleoside triphosphate, and wherein one of the first and second nucleoside triphosphates has a free 2' hydroxyl group and the other one has a free 3' hydroxyl group, thereby preparing the cyclic dinucleotide; and, optionally, (ii) purifying the cyclic dicnucleotide.

Another aspect of the invention relates to a method of identifying a substance having an ability to bind to stimulator of interferon genes (STING), wherein the method comprises the steps of (i) providing an aqueous solution comprising a cyclic dinucleotide and STING, wherein the cyclic dinucleotide comprises a 3'-5' phosphodiester linkage and a 2'-5' phosphodiester linkage between a first nucleoside monophosphate and a second nucleoside monophosphate, wherein at least one of the first and second nucleoside monophosphates is a fluorescent nucleoside monophosphate; (ii) measuring the fluorescence signal of the aqueous solution; (iii) repeating steps (i) and (ii), wherein upon said repetition the aqueous solution further comprises the substance, and wherein the fluorescence is measured under identical or substantially identical conditions following the provision of the respective aqueous solution; and (iv) comparing the fluorescence signal measured in the presence of the substance with the fluorescence signal measured in the absence of the substance; wherein a measured fluorescence signal which is higher in the presence of the substance than in the absence of the substance indicates that the substance has an ability to bind to STING.

According to a further aspect, the invention provides a method of identifying a substance having an ability to modulate the activity of a 2'-5' phosphodiesterase enzyme, wherein the method comprises the steps of (i) providing an aqueous solution comprising a cyclic dinucleotide and a 2'-5' phosphodiesterase, wherein the cyclic dinucleotide comprises a 3'-5' phosphodiester linkage and a 2'-5' phosphodiester linkage between a first nucleoside monophosphate and a second nucleoside monophosphate, wherein at least one of the first and second nucleoside monophosphates is a fluorescent nucleoside monophosphate; (ii) measuring the fluorescence signal of the aqueous solution; (iii) repeating steps (i) and (ii), wherein upon said repetition the aqueous solution further comprises the substance, and wherein the fluorescence is measured under identical or substantially identical conditions following the provision of the respective aqueous solution; and (iv) comparing the fluorescence signal measured in the presence of the substance with the fluorescence signal measured in the absence of the substance; wherein a measured fluorescence signal which is higher in the presence of the substance than in the absence of the substance indicates that the substance is a 2'-5' phosphodiesterase agonist, while a measured fluorescence signal which is lower in the presence of the substance than in the absence of the substance indicates that the substance is a 2'-5' phosphodiesterase antagonist.

Another aspect of the invention relates to a use of a fluorescent nucleoside triphosphate for measuring cGAS activity.

A further aspect of the invention provides a use of a fluorescent nucleoside triphosphate for identifying a substance with an ability to modulate cGAS activity.

According to another aspect, the invention also relates to a use of a cyclic dinucleotide for identifying a substance having an ability to bind to STING.

A further aspect of the invention relates to a use of a cyclic dinucleotide for labeling cGAS or STING.

In a final aspect, the invention relates to complexes of cGAS or STING with a cyclic dinucleotide.

Other embodiments of the invention are set out below.

## Brief description of the Figures

Figure 1: The cGAS/STING axis. Scheme depicting the cGAS/STING axis of cytosolic DNA sensing. Upon DNA recognition, the cGAS protein synthesizes 2'3' cyclic dinucleotides (2'3'CDN) from nucleoside triphosphates (here shown by the example of 2'3'cGAMP synthesized from GTP and ATP). 2'3'CDN binds to the adaptor molecule STING and induces a conformational change, leading to activation of TBK1 and type I interferon (IFN) production.

Figure 2: Domain structure of STING. STING comprises four N-terminal transmembrane domains (TM 40-135aa),

a central c-di-GMP-binding domain (CBD, 150-345aa) and a C-terminal tail (CTT, 346-379). The C-terminal part CBD+CTT - cytosolic domain (CD) - is responsible for CDN binding and signal transduction.

Figure 3: Domain structure of cGAS. cGAS comprises an unstructured and poorly conserved N-terminus spanning amino acid sequences 1-159 and a highly conserved C-terminus (160-513aa, catalytic domain - CD) including Mab-21 domain (213-513 aa). The conserved CD domain comprises both DNA-binding capacity and enzymatic activity.

Figure 4: Structure of 2'3'cGAMP (a) and 2'3'fGAMP (b). 2'3'fGAMP is synthesized by cGAS using 2-aminopurine riboside triphosphate (2-APTP) and GTP substrates.

Figure 5: cGAS activity increases with DNA length. (a) cGAS activation determined by CXCL 10 cytokine expression in BLaER1 cells. Cells were stimulated with 20, 40 and 60 ng DNA of different length (20-100 bp in 5 bp intervals) and herring testes (HT) DNA, and CXCL 10 concentration in the supernatant was measured by ELISA. First two bars of each series represent unstimulated cells and lipofectamine controls. Shown are mean values ± standard deviation, n=3. (b) and (c) Activity of $hcGAS^{cd}$ (truncated human), hcGAS (full length human) (b) and $mcGAS^{cd}$ (truncated mouse) (c) in the presence of DNA of increasing length (20-100 bp) or plasmid DNA. Mean values of initial cGAS reaction rates ($\Delta F/\Delta t$) measured by the rate of 2-APTP incorporation into 2'3'fGAMP are plotted against DNA length ± standard deviation, n=3-5. (d) Radiolabeled cGAMP production of cGAS stimulated with DNA of different lengths (20 bp, 35 bp, 55 bp and plasmid). cGAS reactions in presence of [$\alpha^{32}$P]ATP were stopped at the indicated time points and radiolabeled compounds (shown with black arrows) were visualized.

Figure 6: Fluorescence-based cGAS activity assays. (a) cGAS radiolabeled products formation with different substrate composition: ATP+GTP, 2-APTP +GTP, GTP in presence of [$\alpha^{32}$P]GTP; or ATP+GTP, 2-APTP+ATP, ATP in presence of [$\alpha^{32}$P]ATP. Reactions were stopped at the indicated time points and radiolabeled compounds and cGAS reaction products (shown with black arrows) were visualized. (b) and (c) 2'3'fGAMP mobility in anion-exchange chromatography on MonoQ 5/50 GL column. (b) Comparison of 2'3'fGAMP (straight line) arising from cGAS reaction with 2-APTP and GTP, 2-APTP (dashed line) and GTP (dotted line) mobilities. (c) Comparison of 2'3'fGAMP (straight line), 2'3'-cGAMP (dashed line) and 3'3'-cGAMP (dotted line) mobilities. (d) ESI LC/MS spectrum of enzymatically synthesized fGAMP. Arrows indicate ions corresponding to fGAMP-H (m/z 673.09) and fGAMP-2H+$Na^+$ (m/z 695.07). (e) Tandem mass spectrometry spectrum derived from a parent fGAMP ion (m/z 673.09). Arrows highlight ion products corresponding to depurination of the dinucleotide: guanine (m/z 522.19) or 2-AP (m/z 538.15). The ratio of these peaks is characteristic for the linkage between 2'-OH of GTP and 5'-phosphate of 2-APTP within fGAMP molecule (Ablasser, Goldeck et al. "cGAS produces a 2[prime]-5[prime]-linked cyclic dinucleotide second messenger that activates STING". Nature 498, 380-384, 2013).

Figure 7: The principle of fluorescence-based cGAS activity assay. (a) Fluorescence of 2-APTP is quenched upon its incorporation into cyclic 2'3' fluorescent GMP-AMP (2'3'fGAMP) during the reaction that allows the measurement of the reaction rate by differences in fluorescence intensity. (b) Comparison of 2-APTP fluorescence intensity under physiologically relevant conditions: in ultrapure water (1), 100 mM NaCl+5 mM $MgCl_2$ (2), 40 mM Tris pH 7.5 (3), 500 $\mu$M GTP (4), 40 mM Tris pH 7.5 + 100 mM NaCl + 5 mM $MgCl_2$ (5), 40 mM Tris pH 7.5 + 100 mM NaCl + 5 mM $MgCl_2$ + 500 $\mu$M GTP (6). 2-APTP fluorescence intensity is not altered by cGAS activity assay buffer in comparison to water. (c-e) General workflow for calculating the initial cGAS reaction rates. Initial fluorescence read-out curves (c) represent the changes in fluorescence during the reaction. Next, for each curve the background fluorescence values were subtracted and inverted to give a positive value ($\Delta$F). The resulting curves were scaled, such that $\Delta$F at time point 0 min equals 0 ($\Delta F-\Delta F_{t=0}$) (d). The initial cGAS reaction rates were calculated as a slope of the linear intervals (d, dashed lines) on the resulting curves and are defined as $\Delta F/\Delta t$ [RFU $min^{-1}$] (e).

Figure 8: Reactions of 2-APTP and GTP with different cGAS constructs. (a) Anion exchange chromatography of *Homo sapiens* (straight line), *Mus musculus* (dashed line) and *Sus scrofa* (dotted line) truncated (Mab21 domain) cGAS reactions with 2-APTP and GTP. 2-APTP is incorporated into 2'3'fGAMP in species-unspecific manner. (b), (c) and (d) - Fluorescence quenching assays with truncated mouse cGAS ($mcGAS^{cd}$) (b) and human cGAS ($hcGAS^{cd}$) (c), as well as with full length human cGAS (hcGAS) (d). Bar charts represent mean values of initial steady state activity rates of reactions (calculated as described in Fig.7) plotted against DNA length ± standard deviation, n=3. The assays are suitable for evaluating activity of different cGAS constructs from different species.

Figure 9: DNA-bending proteins boost cGAS activity. (a-d) cGAS activity measured by the rate of 2-APTP incorporation into 2'3'fGAMP in presence of DNA-bending proteins. Mean values of initial cGAS reaction rates ($\Delta F/\Delta t$) are plotted against increasing concentrations of DNA bending proteins ± standard deviation, n=3-8. (a) mTFAM, (b) mHMGB1 and (c) 1HU robustly enhance $mcGAS^{cd}$ activity in a dose dependent manner until they eventually compete cGAS away. (d) hTFAM activates hcGAS.

Figure 10: STING-fGAMP binding. (a) Luciferase activity assays in HEK293T-mSTING flip-in cells (Ablasser, Schmid-Burgk et al. "Cell intrinsic immunity spreads to bystander cells via the intercellular transfer of cGAMP". Nature 503, 530-534, 2013) upon the induction with fluorescent cGAS product fGAMP. IFN-$\beta$ response was measured as a proportion of firefly (FF) luciferase activity to Renilla (Ren) luciferase activity. All ratios were normalized to fGAMP buffer control. Plotted values correspond to mean values, error bars represent ± standard deviation, n=3. IFB-$\beta$

response increases with fGAMP concentration meaning fGAMP is capable of STING activation *in vivo.* (b) STING-dependent fGAMP quenching assay. Mean values of fluorescence read-outs ± standard deviation (n=3) are plotted against increasing concentration of hSTING cytoplasmic domain ($STING^{cd}$). fGAMP fluorescence decreases with increasing STING concentration suggesting STING-binding induced fGAMP quenching. (c) and (d) Competition assays based on reversal of fGAMP quenching by STING. 2'5'-cGAMP (c, d) or c-di-GMP (d) were titrated into fGAMP-STING complex. Diagrams represent fluorescence read-outs. Maximal fGAMP fluorescence in the absence of STING for each experiment is shown with horizontal lines. The observed differences in maximal fGAMP fluorescence are due to inaccuracy in experimental setup and can be better estimated by higher number of replicates. STING ligands displace fGAMP from STING binding pocket resulting in an increase of fluorescence. High affinity STING ligands (e.g. cGAMP, c and d) replace fGAMP more efficiently and at lower concentrations than weaker binding ligands (e.g. c-di-GMP, d) leading to a bigger slope of fluorescence intensity increase (dotted lines $s_1$ and $s_2$, $s_{1\,(c\text{-}di\text{-}GMP)} < S_{2\,(cGAMP)}$). The method offers a quick and high-throughput approach for searching for potent STING activators or inhibitors using the simple fluorescence readout.

## General

It is to be understood that the forgoing general description as well as the following detailed description are exemplary and explanatory only and are not restrictive of the invention as claimed. In this application, the use of the singular may include the plural unless specifically stated otherwise. Further, the use of the term "including" as well as other grammatical forms such as "includes" and "included", is not limiting.

As used herein the term "comprising" has the broad standard meaning "including", "encompassing", or "containing". It includes the explicitly recited elements, and also allows the presence of other elements not recited. In addition to this broad meaning, as used herein, the term "comprising" also encompasses the limiting meaning "consisting of'. This means that any aspect or embodiment of the present invention that is defined as comprising features, also includes, in its most limited form, the meaning of consisting (only) of said features and no others, whether this is explicitly stated or not. In addition, the term "comprising" also includes the meaning of "consisting essentially of", which means that other elements may be present which do not alter the technical effect achieved by the explicitly recited elements.

As used herein the term "about" when referring to a particular value such as a wavelength, sequence homology, and the like, is meant to encompass, in addition to the recited value itself, variations of ± 5.0, ± 4.9%, ± 4.8%, ± 4.7%, ± 4.6%, ± 4.5%, ± 4.4%, ± 4.3%, ± 4.2%, ± 4.1%, ± 4.0%, ± 3.9%, ± 3.8%, ± 3.7%, ± 3.6%, ± 3.5%, ± 3.4%, ± 3.3%, ± 3.2%, ± 3.1%, ± 3.0%, ± 2.9%, ± 2.8%, ± 2.7%, ± 2.6%, ± 2.5%, ± 2.4%, ± 2.3%, ± 2.2%, ± 2.1%, ± 2.0%, ± 1.9%, ± 1.8%, ± 1.7%, ± 1.6%, ± 1.5%, ± 1.4%, ± 1.3%, ± 1.2%, ± 1.1%, ± 1.0%, ± 0.9%, ± 0.8%, ± 0.7%, ± 0.6%, ± 0.5%, ± 0.4%, ± 0.3%, ± 0.2%, or ± 0.1% from the specified value. It is to be understood that the term "about", in reference to the particular value, includes that exact particular value itself, irrespective of any explicit mention that this exact particular value is included. Thus, the absence of an explicit indication that the term "about" includes the particular exact recited values is not to be understood that this particular recited values is excluded from the range of variations created by the term "about". Even in the absence of an explicit indication that the term "about" includes the particular exact recited value, this exact particular value is still included in the range of variation created by the term "about". If the term "about" is recited before a numerical range, the term "about" may refer to the lower end point, the upper end point or both the lower end point and the upper end point.

The skilled person will understand that "measuring a fluorescent signal" as recited in the methods of the invention requires that the parameters (e.g. excitation and emission wavelength or wavelength range) of the fluorescent signal measured should be adapted to each fluorescent nucleoside either in free form or in a form incorporated into a cyclic dinucleotide in order to obtain optimal quantum yields. Different fluorescent nucleosides either in free form or in a form incorporated into a cyclic dinucleotide will have different excitation and emission wavelength optima. This means for example that depending on the fluorescent nucleoside used in the methods of the invention the wavelengths or range of wavelengths at which a fluorescent nucleoside or a cyclic dinucleotide comprising it is excited (i.e. the excitation maxima) and the wavelengths or range of wavelengths at which the fluorescence signal is measured (i.e. the emission maxima) need to be adapted for each fluorescent nucleoside individually in order to avoid false positives by measuring a fluorescence signal outside the optimal wavelength or range of wavelengths for a given fluorescent nucleoside or a cyclic dinucleotide comprising it. The skilled person will know or can readily determine the wavelength or range of wavelengths at which the fluorescence signal for a given fluorescent nucleoside either in free form or in a form incorporated into a cyclic dinucleotide should be measured in order to obtain optimal quantum yields of the fluorescence signal.

The methods of the invention include the step of measuring the fluorescence signal "of a solution", e.g. of the aqueous solution. By this is meant that the fluorescence signal which is measured is that which arises from the totality of all fluorescing entities present in the respective solution. This fluorescence may for example arise from fluorescent nucleoside triphosphates in free form or fluorescent nucleoside monophosphates already incorporated into a CDN, which itself may either be a free CDN or a CDN bound by STING, depending on the particular embodiment of the invention,

or may arise from a combination of the above. As such, the intensity of the overall "fluorescence signal of the aqueous solution" measured will depend on how much of a respective fluorescent nucleoside is in a form in which the fluorescence arising from the fluorescent nucleoside is quenched, i.e. reduced. The skilled person understands, for instance, that the measured intensity of the fluorescence of an aqueous solution containing only free (unquenched) fluorescent nucleoside will be greater than that of an aqueous solution in which a fraction of the fluorescent nucleoside is present in free (unquenched) form and another fraction of the fluorescent nucleoside is present in a quenched form. In either case, the readout is the overall fluorescence signal which arises as a summation of the fluorescence intensity from all fluorescing entities in the respective solution, whether free, partially quenched or fully quenched.

The term "identical conditions" as used herein means that the composition of the aqueous solution in the methods of the invention is the same in the presence and absence of a given substance to be tested. This means that the aqueous solution in the presence and absence of the substance has the same concentrations of e.g. proteins (e.g. cGAS, STING and/or 2'-5' phosphodiesterases), nucleoside triphosphates or cGAS-activating nucleic acids (i.e. for methods relating to cGAS), cyclic dinucleotides (i.e. for methods relating to STING and 2'-5'phosphodiesterases), salts, divalent cations, or any other possible solute. Furthermore, the term "identical conditions" as used herein means that the fluorescence signal of a fluorescent nucleoside or a cyclic dinucleotide comprising it is measured at the same wavelength or range of wavelengths in the presence and absence of a substance to be tested. For example, if it is known that the fluorescent nucleoside either in free form or in a form incorporated into a cyclic dinucleotide fluoresces using a certain excitation wavelength or wavelength range, and that this fluorescence may be measured at a certain emission wavelength or wavelength range, then that same wavelength or wavelength range should be used in measuring the fluorescence of the fluorescent nucleoside or cyclic dinucleotide comprising it in the presence and absence of the substance to be tested. The "identical conditions" as recited in the methods of the invention therefore help ensure comparability of the fluorescence signals measured in the presence and absence of the substance to be tested, so that any change in the relative levels of these signals can confidently be attributed to the effect of that substance itself.

The term "substantially identical" as used herein has the meaning of identical within normal experimental error, e.g. with regard to the reaction conditions used in the measurement of a fluorescent signal in order to allow valid comparison of the measured fluorescence signals from two or more measuring steps. For example, the term "substantially identical" as used in accordance to the present invention refers to the measurement of two or more fluorescence signals at the same time points in the same aqueous solution (i.e. the aqueous solutions of two or more measuring steps comprises same salt and protein concentrations, same pH and the like) in order to allow a reliable comparison of the measured fluorescence signals in the presence or absence of a substance. The skilled person recognizes that normal variance within experimental error, e.g. due to pipetting solution volumes, weighing solutes and the like, are unavoidable, but still lead to results which are substantially identical.

The features of any one embodiment disclosed herein are intended as being combinable with those of any other embodiment. Such combinations of one or more features in any one embodiment with one or more features in any other embodiment belong to the disclosure of the present application as filed.

All documents or portions of documents cited in this application, including but not limited to patents, patent applications, articles and books, are hereby expressly incorporated by reference in their entirety for any purpose.

## Detailed description

As used herein the term "cyclic dinucleotide" (CDN) refers to a molecule in which two nucleoside monophosphates are covalently joined, via two phosphodiester linkages, into a single species. Such "head to toe"-linked CDNs sometimes serve as a second messenger secreted by certain bacteria, such as *Listeria monocytogenes*, or generated by the cyclic GMP-AMP synthase (cGAS) following binding to cytosolic DNA. CDNs produced by the enzyme cGAS comprise an asymmetric 2'-5' and 3'-5' phosphodiester linkage between a first nucleoside monophosphate and a second nucleoside monophosphate. CDNs produced by cGAS are referred to as "2'3' cyclic dinucleotide" or "2'3'CDN" according to the present invention. The first and the second nucleoside monophosphate in the CDN according to the invention can be either a deoxyribonucleoside monophosphate or a ribonucleoside monophosphate provided that one of the first and second nucleoside monophosphate has a free 2' hydroxyl group and the other one has a free 3' hydroxyl group in order to enable the 2'-5' and 3'-5' phosphosdiester linkages. Positions of the sugar backbone of the CDN according to the invention which do not participate in cyclization can be substituted with any moiety such as H-, OH-, methyl-, amino-, methoxy-, fluoro-, methoxyethyl-, -O-propargyl, and O-propylamine, provided that such moiety does not impede cyclization of the two nucleoside monophosphates. Furthermore, the phosphodiester linkages of the 2'3'CDN according to the invention may comprise modifications such as thiophosphate, boranophosphate, methyl phosphonate, N-3'-phosphoramidate (O2P-NH-ribose) e.g. in order to enhance the stability of the phosphodiester linkage.

As used herein, the terms "nucleoside" and "nucleotide" used in various forms (e.g. "nucleoside monophosphate", "cyclic dinucleotide", etc.) have the commonly accepted meaning in the art. These species contain a 1-β-D-furanose moiety with its stereochemical configuration as in naturally occurring DNA and RNA.

As used herein the term "cyclic GMP-AMP synthase" or "cGAS" refers to an enzyme belonging to the nucleotidyltransferase (NTase) superfamily which is responsible for cytosolic DNA recognition. Upon recognition of cytosolic dsDNA, cGAS becomes activated by the formation of a cGAS/dsDNA complex. Activated cGAS converts free nucleotide triphosphates into cyclic dinucleotides having an asymmetric 3'-5' and 2'-5' phosphodiester linkage between a first and a second nucleoside monophosphate (i.e. 2'3'CDN). These cyclic dinucleotides can serve as second messengers in downstream signaling. Non-limiting examples for variants of cGAS may be defined by e.g. SEQ ID NO: 10 and SEQ ID NO: 12.

As used herein the term "stimulator of interferon genes" or "STING" refers to a protein associated with the endoplasmic reticulum that binds to cyclic dinucleotides to trigger cytokine production. STING is expressed in various endothelial and epithelial cell types, as well as in haematopoietic cells, such as T cells, macrophages and dendritic cells. STING is activated upon binding of CDNs and formation of a complex consisting of a V-shaped STING dimer and a CDN molecule. CDNs which are capable of activating STING are either secreted by various bacteria or produced by the enzyme cGAS. 2'3'CDNs produced by cGAS have a much higher binding affinity for STING than CDNs secreted by bacteria which have two conventional 3'5' phosphodiester linkages between the first and the second nucleoside monophosphates (3'3'CDN). Therefore, 2'3'CDNs produced by cGAS play a key role in STING activation. Activated STING then interacts with Tank-binding kinase 1 (TBK1) and traffics through the Golgi to perinuclear endosomal compartments, where TBK1 phosphorylates the transcription factors IRF3 and IRF7 to induce the expression of pro-inflammatory cytokines and chemokines. Non-limiting examples for variants of STING may be defined by e.g. SEQ ID NOs: 2, 4, 6 and 8.

As used herein the term "2'-5' phosphodiesterase" refers to a pyrophosphate/phosphodiesterase enzyme which degrades the phosphodiester bond in 2'3'CDNs. The enzyme regulates the localization, duration and amplitude of cyclic dinuleotide signaling within subcellular domains and therefore plays an important role in the signal transduction mediated by 2'3'CDN second messenger molecules. Beside the cleavage of 2'3'CDNs, the 2'-5' phosphodiesterase is also capable of cleaving 3'3'CDNs secreted by various bacteria, such as *Listeria monocytogenes.*

As used herein the term "DNA-bending protein" refers to proteins that upon interaction with DNA induce a structural bend or kink in the DNA. In general, bending or introducing curves or kinks in DNA can lead to DNA compactification or to structural arrangement of DNA binding sites in cis. Examples are bacterial and mitochondrial genome structuring protein (e.g. bacterial HU, mitochondrial TFAM), where DNA bending in conjunction with protein dimerization induces loop structures in DNA, resulting in genome packaging as nucleoids. Other DNA bending proteins such as the transcription factor TBP shape DNA to be recognized by the transcription complexes. Histone proteins also bend or curve DNA, forming nucleosomes. HMG box proteins also bend DNA upon binding. These proteins function in stress response. In one illustrative example of the present invention, a "DNA-bending protein" may represent a "substance" having an ability to enhance cGAS activity. The "DNA-bending proteins" as used in the illustrative examples of the present invention therefore provide evidence that the below described methods of the invention are suitable to identify a substance having an ability to modulate (e.g. enhance) cGAS activity. In any event, the skilled person will understand that a "DNA-bending protein" is nonessential for the herein described methods of the invention and merely serves as an illustrative example representing a substance having the ability to enhance cGAS activity.

As used herein the term "affinity tag" refers to a peptide sequence which is genetically linked to the N- or C-terminus of an amino acid sequence of a protein or a polypeptide. Affinity tags according to the invention are used for protein purification, protein detection and enhancing the solubility of a protein. Examples of affinity tags include but are not limited to albumin-binding protein (ABP), alkaline phosphodiesterase (AP), AU1 epitope, AU5 epitope, bacteriophage T7 epitope, bacteriophage V5 epitope, biotin-carboxy carrier protein (BCCP), bluetongue virus tag (B-tag), calmodulin binding peptide (CBP), chloramphenicol acetyl transferase (Crow, Chase et al. "Characterization of Human Disease Phenotypes Associated with Mutations in TREX1, RNASEH2A, RNASEH2B, RNASEH2C, SAMHD1, ADAR, and IFIH1". American journal of medical genetics. Part A 0, 296-312) cellulose binding domain (CBP), chitin binding domain (CBD), choline-binidng domain (CBD), dihydrofolate reductase (DHFR), E2 epitope, FLAG epitope, galactose-binding protein (GBP), green fluorescent protein (GFP), Glu-Glu (EE-tag), glutathione S-transferase (GST), human influenza hemagglutinin (HA), Halo Tag® (Promega), histidine affinity tag (Li, Shu et al. "Cyclic GMP-AMP Synthase Is Activated by Double-Stranded DNA-Induced Oligomerization". Immunity 39, 1019-1031), horseradish peroxidase (HRP), HSV epitope, ketosteorid isomerase (KSI), KT3 epitope, LacZ, luciferase, maltose-binding protein (MBP), Myc epitope, NusA, PDZ domain, PDZ ligand, polyarginine (Arg-tag), polyaspartate (Asp-tag), polycysteine (Cys-tag), polyhistidine (e.g. $His_6$-tag), polyphenylalanine (Phe-tag), Profinity eXact™ fusion Tag (Bio-Rad), protein C, S1-tag, S-tag, streptavidin-binding peptide (SBP), Staphylococcal protein A (protein A), Staphylococcal protein G (protein G), Strep-tag, Streptavidin, small ubiquitin-like modifier (SUMO), tandem affinity purification (TAP), T7 epitope, thioredoxin (Trx), TrpE, ubiquitin, Universal, VSV-G. Furthermore, the term "affinity tag" as used herein may refer to a combination of one or more of the above recited peptide sequences.

As used herein the term "nucleoside" refers to a molecule comprising a ribofuranosyl or deoxyribofuranosyl ring linked to a nucleobase via a beta glycosidic linkage. If the 5'oxygen of the (deoxy)ribofuranosyl is linked to one, two or three phosphate groups, the nucleoside is referred to as a nucleoside monophosphate, diphosphate or triphosphate,

respectively, as commonly denoted in the art. The one, two or three phosphate groups can comprise modifications such as thiophosphates, boranophosphate, methyl phosphonate, N-3'-phosphoramidate (O2P-NH-ribose) e.g. in order to increase the stability of phosphodiester bonds. Examples of nucleobases comprised in the nucleoside molecule include but are not limited to adenine, guanine, thymine, uracil, cytosine and fluorescent nucleobase analogues.

As used herein the term "fluorescent nucleoside" refers to a nucleoside comprising a fluorescent nucleobase analogue having an intrinsic fluorescence, i.e. the fluorescence is a property of the heterocyclic ring of the nucleobase analogue itself and not a property from an extrinsic fluorescent label attached to it. Fluorescent nucleobase analogues resemble the shape of naturally occurring nucleobases, such as cytosine (C), guanine (G), adenine (A), thymine (T) or uracil (U), and also have the ability to form hydrogen bonds with other nucleobases, e.g. in the manner known for nucleic acid base-pairing. The fluorescent nucleobase analogues herein therefore include those having a high structural similarity to the respective natural nucleobases and, when incorporated into cyclic dinucleotides (CDNs) or polynucleotides, result in fluorescent CDNs or fluorescent poynucleotides which resemble the shape of the natural molecules. Importantly, the fluorescent nucleobase analogues according to the present invention have a fluorescence activity which is higher than the fluorescence activity of natural occurring nucleobases. According to the present invention, the intensity of the fluorescence emitted by the fluorescent nucleobase analogue in a "fluorescent nucleoside" is quenched, i.e. reduced, by a change in the chemical environment surrounding the fluorescent nucleobase analogue in the manner well known for fluorescence resonance energy transfer (FRET), as known in the art. This means that if the fluorescent nucleoside changes its location, e.g. when it is incorporated into a larger molecule such as a CDN, the quantum yield of the fluorescent signal emitted by the fluorescent nucleobase analogue will be quenched, i.e. reduced, thereby allowing one to analyze environmental changes of the fluorescent nucleoside comprising the fluorescent nucleobase analogue by measuring its fluorescence. In particular, the fluorescence intensity (i.e. the quantum yield $\varphi$) of a fluorescent nucleoside decreases upon incorporation of the fluorescent nucleoside into a CDN molecule, and/or upon binding of the fluorescent nucleoside or a CDN comprising the fluorescent nucleoside to the cGAS/STING signaling pathway, e.g. upon binding to cGAS and/or STING. Due to the dependence of fluorescence intensity of the fluorescent nucleosides on environment, the fluorescent nucleosides of the present invention can therefore be utilized to measure physiological processes including but not limited to enzymatic activity and interaction of molecules as described herein.

As used herein the term "2-aminopurine" or "2-AP" refers to a highly fluorescent nucleobase analogue of adenine having the lowest energy absorption band centered at 305 nm and a molar absorptivity ($\varepsilon$) of 6000 $M^{-1}$ $cm^{-1}$. The position of the band is outside the absorption of the natural nucleic acids, so 2-AP can be selectively excited in the presence of natural nucleobases. The high quantum yield of free 2-AP in water at 25°C ($\varphi$=0.68) is considerably quenched, i.e. reduced, (~100 fold) when 2-AP is incorporated into a CDN or nucleic acid. It is quenched further still when the 2'3'CDN in which 2-AP exists is bound by the protein STING. This high sensitivity of 2-AP to its microenvironment can be utilized in a variety of assays to study e.g. DNA or nucleotide/protein interactions and enzymatic activity. 2-AP has the following structure:

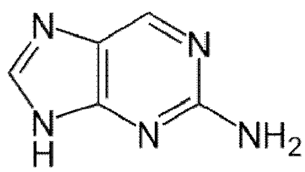

.

As used herein the term "quantum yield" refers to the efficiency of the fluorescence process. The term "quantum yield" as used herein is defined as the ratio of the number of photons emitted to the number of photons absorbed. It is defined by the following equation:

$$\varphi = \frac{\text{Number of photons emitted}}{\text{Number of photons absorbed}}.$$

The maximum fluorescence quantum yield is 1.0 (100%), which means that each photon absorbed results in a photon emitted. The fluorescence signal of a "fluorescent nucleoside" according to the present invention may have a quantum yield ($\varphi$) in free form in water at 25°C of about 0.10 to about 1.0 or between about 0.10 and 1.0. In one embodiment of the invention a fluorescent nucleoside according to the invention has a quantum yield in free form in water at 25°C of at least: about 0.10, about 0.11, about 0.12, about 0.13, about 0.14, about 0.15, about 0.16, about 0.17, about 0.18, about 0.19, about 0.20, about 0.21, about 0.22, about 0.23, about 0.24, about 0.25, about 0.26, about 0.27, about 0.28, about 0.29, about 0.30, about 0.31, about 0.32, about 0.33, about 0.34, about 0.35, about 0.36, about 0.37, about 0.38, about 0.39, about 0.40, about 0.41, about 0.42, about 0.43, about 0.44, about 0.45, about 0.46, about 0.47,

about 0.48, about 0.49, about 0.50, about 0.51, about 0.52, about 0.53, about 0.54, about 0.55, about 0.56, about 0.57, about 0.58, about 0.59, about 0.60, about 0.61, about 0.62, about 0.63, about 0.64, about 0.65, about 0.66, about 0.67, about 0.68, about 0.69, about 0.70, about 0.71, about 0.72, about 0.73, about 0.74, about 0.75, about 0.76, about 0.77, about 0.78, about 0.79, about 0.80, about 0.81, about 0.82, about 0.83, about 0.84, about 0.85, about 0.86, about 0.87, about 0.88, about 0.89, about 0.90, about 0.91, about 0.92, about 0.93, about 0.94, about 0.95, about 0.96, about 0.97, about 0.98, about 0.99, or about 1.00. A nucleoside is still considered "intrinsically fluorescent", and therefore a "fluorescent nucleoside" according to the present invention, if it has a quantum yield in free form in water at 25°C of at least 0.10.

As used herein the term "molar absorptivity" refers to the molar attenuation coefficient or molar extinction coefficient and is a measurement of how strongly a chemical species, e.g. a fluorophore, absorbs light at a given wavelength. It is an intrinsic property of the chemical species, i.e. it depends on the chemical structure of the species.

As used herein the term "aqueous solution" refers to a solution comprising water, e.g. a solution comprising water and at least one buffer. A buffer comprises a weak acid and its conjugate base or a weak base and its conjugate acid. A buffer keeps the pH of a solution constant by taking up protons that are released during reactions, or by releasing protons when they are consumed by reactions. A buffer therefore stabilizes the $H^+$ concentration in vitro without affecting the functioning of a system under investigation. As used herein, an "aqueous solution" can further comprise essential cofactors for enzymatic reaction, such as metal ions, critical salts and essential nutrients for cells or tissues. An aqueous solution may also comprise one or more solvents other than water, as long as such solvents are sufficiently miscible in water such that multiple phases do not form.

As used herein the term "cGAS-activating nucleic acid" refers to any nucleic acid molecule capable of activating cGAS. In particular, a cGAS-activating nucleic acid may be a double-stranded (ds), single-stranded (ss) DNA or a DNA/RNA hybrid. In the case of nucleic acid, e.g. dsDNA, the cGAS-activating nucleic acid has a length of at least 10 base pairs (bp). Furthermore, the double-stranded cGAS-activating nucleic acid may have a minimal length selected from the group consisting of at least 11bp, 12bp, 13bp, 14bp, 15bp, 16bp, 17bp, 18bp, 19bp, 20bp, 21bp, 22bp, 23bp, 24bp, 25bp, 26bp, 27bp, 28bp, 29bp, 30bp, 31bp, 32bp, 33bp, 34bp, 35bp, 36bp, 37bp, 38bp, 39bp, 40bp, 41bp, 42bp, 43bp, 44bp, 45bp, 46bp, 47bp, 48bp, 49bp, 50bp, 51bp, 52bp, 53bp, 54bp, 55bp, 56bp, 57bp, 58bp, 59bp, 60bp, 61bp, 62bp, 63bp, 64bp, 65bp, 66bp, 67bp, 68bp, 69bp, 70bp, 71bp. 72bp, 73bp, 74bp, 75bp, 76bp, 77bp, 78bp, 79bp, 80bp, 81bp, 82bp, 83bp, 84bp, 85bp, 86bp, 87bp, 88bp, 89bp, 90bp, 91bp, 92bp, 93bp, 94bp, 95bp, 96bp, 97bp, 98bp, 99bp, and 100bp. The double-stranded cGAS-activating nucleic acid may also have a minimal length of at least 200bp, 300bp, 400bp, 500bp, 600bp, 700bp, 800bp, 900bp, 1kbp, 2kbp, 3kbp, 4kbp, 5kbp, 6kbp and the like. Corresponding lengths also apply in the event the cGAS-activating nucleic acid, e.g. DNA, is single-stranded. The double-stranded cGAS-activating nucleic acid may be DNA, for example a plasmid, such as a commonly known expression vector. The cGAS-activating nucleic acid may also be in the form of single-stranded DNA or DNA/RNA hybrids. Such DNA/RNA hybrids may also have the lengths as set out above.

As used herein the term "substance" refers to any molecule which can be tested for its binding to cGAS or STING. The term "substance" as used herein may for example refer to any molecule having an ability to modulate cGAS or 2'-5' phosphodiesterase activity. A "substance" according to the invention may be a small molecule, a protein, a polypeptide, or a single amino acid. The protein may be an enzyme having biological activity, or an antibody. The term "antibody" as used herein comprises the full-length antibody or fragments thereof, wherein the term "antibody fragment" comprises a Fab, F(ab')2, monospecific Fab2, bispecific Fab2, trispecific Fab2, monovalent IgG, scFv, bispecific scFv, bispecific diabody, trispecific triabody, scFv-Fc, minibody and the like. The term "substance" as used herein may also comprise chemical molecules having saturated or unsaturated hydrocarbons, such as alkanes, cycloalkanes, alkenes and the like, i.e. may generally represent any member of the class of "small molecules" which the skilled person recognizes as constituted by substances which may be synthesized by an organic synthetic chemist without the need to implement microorganisms. A "substance" according to the invention may further comprise an alcohol, ester, ether, amide and the like. Furthermore, a "substance" as used herein may be a carbohydrate or a lipid. Nucleic acids or single nucleotides may also be a "substance" according to the present invention. A nucleic acid may be a double-stranded (ds) or single standed (ss) DNA. A nucleic acid may also be an RNA, such as a miRNA or siRNA, or a DNA/RNA hybrid. A nucleic acid may also refer to poly(I:C), poly(dI:dC), poly(dA:dU), or any combination of ribose and deoxyribose backbones. The backbones furthermore can contain phosphothioate esters, instead of phosphates esters between the backbone sugars. In addition, it may contain or consist of peptide nucleic acid (PNA) backbones, locked nucleic acid (LNA) backbones, Glycol nucleic acid (GNA) backbones, threose nucleic acid (TNA) backbones and other Xeno nucleic acid (XNA) alternatives. In one illustrative example, a substance according to the present invention is represented by a "DNA bending protein" which is capable of enhancing cGAS activity as shown by the methods of the present invention.

As used herein the term "systemic lupus erythematosus" or "SLE" refers to an autoimmune disease in which antibodies specific for DNA, RNA or proteins associated with nucleic acids form immune complexes that damage small blood vessels, especially in the kidneys. Patients with SLE generally have abnormal B and T cell function. SLE can be associated with hyperproduction of type I interferon.

As used herein the term "Aicardi-Goutières syndrome" or "AGS" refers to a neurodegenerative disorder that

can be caused by STING-dependent cytokine hyperproduction owing to mutations in genes such as TREX-1 (three-prime repair exonuclease 1).

As used herein the term "idiopathic arterial calcification of infancy" or "IAIC" refers to a rare condition characterized by extensive calcification and stenosis of large and medium sized arteries. IACI is an unusual genetically inherited autosomal recessive condition which may be dystrophic or metastatic.

As used herein the term "stenosis" refers to an abnormal narrowing in a blood vessel or other tubular organ or structure.

As used herein the term "pseudoxanthoma elasticum" or "PXE" refers to a progressive disorder that is characterized by the accumulation of deposits of calcium and other minerals (mineralization) in elastic fibers. Elastic fibers are a component of connective tissue, which provides strength and flexibility to structures throughout the body.

As used herein the term "diabetic kidney disease" refers to a kidney disease caused by or coexistent with diabetes.

As used herein the terms "engineered", "genetic engineering" or "genetic modification" refer to the manipulation of a nucleotide sequence of a gene encoding a protein or enzyme. A nucleotide sequence may for example be manipulated by introducing mutations into the nucleotide sequence of the gene, thereby altering the amino acid sequence of the encoded protein or enzyme. For example, mutations can be introduced into the active site of an enzyme, thereby altering its enzymatic activity. By choosing the appropriate mutation the enzymatic activity can be increased, decreased or completely silenced compared to the unmodified enzyme. A mutation in the nucleotide sequence of a gene encoding a protein can alter the conformation of the protein thereby influencing its functionality and stability. Furthermore, specific mutations can be introduced into the nucleotide sequence of a gene to investigate polymorphisms, such as single nucleotide polymorphisms, that might be associated with specific diseases. A mutation can be a substitution, deletion or insertion of a single nucleotide or more than one nucleotide in the nucleotide sequence of a gene encoding a protein, e.g. an enzyme. As used herein the terms "engineered", "genetic engineering" or "genetic modification" may also refer to the deletion of nucleotide sequences encoding one or more specific domains of the protein or enzyme, resulting in a truncated version of the molecule. For example, a truncated protein, e.g. a truncated enzyme, might only include the C-terminus, N-terminus or one or more highly conserved catalytic domains compared to the full length molecule. By deleting nucleotide sequences encoding e.g. unstructured regions, the truncated protein, e.g. the enzyme, may gain advantageous properties compared to the full length molecule, including but not limited to increased expression, improved solubility, increased activity or binding affinity. Nucleotide sequences can be further engineered by replacing specific nucleotide sequences of a gene with orthologous nucleotide sequences from different species. Furthermore, nucleotide sequences can be engineered to comprise affinity tags or other labels at their N- or C-terminus allowing for purification or detection of the encoded protein or enzyme. "Genetic engineering" or "genetic modification" is achieved by well known methods in the art including chemical synthesis and PCR technology (Russell. "Molecular Cloning, a laboratory manual". Cold Spring Harbor Laboratory Press 1, 2012);(Hughes, Miklos et al. "Chapter twelve - Gene Synthesis: Methods and Applications". Methods in Enzymology Volume 498, 277-309, 2011; Throop and LaBaer. "Recombinational Cloning Using Gateway and In-Fusion Cloning Schemes". Current protocols in molecular biology / edited by Frederick M. Ausubel ... [et al.] 110, 3.20.21-23.20.23, 2015).

As used herein the term "modulate" or "modulating" refers to the capability of a substance to inhibit, activate or enhance the activity or properties of another substance, e.g. cGAS, STING or 2'-5' phosphodiesterase, thereby changing the concentration of CDNs, in particular 2'3'CDNs. A substance that acts as an antagonist of one of the aforementioned enzymes may partially or fully reduce the physiological function of said protein, thereby resulting in a reduction of CDN production (in the case of cGAS inhibition) or CDN cleavage (in the case of 2'-5' phosphodiesterase inhibition). Specifically, a substance which antagonizes the activity of cGAS may partially or fully inhibit the conversion of the nucleoside triphosphates into 2'3'CDNs. A substance which is an antagonist of cGAS might bind to the active center of the enzyme thereby competing with the nucleoside triphosphates for binding to cGAS. A substance which antagonizes cGAS activity (partially or fully) might also bind to the enzyme in an uncompetitive manner, i.e. to an allosteric site of cGAS which results in an alteration of the active binding site thereby preventing nucleoside triphosphate binding. A substance that acts as an antagonist of 2'-5' phosphodiesterase may function in a competitive or uncompetitive manner similar to a substance inhibiting cGAS activity. A substance which acts as an agonist of cGAS may enhance (partially or fully) cGAS activity, e.g. an agonist of cGAS enhances the conversion of nucleoside triphosphates into 2'3'CDNs in comparison to the conversion of nucleoside triphosphates into 2'3'CDNs in the absence of such an agonist. A substance which activates 2'-5' phosphodiesterase (partially or fully) may enhance the cleavage of the 2'-5' phosphodiester linkage of the 2'3' CDN, e.g. an agonist of 2'-5' phosphodiesterase reduce the overall concentration of 2'3' CDN molecules in a biological system.

As used herein the term "divalent cation" refers to a cation having a valence of 2. A "divalent cation" according to the invention is selected from the group consisting of $Mg^{2+}$, $Ca^{2+}$, $Zn^{2+}$, $Fe^{2+}$, $Cu^{2+}$, $Ni^{+2}$ and $Mn^{2+}$.

As used herein the term "measuring" refers to determining the intensity of a fluorescence signal measured at a predetermined time point or to determining the change of a fluorescence signal measured continuously or at intervals

over a predetermined time interval.

The present invention is now described in more detail by reference to certain preferred embodiments.

## Cyclic dinucleotides

As mentioned above, one aspect of the present invention relates to a cyclic dinucleotide comprising a 3'-5' phosphodiester linkage and a 2'-5' phosphodiester linkage between a first and a second nucleoside monophosphate, wherein at least one of the first and second nucleoside monophosphates is a fluorescent nucleoside monophosphate. The fluorescent cyclic dinucleotides of the present invention are hereinafter referred to as 2'3'fCDNs.

The fluorescent nucleoside comprises a fluorescent nucleobase analogue which possesses intrinsic fluorescence. Furthermore, the intrinsic fluorescence of the fluorescent nucleobase analogue is sensitive to environmental changes. This means that if the fluorescent nucleoside comprising the fluorescent nucleobase analogue changes its disposition with respect to other chemical species, e.g. other nucleobases in a CDN, the quantum yield will be quenched, i.e. reduced, thereby allowing the analysis of environmental changes of the fluorescent nucleoside. In particular, the fluorescence intensity, i.e. the quantum yield of the fluorescent nucleoside, is reduced upon incorporation of the fluorescent nucleoside into a CDN, and/or upon binding of the nucleoside or a CDN comprising it to cGAS and/or STING. Due to their environmental sensitivity the fluorescent nucleosides of present invention can therefore be utilized to measure physiological processes including but not limited to enzymatic activity and interaction of molecules as will be discussed in more detail below.

The fluorescent nucleoside according to the present invention has a fluorescence which is higher than the fluorescence of natural occurring nucleosides, such as adenosine, guanosine, cytosine, thymidine, uracil and the like. In a further embodiment of the present invention, the fluorescence signal of a fluorescent nucleoside according to the present invention has a quantum yield ($\varphi$) in free form in water at 25°C of about 0.10 to about 1.0 or between about 0.10 and 1.0. In one embodiment of the invention a fluorescent nucleoside according to the invention has a quantum yield in free form in water at 25°C of at least: about 0.10, about 0.11, about 0.12, about 0.13, about 0.14, about 0.15, about 0.16, about 0.17, about 0.18, about 0.19, about 0.20, about 0.21, about 0.22, about 0.23, about 0.24, about 0.25, about 0.26, about 0.27, about 0.28, about 0.29, about 0.30, about 0.31, about 0.32, about 0.33, about 0.34, about 0.35, about 0.36, about 0.37, about 0.38, about 0.39, about 0.40, about 0.41, about 0.42, about 0.43, about 0.44, about 0.45, about 0.46, about 0.47, about 0.48, about 0.49, about 0.50, about 0.51, about 0.52, about 0.53, about 0.54, about 0.55, about 0.56, about 0.57, about 0.58, about 0.59, about 0.60, about 0.61, about 0.62, about 0.63, about 0.64, about 0.65, about 0.66, about 0.67, about 0.68, about 0.69, about 0.70, about 0.71, about 0.72, about 0.73, about 0.74, about 0.75, about 0.76, about 0.77, about 0.78, about 0.79, about 0.80, about 0.81, about 0.82, about 0.83, about 0.84, about 0.85, about 0.86, about 0.87, about 0.88, about 0.89, about 0.90, about 0.91, about 0.92, about 0.93, about 0.94, about 0.95, about 0.96, about 0.97, about 0.98, about 0.99, or about 1.00. Any of the above recited quantum yield values may be combined with one another to form a quantum yield range. Also contemplated within such quantum yield ranges are corresponding ranges in which the lower quantum yield is included while the upper quantum yield is excluded, as well as ranges in which the lower quantum yield is excluded while the upper quantum yield is included. In a particular preferred embodiment, the quantum yield of a fluorescent nucleoside in free form in water at 25°C is about 0.68, more preferably is 0.68. This is for example the fluorescent yield of 2-AP in free form in water at 25°C.

In one embodiment of the present invention, the 3'-5' phosphodiester linkage of the cyclic dinucleotide is between the 3' oxygen of the first nucleoside monophosphate and the 5' oxygen of the second nucleoside monophosphate and the 2'-5' phosphodiester linkage of the cyclic dinucleotide is between the 5' oxygen of the first nucleoside monophosphate and the 2' oxygen of the second nucleoside monophosphate. This asymmetric nature of the phosphodieseters is advantageous for high affinity binding to human STING.

In a further embodiment of the present invention, the fluorescent nucleoside monophosphate is a fluorescent purine nucleoside monophosphate. The fluorescent purine nucleoside monophosphate is advantageous for recognition by cGAS and may also enhance binding to STING.

In a preferred embodiment of the present invention, the fluorescent nucleoside monophosphate is selected from the group consisting of a 2-aminopurine nucleoside monophosphate (2-APMP), a 3-methyl-isoxanthopterin nucleoside monophosphate (3-MIMP), a 6-methyl isoxanthopterin nucleoside monophosphate (6-MIMP), 4-amino-6-methyl-8-(2-deoxy-beta-d-ribofuranosyl)-7(8H)-pteridone nucleoside monophosphate (6-MAPMP), a 4-amino-2,6-dimethyl-8-(2'-deoxy-beta-d-ribofuranosyl)-7(8H)-pteridone nucleoside monophosphate (DMAPMP), a pyrrolocytosine nuceloside monophosphate (pyrrolo-CMP), a 6-phenylpyrrolocytosine nucleoside monophosphate (PhpCMP), a (aminoethoxy)phenylpyrrolocytosine nucleoside monophosphate (moPhpCMP), a [bis-o-(aminoethoxy)phenyl]pyrrolocytosine nucleoside monophosphate (boPhpCMP), a hydropyrimidopyrimidine nucleoside monophosphate ($C^{hpp}MP$), a pyrrolopyrimidopyrimidine nucleoside monophosphate ($C^{ppp}MP$), a pyrimidopyrimidoindole nucleoside monophosphate ($C^{ppi}MP$), a benzopyridopyrimidine nucleoside monophosphate (BPPMP), a naphthopyridopyrimidine nucleoside monophosphate (NPPMP), a methoxybenzodeazaadenine nucleoside monophosphate ($^{MD}AMP$), a methoxybenzodeazainosine nucleoside monophosphate ($^{MD}$IMP), a naphthodeazaadenine nucleoside monophosphate ($^{ND}$AMP), a furan-modified pyrimidine nucleoside mohophosphate, a thieno[3,2]pyrimidine nucleoside monophosphate, a thieno[3,4]pyrimidine nucleoside monophosphate, a 5-methoxy-quinazoline-2,4-(1H,3H) dione nucleoside monophosphate, a 5-methylpyrimidine-2-one nucleoside monophosphate, a 7-deazapurine nucleoside monophosphate, a 5-alkyluridine nucleoside monophosphate, a benzoquinalozines nucleoside monophosphate, a triazoleadenosine nucleoside monophosphate, and a 1,$N^6$-ethenoadenosine nucleoside monophosphate.

In a further embodiment of the present invention, a fluorescent nucleoside monophosphate can comprise further chemical modification to the fluorescent nucleobase analogue and the ribofuranosyl backbone provided that such modification does not affect the fluorescence of the fluorescent nucleobase analogue. Furthermore, such modification should not affect the structural and chemical properties of a fluorescent nucleoside monophosphate, i.e. the modification may not affect the fluorescent nucleoside monophosphate's ability to incorporate into nucleotide molecules, such as polynucleotides and CDNs. Possible modifications of the fluorescent nucleoside monophosphate according to the present invention include but are not limited to phosphothioate linker. In addition, it may contain or consist of peptide nucleic acid (PNA) backbones, locked nucleic acid (LNA) backbones, Glycol nucleic acid (GNA) backbones, threose nucleic acid (TNA) backbones and other Xeno nucleic acid (XNA) alternatives.

In a particularly preferred embodiment, the fluorescent nucleoside monophosphate is a 2-aminopurine nucleoside monophosphate (2-APMP), wherein the fluorescent nucleobase analogue is 2-aminopurine. 2-aminopurine (2-AP) is a highly fluorescent nucleobase analogue of adenine having the lowest energy absorption band centered at 305 nm and a molar absorptivity ($\varepsilon$) of 6000 $M^{-1}$ $cm^{-1}$. The position of the band is outside the absorption of the natural nucleic acids and, thus, 2-AP can be selectively excited in the presence of natural nucleobases. The high quantum yield of free 2-AP in water ($\varphi$=0.68) is considerably quenched, i.e. reduced, (~100 times) when incorporated into biological molecules such as nucleic acids or CDNs. This high sensitivity of 2-AP to its microenvironment can be utilized in a variety of methods of measuring enzyme activity or investigating molecule interactions as will be discussed in more detail below.

In a further preferred embodiment of the present invention, 2-APMP is the first nucleoside monophosphate. A CDN comprising 2-APMP as the first nucleoside monophosphate resembles the shape of 2'3'CDNs made by cGAS and recognized by STING, thereby making such CDN in particular suitable for methods of e.g. measuring cGAS activity or identifying substances with an ability to modulate the properties of another substance, e.g. cGAS, STING or 2'-5' phosphodiesterases.

Due to the chemical structure of the 2'3'CDN it is necessary that one of the two nucleoside monophosphates has a free 2' hydroxyl group and the other one has a free 3' hydroxyl group in order to enable the 2'-5' and 3'-5' phosphodiester linkages in the 2'3'CDN. Thus, in a further embodiment of the invention the second nucleoside monophosphate of the cyclic dinucleotide is a purine ribonucleoside monophosphate, preferably a guanosine monophosphate (GMP) having a free 2' hydroxyl group, whereas 2-APMP is the first nucleoside monophosphate having a free 3' hydroxyl group. The chemical structure of a CDN comprising 2-APMP as the first nucleoside monophosphate and guanosine monophosphate as the second nucleoside monophosphate is almost identical to the chemical structure of the 2'3'CDN made by cGAS. Such a CDN is therefore an ideal fluorescent analogue of the naturally occurring 2'3'CDN made by cGAS and recognized by STING, and may thus be used in various methods as set out below in more detail.

In another preferred embodiment of the present invention, the fluorescent cyclic dinucleotide has the following formula

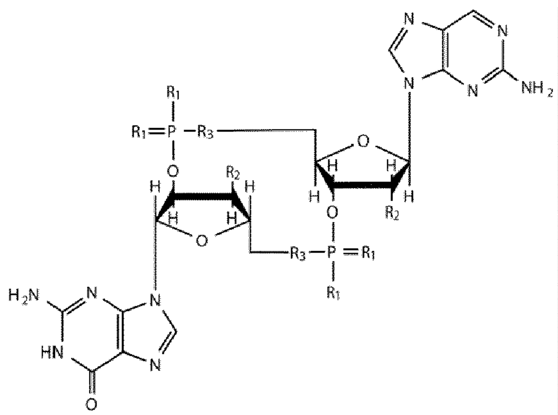

,

wherein

when $R_1$ is bound to P through a single bond, $R_1$ is independently selected from the group consisting of O, S, $BH_3$

and $CH_3$;
when $R_1$ is bound to P through a double bond, $R_1$ is independently selected from the group consisting of O, S and NH;
wherein at least one $R_1$ bound to each P is O;
$R_2$ is independently selected from the group consisting of H, OH, methyl, amino-, methoxy-, fluoro-, methoxyethyl-, -O-propargyl and O-propylamine; and
$R_3$ is O.

In a particularly preferred embodiment of the present invention, the cyclic dinucleotide has the following formula:

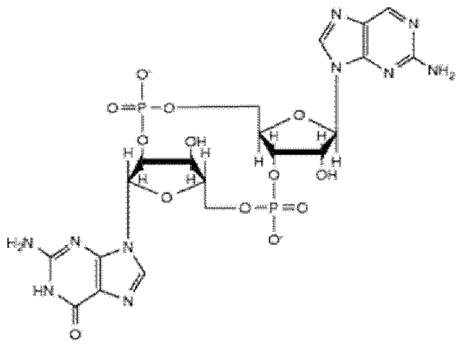

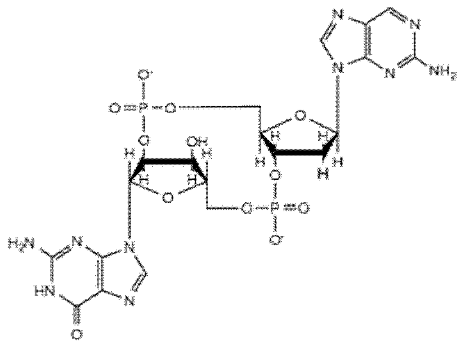

or

or

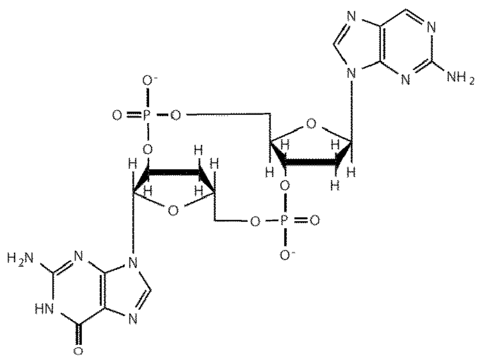

or

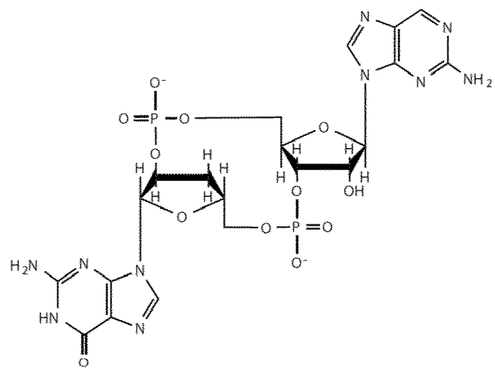

.

This cyclic dinucleotide resembles the structural and chemical properties of cyclic dinucleotide generated by cGAS and recognized by STING and can thus be used in a variety of methods according to the present invention as will be discussed in more detail below.

For example, the 2'3'fCDNs according to the present invention can be used to identify a substance having an ability to bind to STING. The cyclic dinucleotide of the present invention can be further used to identify a substance having an ability to modulate 2'-5' phosphodiesterase activity. Furthermore, the cyclic dinucleotide of the present invention can be used *in vitro* to label enzymes or proteins, such as cGAS and STING, in order to investigate their cellular localization during different diseases and during bacterial or viral infection. For example, the cyclic dinucleotide according to the present invention may form a complex with cGAS or STING, which can be readily localized in an *in vitro* system, such as a mammalian cell culture, due to the fluorescence signal of the fluorescent nucleoside of the cyclic dinucleotide. The fluorescence signal can be detected by using commonly known techniques such as fluorescence microscopy (Lakowicz. "Principles of Fluorescence Spectroscopy". Springer 3th edition, 2006).

__**Method of identifying a substance having an ability to bind to STING**__

Stimulator of interferon genes (STING) is a protein which is expressed in various endothelial and epithelial cell types, as well as in hematopoietic cells, such as T cells, macrophages and dendritic cells. Homologues of STING have been identified in different eukaryotic species as well as in invertebrates. STING stimulates the transcription of numerous innate immune genes, including type I interferon encoding genes, upon binding of cyclic dinucleotides (CDNs). Beside the bacterial 3'3' CDNs, it was found out that the most potent activator of STING is a 2'3'CDN generated by the enzyme cGAS. This second messenger binds STING with approximately 250-fold higher affinity compared to CDNs secreted by

bacteria. Binding of 2'3'CDN to STING results in dimerization of the signaling molecule and triggers a signaling cascade resulting in the expression of various immune modulatory molecules including proinflammatory cytokines, such as type I interferon, and chemokines. STING therefore plays an essential role in controlling the transcription of numerous immune modulatory genes and its dysfunction is related to the development of several diseases. For example, hyperactivity of STING results in hyperproduction of proinflammatory cytokines and chemokines causing autoimmune disorders and inflammatory diseases such as systemic lupus erythematosus (SLE) and Aicardi-Goutières Syndrome (AGS). On the other hand, impaired activity of STING is associated with the development of cancer, promoting the proliferation and spread of tumor cells (Nowarski, Gagliani et al. "Innate Immune Cells in Inflammation and Cancer". Cancer Immunology Research 1, 77-84, 2013).

Up to now, methods for identifying substances that are capable of binding to STING do not provide a reliable and simple assay for identifying STING ligands in a quantitative manner. However, due to its central role in the regulation of the innate immune system and its involvement in the development of various diseases, it would be of great importance to have a robust and simple-to-implement high-throughput method that enables for direct identification of substances capable to bind to STING. Such substances could then serve as a promising starting point in the development of therapeutic drugs specifically targeting STING protein and modulating its activity.

A further aspect of the present invention therefore relates to a method of identifying a substance having an ability to bind to stimulator of interferon genes (STING), wherein the method comprises the steps of (i) providing an aqueous solution comprising a cyclic dinucleotide and STING, wherein the cyclic dinucleotide comprises a 3'-5' phosphodiester linkage and a 2'-5' phosphodiester linkage between a first nucleoside monophosphate and a second nucleoside monophosphate, wherein at least one of the first and second nucleoside monophosphate is a fluorescent nucleoside monophosphate; (ii) measuring the fluorescence signal of the aqueous solution; (iii) repeating steps (i) and (ii), wherein upon said repetition the aqueous solution further comprises the substance, and wherein the fluorescence signal is measured under identical or substantially identical conditions following the provision of the respective aqueous solution, and (iv) comparing the fluorescence signal measured in the presence of the substance with the fluorescence signal measured in the absence of the substance; wherein a measured fluorescence signal which is higher in the presence of the substance than in the absence of the substance indicates that the substance has an ability to bind to STING.

The above method of the present invention is based on the unexpected finding that the fluorescent cyclic dinucleotide (2'3'fCDN) of the present invention, e.g. a CDN comprising 2-APMP, binds to STING, thereby activating the signaling molecule. The inventors surprisingly found out that upon binding of 2'3'fCDN to STING, the fluorescence signal of the cyclic dinucleotide is quenched, thereby allowing the measurement of 2'3'fCDN binding to STING with a simple fluorescence read out technique. While incorporation of 2-AP in the 2-APTP into cyclic 2'3' fluorescent GMP/AMP (2'3'fGAMP) quenches the fluorescence signal of 2-AP relative to its fluorescence as observed in free solution, the binding of 2'3'fGAMP to STING quenches the fluorescence signal of 2-AP further still. Based on this further fluorescence quenching it is thus possible to measure the fluorescence signal in the absence or presence of a substance with a simple-to-implement method and to reliably identify substances that bind to STING. These STING ligands could than serve as promising starting points for the development of therapeutic drugs that specifically modulate STING activity.

According to the present invention, STING is a protein which may be obtained by conventional cloning and expression systems, including *E. coli* expression strains (e.g. BL21(DE3), Lemo21(DE3), ArcticExpress), baculovirus expression systems (ex. in High Five insect cell line), non-viral insect cells expression system (ex. transient transfection to S2 insect cell line using ExpreS2), yeast strains (ex. BCY123) or mammalian cell line (ex. HEK293T, CHO) expression systems. STING can be expressed as full-length protein or as a truncated version comprising the catalytic domain only. Furthermore, STING can be engineered to contain modifications in its nucleic acid sequence altering the amino acid sequence of the protein. For example, STING can be engineered (Gao, Ascano et al. "Structure-Function Analysis of STING Activation by c[G(2',5')pA(3',5')p] and Targeting by Antiviral DMXAA". Cell 154, 748-762, 2013; Gao, Zillinger et al. "Binding-Pocket and Lid-Region Substitutions Render Human STING Sensitive to the Species-Specific Drug DMXAA". Cell Reports 8, 1668-1676, 2014) in its CDN binding site to result in an increased or decreased binding affinity for the 2'3'CDN compared to unmodified STING. Furthermore, STING can be engineered to be constitutively active. Modifications in its nucleic acid can facilitate STING dimerization and/or TBK1 binding (Tang and Wang. "Single Amino Acid Change in STING Leads to Constitutive Active Signaling". PLOS ONE 10, e0120090, 2015). STING may also be modified by expressing the protein as a fusion construct together with a tag, such as a GFP tag that would allow for determination of its cellular localization.

In a further embodiment of the present invention, the protein may further comprise affinity tags allowing purification and detection of the expressed protein.

According to the method of the present invention, STING may be derived from any vertebrate, including mammals such as human, macaque, mouse, sus, bos, and the like, birds, such as chicken and duck, fish, such as zebra fish, other vertebrates such as Xenopus laevis, Choanoflagellate (e.g. Nematostella vectensis), or invertebrates (e.g. insects).

In a preferred embodiment of the present invention, STING is human STING. The nucleotide sequence of full-length human STING is defined by SEQ ID NO: 1.

SEQ ID NO: 1

```
ATGCCCCACTCCAGCCTGCATCCATCCATCCCGTGTCCCAGGGGTCACGGGCC
CAGAAGGCAGCCTTGGTTCTGCTGAGTGCCTGCCTGGTGACCCTTTGGGGGCTA
GGAGAGCCACCAGAGCACACTCTCCGGTACCTGGTGCTCCACCTAGCCTCCCTG
CAGCTGGGACTGCTGTTAAACGGGGTCTGCAGCCTGGCTGAGGAGCTGCGCCAC
ATCCACTCCAGGTACCGGGGCAGCTACTGGAGGACTGTGCGGGCCTGCCTGGGC
TGCCCCCTCCGCCGTGGGGCCCTGTTGCTGCTGTCCATCTATTTCTACTACTCC
CTCCCAAATGCGGTCGGCCCGCCCTTCACTTGGATGCTTGCCCTCCTGGGCCTC
TCGCAGGCACTGAACATCCTCCTGGGCCTCAAGGGCCTGGCCCCAGCTGAGATC
TCTGCAGTGTGTGAAAAAGGGAATTTCAACGTGGCCCATGGGCTGGCATGGTCA
TATTACATCGGATATCTGCGGCTGATCCTGCCAGAGCTCCAGGCCCGGATTCGA
ACTTACAATCAGCATTACAACAACCTGCTACGGGGTGCAGTGAGCCAGCGGCTG
TATATTCTCCTCCCATTGGACTGTGGGGTGCCTGATAACCTGAGTATGGCTGAC
CCCAACATTCGCTTCCTGGATAAACTGCCCCAGCAGACCGGTGACCATGCTGGC
ATCAAGGATCGGGTTTACAGCAACAGCATCTATGAGCTTCTGGAGAACGGGCAG
CGGGCGGGCACCTGTGTCCTGGAGTACGCCACCCCCTTGCAGACTTTGTTTGCC
ATGTCACAATACAGTCAAGCTGGCTTTAGCCGGGAGGATAGGCTTGAGCAGGCC
AAACTCTTCTGCCGGACACTTGAGGACATCCTGGCAGATGCCCCTGAGTCTCAG
AACAACTGCCGCCTCATTGCCTACCAGGAACCTGCAGATGACAGCAGCTTCTCG
CTGTCCCAGGAGGTTCTCCGGCACCTGCGGCAGGAGGAAAAGGAAGAGGTTACT
GTGGGCAGCTTGAAGACCTCAGCGGTGCCCAGTACCTCCACGATGTCCCAAGAG
CCTGAGCTCCTCATCAGTGGAATGGAAAAGCCCCTCCCTCTCCGCACGGATTTC
TCTTGA
```

SEQ IN NO: 1 as shown above includes the terminal TGA stop codon. The stop codon may be useful in expressing the protein as a discrete product. However, there might be instances where the protein is to be expressed together with at least one other protein product, e.g. as a protein fusion. In such instances, it is possible to remove the terminal TGA stop codon, replacing it with a nucleic acid sequence encoding the other protein(s) of interest. The skilled person understands under what circumstances the terminal stop codon TGA is to be retained or dispensed with, and is readily able to reproduce the above sequence in the presence or absence of the terminal stop codon TGA.

In some embodiments of the invention, the identity between STING nucleotide sequence and SEQ ID NO: 1 is at least about 54% to 100% or between about 54% and 100%. In some embodiments of the invention, the identity between STING nucleotide sequence and SEQ ID NO: 1 is at least: about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the invention, the identity between STING nucleotide sequence and SEQ ID NO: 1 is about 100%, more preferably is 100%. In view of the teaching of the present invention, the skilled person will understand that any STING nucleotide sequence having the above recited sequence identities to SEQ ID NO: 1 may be used according to the present invention, provided that such sequence still has the same qualitative or qualitative and quantitative physiological activity as STING defined by SEQ ID NO: 1. The skilled person can incorporate and express sequences comprising the above sequences in recombinant expression systems according to known methods in the art, in order to obtain recombinant STING for use in the present invention.

The amino acid sequence of human full-length STING is defined by SEQ ID NO: 2.

SEQ ID NO: 2

MPHSSLHPSIPCPRGHGAQKAALVLLSACLVTLWGLGEPPEHTLRYLVLHLAS
LQLGLLLNGVCSLAEELRHIHSRYRGSYWRTVRACLGCPLRRGALLLLSIYFY

YSLPNAVGPPFTWMLALLGLSQALNILLGLKGLAPAEISAVCEKGNFNVAHGL
AWSYYIGYLRLILPELQARIRTYNQHYNNLLRGAVSQRLYILLPLDCGVPDNL
SMADPNIRFLDKLPQQTGDHAGIKDRVYSNSIYELLENGQRAGTCVLEYATPL
QTLFAMSQYSQAGFSREDRLEQAKLFCRTLEDILADAPESQNNCRLIAYQEPA
DDSSFSLSQEVLRHLRQEEKEEVTVGSLKTSAVPSTSTMSQEPELLISGMEKP
LPLRTDFS

In some embodiments of the present invention, the identity between STING amino acid sequence and SEQ ID NO: 2 is at least about 36% to 100% or between about 36% and 100%. In some embodiments of the invention, the identity between STING amino acid sequence and SEQ ID NO: 2 is at least: about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the invention, the identity between STING amino acid sequence and SEQ ID NO: 2 is about 100%, more preferably is 100%. In view of the teaching of the present invention, the skilled person will understand that any STING amino acid sequence having the above recited sequence identities to SEQ ID NO: 2 may be used according to the present invention, provided that such sequence still has the same qualitative or qualitative and quantitative physiological activity as STING defined by SEQ ID NO: 2. The skilled person can incorporate and express sequences comprising the above sequence identities to SEQ ID NO: 2 in recombinant expression systems according to known methods in the art, in order to obtain recombinant STING for use in the present invention.

In another preferred embodiment of the present invention, the nucleotide sequence of full-length STING is as defined by SEQ ID NO: 3. This sequence encodes a human STING variant which is derived from a macrophage cell line having an arginine to histidine substitution at position 220 (R220H) and a histidine to arginine substitution at position 232 (H232R). The $STING^{H220R232}$ variant was shown to have greater affinity to 2'3' cyclic dinucleotides generated by cGAS than wild-type STING encoded by SEQ ID NO: 1, represented by SEQ ID NO: 2.

SEQ ID NO: 3

ATGCCCCACTCCAGCCTGCATCCATCCATCCCGTGTCCCAGGGGTCACGGGGC
CCAGAAGGCAGCCTTGGTTCTGCTGAGTGCCTGCCTGGTGACCCTTTGGGGGC
TAGGAGAGCCACCAGAGCACACTCTCCGGTACCTGGTGCTCCACCTAGCCTCC
CTGCAGCTGGGACTGCTGTTAAACGGGGTCTGCAGCCTGGCTGAGGAGCTGCG
CCACATCCACTCCAGGTACCGGGGCAGCTACTGGAGGACTGTGCGGGCCTGCC
TGGGCTGCCCCCTCCGCCGTGGGGCCCTGTTGCTGCTGTCCATCTATTTCTAC
TACTCCCTCCCAAATGCGGTCGGCCCGCCCTTCACTTGGATGCTTGCCCTCCT
GGGCCTCTCGCAGGCACTGAACATCCTCCTGGGCCTCAAGGGCCTGGCCCCAG
CTGAGATCTCTGCAGTGTGTGAAAAAGGGAATTTCAACGTGGCCCATGGGCTG
GCATGGTCATATTACATCGGATATCTGCGGCTGATCCTGCCAGAGCTCCAGGC
CCGGATTCGAACTTACAATCAGCATTACAACAACCTGCTACGGGGTGCAGTGA
GCCAGCGGCTGTATATTCTCCTCCCATTGGACTGTGGGGTGCCTGATAACCTG
AGTATGGCTGACCCCAACATTCACTTCCTGGATAAACTGCCCCAGCAGACCGG
TGACCGTGCTGGCATCAAGGATCGGGTTTACAGCAACAGCATCTATGAGCTTC
TGGAGAACGGGCAGCGGGCGGGCACCTGTGTCCTGGAGTACGCCACCCCCTTG
CAGACTTTGTTTGCCATGTCACAATACAGTCAAGCTGGCTTTAGCCGGGAGGA
TAGGCTTGAGCAGGCCAAACTCTTCTGCCGGACACTTGAGGACATCCTGGCAG
ATGCCCCTGAGTCTCAGAACAACTGCCGCCTCATTGCCTACCAGGAACCTGCA
GATGACAGCAGCTTCTCGCTGTCCCAGGAGGTTCTCCGGCACCTGCGGCAGGA
GGAAAAGGAAGAGGTTACTGTGGGCAGCTTGAAGACCTCAGCGGTGCCCAGTA
CCTCCACGATGTCCCAAGAGCCTGAGCTCCTCATCAGTGGAATGGAAAAGCCC
CTCCCTCTCCGCACGGATTTCTCTTGA

SEQ IN NO: 3 as shown above includes the terminal TGA stop codon. The stop codon may be useful in expressing the protein as a discrete product. However, there might be instances where the protein is to be expressed together with at least one other protein product, e.g. as a protein fusion. In such instances, it is possible to remove the terminal TGA stop codon, replacing it with a nucleic acid sequence encoding the other protein(s) of interest. The skilled person understands under what circumstances the terminal stop codon TGA is to be retained or dispensed with, and is readily able to reproduce the above sequence in the presence or absence of the terminal stop codon TGA.

In some embodiments of the present invention, the identity between STING nucleotide sequence and SEQ ID NO: 3 is at least about 54% to 100% or between about 54% and 100%. In some embodiments of the invention, the identity between STING nucleotide sequence and SEQ ID NO: 3 is at least: about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the invention, the identity between STING nucleotide sequence and SEQ ID NO: 3 is about 100%, more preferably is 100%. In view of the teaching of the present invention, the skilled person will understand that any STING nucleotide sequence having the above recited sequence identities to SEQ ID NO: 3 may be used according to the present invention, provided that such sequence still has the same qualitative or qualitative and quantitative physiological activity as STING defined by SEQ ID NO: 3. The skilled person can incorporate and express sequences comprising the above sequence identities to SEQ ID NO: 3 in recombinant expression systems according to known methods in the art, in order to obtain recombinant STING for use in the present invention, in order to obtain recombinant STING for use in the present invention. The amino acid sequence of human full-length STING $^{H220R232}$ is as defined by SEQ ID NO: 4.

SEQ ID NO: 4

MPHSSLHPSIPCPRGHGAQKAALVLLSACLVTLWGLGEPPEHTLRYLVLHLAS
LQLGLLLNGVCSLAEELRHIHSRYRGSYWRTVRACLGCPLRRGALLLLSIYFY
YSLPNAVGPPFTWMLALLGLSQALNILLGLKGLAPAEISAVCEKGNFNVAHGL
AWSYYIGYLRLILPELQARIRTYNQHYNNLLRGAVSQRLYILLPLDCGVPDNL
SMADPNIHFLDKLPQQTGDRAGIKDRVYSNSIYELLENGQRAGTCVLEYATPL
QTLFAMSQYSQAGFSREDRLEQAKLFCRTLEDILADAPESQNNCRLIAYQEPA
DDSSFSLSQEVLRHLRQEEKEEVTVGSLKTSAVPSTSTMSQEPELLISGMEKP
LPLRTDFS

In some embodiments of the present invention, the identity between STING amino acid sequence and SEQ ID NO: 4 is at least about 36% to 100% or between about 36% and 100%. In some embodiments of the invention, the identity between STING amino acid sequence and SEQ ID NO: 4 is at least: about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the invention, the identity between STING amino acid sequence and SEQ ID NO: 4 is about 100%, more preferably is 100%. In view of the teaching of the present invention, the skilled person will understand that any STING amino acid sequence having the above recited sequence identities to SEQ ID NO: 4 may be used according to the present invention, provided that such sequence still has the same qualitative or qualitative and quantitative physiological activity as STING defined by SEQ ID NO: 4. The skilled person can incorporate and express sequences comprising the above sequence identities to SEQ ID NO: 4 in recombinant expression systems according to known methods in the art, in order to obtain recombinant STING for use in the present invention. As can be seen from Figure 2 of the description, human STING comprises an endoplasmic reticulum localization signal (1-36 aa) followed by transmembrane domain (TM) at the N-terminus (40-135 aa), a highly conserved cyclic dinucleotide binding domain (CBD) at the C-terminus (139-344 aa) which is sufficient for CDN binding and a C-terminal tail (CTT, 345-379) responsible for signal transduction. The inventors have found that truncated human STING consisting only of the highly conserved CBD domain has improved characteristics when used in the herein described methods of the invention. In particular, truncated human STING consisting only of CBD or of CBD and CTT has improved solubility in the aqueous solution, while retaining its capability to bind to CDN and to form STING dimers upon CDN binding. In addition, the truncated construct can efficiently produced recombinantly in soluble form in *E. coli.*

Thus, in a further preferred embodiment of the present invention, STING is a truncated version of the human full-length protein comprising the soluble CDN-binding domain CBD (139-344 aa). The nucleotide sequence of truncated human STING is defined by SEQ ID NO: 5.

<u>SEQ ID NO: 5</u>

CTGGCCCCAGCTGAGATCTCTGCAGTGTGTGAAAAAGGGAATTTCAACGTGGC
CCATGGGCTGGCATGGTCATATTACATCGGATATCTGCGGCTGATCCTGCCAG
AGCTCCAGGCCCGGATTCGAACTTACAATCAGCATTACAACAACCTGCTACGG
GGTGCAGTGAGCCAGCGGCTGTATATTCTCCTCCCATTGGACTGTGGGGTGCC
TGATAACCTGAGTATGGCTGACCCCAACATTCGCTTCCTGGATAAACTGCCCC
AGCAGACCGGTGACCATGCTGGCATCAAGGATCGGGTTTACAGCAACAGCATC
TATGAGCTTCTGGAGAACGGGCAGCGGGCGGGCACCTGTGTCCTGGAGTACGC
CACCCCCTTGCAGACTTTGTTTGCCATGTCACAATACAGTCAAGCTGGCTTTA
GCCGGGAGGATAGGCTTGAGCAGGCCAAACTCTTCTGCCGGACACTTGAGGAC
ATCCTGGCAGATGCCCCTGAGTCTCAGAACAACTGCCGCCTCATTGCCTACCA
GGAACCTGCAGATGACAGCAGCTTCTCGCTGTCCCAGGAGGTTCTCCGGCACC
TGCGGCAGGAGGAAAAGGAAGAGGTTACTGTGGGC

In some embodiments of the invention, the identity between STING nucleotide sequence and SEQ ID NO: 5 is at least about 58% to 100% or between about 58% and 100%. In some embodiments of the invention, the identity between STING nucleotide sequence and SEQ ID NO: 5 is at least: about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the invention, the identity between STING nucleotide sequence and SEQ ID NO: 5 is about 100%, more preferably is 100%. In view of the teaching of the present invention, the skilled person will understand that any STING nucleotide sequence having the above recited sequence identities to SEQ ID NO: 5 may be used according to the present invention, provided that such sequence still has the same qualitative or qualitative and quantitative physiological activity as STING defined by SEQ ID NO: 5. The skilled person can incorporate and express sequences comprising the above sequence identities to SEQ ID NO: 5 in recombinant expression systems according to known methods in the art, in order to obtain recombinant STING for use in the present invention.

The amino acid sequence of truncated human STING is defined by SEQ ID NO: 6.

SEQ ID NO: 6

LAPAEISAVCEKGNFNVAHGLAWSYYIGYLRLILPELQARIRTYNQHYNNLLR
GAVSQRLYILLPLDCGVPDNLSMADPNIRFLDKLPQQTGDHAGIKDRVYSNSI
YELLENGQRAGTCVLEYATPLQTLFAMSQYSQAGFSREDRLEQAKLFCRTLED
ILADAPESQNNCRLIAYQEPADDSSFSLSQEVLRHLRQEEKEEVTVG

In some embodiments of the present invention, the identity between STING amino acid sequence and SEQ ID NO: 6 is at least about 43% to 100% or between about 43% and 100%. In some embodiments of the invention, the identity between STING amino acid sequence and SEQ ID NO: 6 is at least: about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the invention, the identity between STING amino acid sequence and SEQ ID NO: 6 is about 100%, more preferably is 100%. In view of the teaching of the present invention, the skilled person will understand that any STING amino acid sequence having the above

recited sequence identities to SEQ ID NO: 6 may be used according to the present invention, provided that such sequence still has the same qualitative or qualitative and quantitative physiological activity as STING defined by SEQ ID NO: 6. The skilled person can incorporate and express sequences comprising the above sequence identities to SEQ ID NO: 6 in recombinant expression systems according to known methods in the art, in order to obtain recombinant STING for use in the present invention.

In a further preferred embodiment of the present invention, the nucleotide sequence of truncated human STING is defined by SEQ ID NO: 7. This sequence represents the truncated STING$^{H220R232}$ variant having a higher binding affinity for 2'3'CDN.

SEQ ID NO: 7

```
CTGGCCCCAGCTGAGATCTCTGCAGTGTGTGAAAAAGGGAATTTCAACGTGGC
CCATGGGCTGGCATGGTCATATTACATCGGATATCTGCGGCTGATCCTGCCAG
AGCTCCAGGCCCGGATTCGAACTTACAATCAGCATTACAACAACCTGCTACGG
GGTGCAGTGAGCCAGCGGCTGTATATTCTCCTCCCATTGGACTGTGGGGTGCC
TGATAACCTGAGTATGGCTGACCCCAACATTCACTTCCTGGATAAACTGCCCC
AGCAGACCGGTGACCGTGCTGGCATCAAGGATCGGGTTTACAGCAACAGCATC
TATGAGCTTCTGGAGAACGGGCAGCGGGCGGGCACCTGTGTCCTGGAGTACGC
CACCCCCTTGCAGACTTTGTTTGCCATGTCACAATACAGTCAAGCTGGCTTTA
GCCGGGAGGATAGGCTTGAGCAGGCCAAACTCTTCTGCCGGACACTTGAGGAC
ATCCTGGCAGATGCCCCTGAGTCTCAGAACAACTGCCGCCTCATTGCCTACCA
GGAACCTGCAGATGACAGCAGCTTCTCGCTGTCCCAGGAGGTTCTCCGGCACC
TGCGGCAGGAGGAAAAGGAAGAGGTTACTGTGGGC
```

In some embodiments of the invention, the identity between STING nucleotide sequence and SEQ ID NO: 7 is at least about 60% to 100% or between about 60% and 100%. In some embodiments of the invention, the identity between STING nucleotide sequence and SEQ ID NO: 7 is at least: about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the invention, the identity between STING nucleotide sequence and SEQ ID NO: 7 is about 100%, more preferably is 100%. In view of the teaching of the present invention, the skilled person will understand that any STING nucleotide sequence having the above recited sequence identities to SEQ ID NO: 7 may be used according to the present invention, provided that such sequence still has the same qualitative or qualitative and quantitative physiological activity as STING defined by SEQ ID NO: 7. The skilled person can incorporate and express sequences comprising the above sequence identities to SEQ ID NO: 7 in recombinant expression systems according to known methods in the art, in order to obtain recombinant STING for use in the present invention.

The amino acid sequence of truncated human STING$^{H220R232}$ is defined by SEQ ID NO: 8.

SEQ ID NO: 8

```
LAPAEISAVCEKGNFNVAHGLAWSYYIGYLRLILPELQARIRTYNQHYNNLLR
GAVSQRLYILLPLDCGVPDNLSMADPNIHFLDKLPQQTGDRAGIKDRVYSNSI
YELLENGQRAGTCVLEYATPLQTLFAMSQYSQAGFSREDRLEQAKLFCRTLED
ILADAPESQNNCRLIAYQEPADDSSFSLSQEVLRHLRQEEKEEVTVG
```

In some embodiments of the present invention, the identity between STING amino acid sequence and SEQ ID

NO: 8 is at least about 44% to 100% or between about 44% and 100%. In some embodiments of the invention, the identity between STING amino acid sequence and SEQ ID NO: 8 is at least: about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a range. Also contemplated within such ranges are corresponding ranges in which the lower percent identity is included while the upper percent identity is excluded, as well as ranges in which the lower percent identity is excluded while the upper percent identity is included. In a particularly preferred embodiment of the present invention, the identity between STING amino acid sequence and SEQ ID NO: 8 is about 100%, more preferably is 100%. In view of the teaching of the present invention, the skilled person will understand that any STING amino acid sequence having the above recited sequence identities to SEQ ID NO: 8 may be used according to the present invention, provided that such sequence still has the same qualitative or qualitative and quantitative physiological activity as STING defined by SEQ ID NO: 8. The skilled person can incorporate and express sequences comprising the above sequence identities to SEQ ID NO: 8 in recombinant expression systems according to known methods in the art, in order to obtain recombinant STING for use in the present invention.

In one embodiment of the present invention, the fluorescent signal of the fluorescent nucleoside monophosphate of the 2’3’fCDN is measured at a predetermined time-point. This time-point should be determined in preliminary experiments taking into account the "dead time" of the instrument used as the fluorescence reader, i.e. the shortest time it takes between mixing the reagents and measuring the first data point in a given instrument. Once the correct time point is determined for each experimental setup, measurement of the fluorescent signal at a predetermined time point allows fast and reliable measurement of the fluorescent signal of the fluorescent nucleoside monophosphate in the aqueous solution. This allows fast and reliable identification of a substance having an ability to bind to STING.

In another embodiment of the present invention, the fluorescence signal is measured continuously or at intervals over a predetermined time interval. In some embodiments of the present invention, the fluorescent signal of the fluorescent nucleoside is measured starting from about 10 seconds after mixing all compounds together up to about 1 hour after mixing all compounds together. Measuring over a time interval includes the measurement over a series of specific time points. This allows one to obtain information about the linearity of the reaction and reduces the possibility that measuring occurs at a time point when the reaction has already long been completed. The measurement over a time interval therefore reduces the possibility that the measured fluorescence signal is misinterpreted.

According to the method of the present invention, the 2’3’fCDN of the present invention binds to STING in the aqueous solution, thereby resulting in a quenched (i.e. reduced) fluorescent signal of the (at least one) fluorescent nucleoside monophosphate of the 2’3’fCDN of the present invention. A reduction of the fluorescence signal of the aqueous solution therefore directly correlates with the binding of 2’3’fCDN to STING. Thus if, after addition of a substance, the fluorescence signal measured in the presence of the substance increases compared to the fluorescence signal measured in the absence of the substance, this is an indication that previously bound 2’3’fCDN is released from STING, thereby reversing the quenching effect of STING on the fluorescence signal of bound 2’3’fCDN. An increase of the fluorescence signal in the presence of a substance therefore indicates that the 2’3’fCDN of the present invention is released from STING due to an interaction of the substance with STING. Binding of the substance to STING can either occur at the same binding site as the 2’3’fCDN of the present invention, thereby displacing the cyclic dinucleotide, or at a site different from the binding site of 2’3’fCDN, thereby causing a conformational change in STING which then releases the cyclic dinucleotide. Furthermore, the substance might prevent 2’3’fCDN binding to STING to begin with, by blocking the binding site of STING. The method according to the present invention thus provides a robust and reliable tool to identify STING ligands that interfere with 2’3’CDN binding to STING and which could therefore be a promising starting point for the development of therapeutic strategies to specifically target STING with the aim of modulating its activity.

In one embodiment of the present invention, the increase of the fluorescence signal measured in the presence of the substance compared to the fluorescence signal measured in the absence of the substance may indicate that the substance is a competitive binder of STING. A competitive binder of STING binds at the same site of the signaling molecule than 2’3’fCDN. Due to its higher binding affinity to STING, the competitive binder either displaces already bound 2’3’fCDN or blocks the binding site for 2’3’fCDN. A substance which results in an increase of the fluorescence signal of the aqueous solution may therefore compete with the 2’3’fCDN of the present invention for binding to STING.

In a further embodiment according to the present invention, the increase in the fluorescence signal measured in the presence of the substance compared to the fluorescence signal measured in the absence of the substance may indicate that the substance is a noncompetitive binder of STING. A noncompetitive binder binds to an allosteric site, other than or in addition to the binding site of 2’3’ fCDN of the present invention, thereby inducing a conformational change in STING. This conformational change can affect the complex formation between the 2’3’fCDN and STING by

either preventing binding of 2'3'fCDN to STING, or if the cyclic dinucleotide is already bound to STING, can result in a release of 2'3'fCDN due to a conformational change in the binding site which reduces the binding affinity of 2'3'fCDN to STING. Thus, according to a further embodiment of the present invention, a substance which results in an increase of the fluorescence signal of the aqueous solution may therefore bind in an uncompetitive manner to STING, resulting in a conformational change in the protein that affects the interaction between STING and the 2'3'fCDN of the present invention.

In one embodiment of the invention, the substance is added to the aqueous solution comprising STING after the addition of the 2'3'fCDN.

Due to its environmental sensitivity an unchanged fluorescence signal measured in the presence of the substance in comparison with a fluorescence signal measured in the absence of the substance might indicate that the 2'3'fCDN of the present invention did not change its location or that STING did not undergo a conformational change. In either case, an unchanged fluorescence intensity in the presence of the substance compared to the fluorescence signal measured in the absence of the substance might indicate that the 2'3'fCDN is still bound to STING, either because binding of the substance does not affect 2'3'fCDN binding to STING, or because the substance did not bind to STING or binds to STING with such a low affinity that it does not affect 2'3'fCDN binding. However, an unchanged fluorescence signal measured in the presence of the substance compared to the fluorescence signal measured in the absence of the substance might also indicate that the chemical environment of the 2'3'fCDN has changed, but that the old and new environments of 2'3'fCDN are sufficiently similar and therefore give rise to a net unchanged fluorescence signal. So based on an unchanged fluorescence signal measured in the presence of the substance compared to the fluorescence signal measured in the absence of the substance, it may not be possible to positively conclude that the substance did not change the location of the 2'3'fCDN of the present invention and thus did not bind to STING, or indeed that the substance in question bound STING at all. A measured fluorescence signal which is higher in the presence of the substance than in the absence of the substance may be taken as positive proof that the substance has an ability to bind sting, as recited in the present method of the invention. Thus, the method according to the invention, is a method for identifying a substance having an ability to bind to STING, thereby replacing the bound 2'3'fCDN and/or changing its chemical environment in a manner that the quenching effect caused by STING binding is partially or wholly reversed. The present method according to the invention may thus also be characterized as a method for identifying a substance having an ability to bind to STING and modulate, e.g. reduce, binding of the CDN, e.g. the 2'3'fCDN by STING.

In practice, one would therefore need to tune/choose the system such that a fluorescent change is correlated with a change in localization of the 2'3'fCDN of the present invention (e.g. by choosing appropriate fluorophores, or attaching them to sensitive positions at the 2'3' CDN). In principle, the skilled person could use fluorescence polarization anisotropy to measure CDN binding in the absence of any quenching effects to fine tune the method in order to allow an unambiguous interpretation of the measured fluorescence signal (Zhang H, Wu Q, Berezin MY. Fluorescence anisotropy (polarization): from drug screening to precision medicine. Expert Opin Drug Discov. 10(11):1145-61, 2015).

According to the present invention, a decrease in the fluorescent signal measured in the presence of the substance in comparison the fluorescent signal measured in the absence of the substance might indicate that the substance either further quenches the fluorescence signal of the fluorescent nucleoside monophosphate of the 2'3'fCDN or stabilizes the interaction between 2'3'fCDN and STING. For example, the substance could lower the dissociation constant resulting in a higher binding affinity of 2'3'fCDN to STING and in increased binding of the molecule to STING, thereby further reducing the measured fluorescence signal in the reaction mixture containing bound and free 2'3'fCDN .

In one embodiment of the present invention, the measured fluorescence signal of the fluorescent nucleoside monophosphate in the 2'3'fCDN is measured at different wavelengths or ranges of wavelengths depending on the fluorescent nucleoside monophosphate in the 2'3'fCDN used in the method according to the invention. Specifically, each 2'3'fCDN comprising a specific fluorescent nucleoside monophosphate may have different excitation and emission maxima which influence the wavelengths at which the fluorescence signal is measured. Thus, depending on the fluorescent nucleoside monophosphate of the 2'3'fCDN of the present invention, the wavelengths or range of wavelengths at which the fluorescent nucleoside monophosphate is excited (i.e. the excitation maxima) and the wavelengths or range of wavelengths at which the fluorescence signal is measured (i.e. the emission maxima) need to be adapted for each 2'3'fCDN of the present invention individually in order to avoid false positives by measuring a fluorescence signal outside the optimal wavelength or range of wavelengths for a given 2'3'fCDN of the present invention. In some embodiments of the present invention, the excitation wavelength is in the range of about 290nm to about 365nm, or between about 290nm and 365nm. In some embodiments of the invention, the excitation wavelength is about 290nm, about 291nm, about 292nm, about 293nm, about 294nm, about 295nm, about 296nm, about 297nm, about 298nm, about 299nm, about 300nm, about 301nm, about 302nm, about 303nm, about 304nm, about 305nm, about 306nm, about 307nm, about 308nm, about 309nm, about 310nm, about 311nm, about 312nm, about 312nm, about 313nm, about 314nm, about 315nm, about 316nm, about 317nm, about 318nm, about 319nm, about 320nm, about 321nm, about 322nm, about 323nm, is about 324nm, about 325nm, about 326nm, about 327nm, about 329nm, about 330nm, about 331nm, about 332nm, about 333nm, about 334nm, about 335nm, about 336nm, about 337nm, about 338nm, about 339nm, about

340nm, about 341nm, about 342nm, about 343nm, about 344nm, about 345nm, about 346nm, about 347nm, about 348nm, about 349nm, about 350nm, about 351nm, about 352nm, about 353nm, about 354nm, about 355nm, about 356nm, about 357nm, about 358nm, about 359nm, about 360nm, about 361nm, about 362nm, about 363nm, about 364nm, or about 365nm. Any of the above recited excitation wavelength may be combined with one another to form an excitation wavelength range. Also contemplated within such excitation wavelength ranges are corresponding ranges in which the lower excitation wavelength is included while the upper excitation wavelength is excluded, as well as ranges in which the lower excitation wavelength is excluded while the upper excitation wavelength is included. In a particularly preferred embodiment of the invention, the excitation wavelength ranges from about 305nm to about 315nm or between about 305 nm and 315nm. In a most preferred embodiment the excitation wavelength is 305nm.

In some embodiments of the present invention the emission wavelength ranges from about 345nm to about 500 nm or between about 345nm and 5000nm. In some embodiments the excitation wavelength is about 345nm, about 346nm, about 347nm, about 348nm, about 349nm, about 350nm, about 351nm, about 352nm, about 353nm, about 354nm, about 355nm, about 356nm, about 357nm, about 358nm, about 359nm, about 360nm, about 361nm, about 362nm, about 363nm, about 364nm, about 365nm, about 366nm, about 367nm, about 368nm, about 369, about 370nm, about 371nm, about 372nm, about 373nm, about 374nm, about 375nm, about 376nm, about 377nm, about 378nm, about 379nm, about 380nm, about 381nm, about 382nm, about 383nm, about 384nm, about 385nm, about 386nm, about 387nm, about 388nm, about 389nm, about 390nm, about 391nm, about 392nm, about 393nm, about 394nm, about 395nm, about 396nm, about 397nm, about 398nm, about 399nm, about 400nm, about 401nm, about 402nm, about 403nm, about 404nm, about 405nm, about 406nm, about 407nm, about 408nm, about 409nm, about 410nm, about 411nm, about 412nm, about 413nm, about 414nm, about 415nm, about 416nm, about 417nm, about 418nm, about 419nm, about 420nm, about 421nm, about 422nm, about 423nm, about 424nm, about 425nm, about 426nm, about 427nm, about 428nm, about 429nm, about 430nm, about 431nm, about 432nm, about 433nm, about 434nm, about 435nm, about 436nm, about 437nm, about 438nm, about 439nm, about 440nm, about 441nm, about 442nm, about 443nm, about 444nm, about 445nm, about 446nm, about 447nm, about 448nm, about 449nm, about 450nm, about 451nm, about 452nm, about 453nm, about 454nm, about 455nm, about 456nm, about 457nm, about 458nm, about 459nm, about 460nm, about 461nm, about 462nm, about 463nm, about 464nm, about 465nm, about 466nm, about 467nm, about 468nm, about 469nm, about 470nm, about 471nm, about 472nm, about 473nm, about 474nm, about 475nm, about 476nm, about 477nm, about 478nm, about 479nm, about 480nm, about 481nm, about 482nm, about 483nm, about 484nm, about 485nm, about 486nm, about 487nm, about 488nm, about 489nm, about 490nm, about 491nm, about 492nm, about 493nm, about 494nm, about 495nm, about 496nm, about 497nm, about 498nm, about 499nm or about 500nm. Any of the above recited emission wavelength may be combined with one another to form an emission wavelength range. Also contemplated within such emission wavelength ranges are corresponding ranges in which the lower emission wavelength is included while the upper emission wavelength is excluded, as well as ranges in which the lower emission wavelength is excluded while the upper emission wavelength is included. In a particularly preferred embodiment of the invention, the emission wavelength ranges from about 350nm to about 380nm, or between about 350nm and 380nm. In a most preferred embodiment of the invention the emission wavelength is 363nm.

Furthermore, the quantum yield of a fluorescent nucleoside monophosphate of the 2'3'fCDN the present invention may also differ depending on the experimental conditions. Thus, for each 2'3'fCDN used in the method according to the invention, the experimental conditions may be modified in order to optimize the quantum yield of the fluorescent molecule. Within the biochemical constraints of the reaction under study, tuning of pH, temperature, buffer composition could be optimized. A reference for the quantum yield can be measured e.g. in ultrapure water or a standard reaction buffer. Experimental conditions that might influence the quantum yield of the 2'3'fCDN of the present invention include but are not limited to temperature, pH, ionic strength.

The change of the fluorescence signal can be measured by any known fluorescence read out technique using a conventional plate reader or a single-cuvette photometer. In a preferred embodiment of the invention, the fluorescence signal is measured with Tecan infinite M1000 using 96-well black non-binding PS plates (Greiner Bio-One), specifically FluoroMax-P spectrofluorometer (Horiba Scientific) or similar instruments can be used.

The inventors surprisingly found out that, when incorporated into a cyclic dinucleotide of the present invention, 2-aminopurine nucleoside monophosphate (2-APMP) is still fluorescently active and possess a high fluorescence environmental sensitivity which can be exploited to measure 2'3'fCDN binding to STING. In particular, the inventors found out that the fluorescence signal of the 2'3'fCDN comprising 2-APMP is significantly quenched, i.e. reduced, resulting from the environmental change of the 2'3'fCDN of the present invention when bound to STING. The quenching of the fluorescence signal upon binding of 2'3'fCDN comprising 2-APMP to STING thus allows one to measure 2'3'fCDN binding to STING in the presence or absence of a test substance in a continuous assay using conventional fluorescence plate readers, to yield valuable information about that substance's ability to bind to STING.

Thus, in a preferred embodiment according to the method of the present invention, the 2'3'fCDN comprises 2-APMP.

In another preferred embodiment, the 2'3'fCDN has the structure

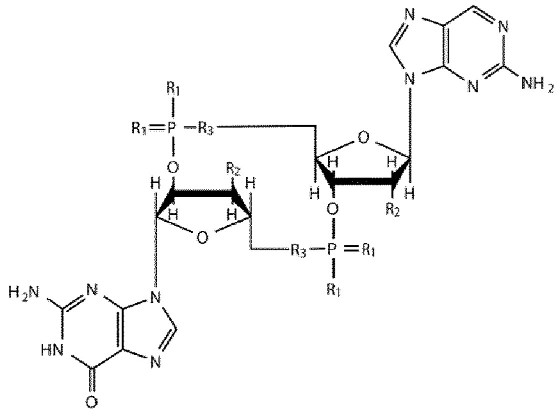

,

wherein

when $R_1$ is bound to P through a single bond, $R_1$ is independently selected from the group consisting of O, S, $BH_3$ and $CH_3$;
when $R_1$ is bound to P through a double bond, $R_1$ is independently selected from the group consisting of O, S and NH; wherein at least one $R_1$ bound to each P is O;
$R_2$ is independently selected from the group consisting of H, OH, methyl, amino-, methoxy-, fluoro-, methoxyethyl-, -O-propargyl and O-propylamine; and
$R_3$ is O.

Most preferred the 2'3'fCDN has the structure:

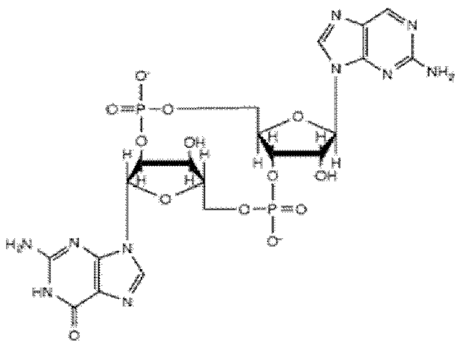

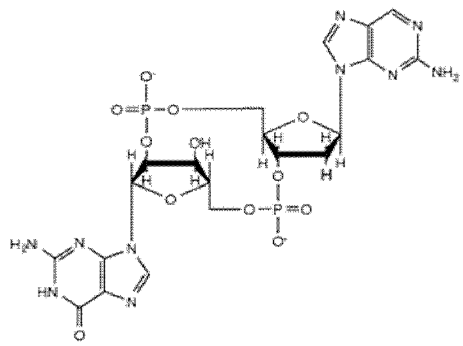

or

or

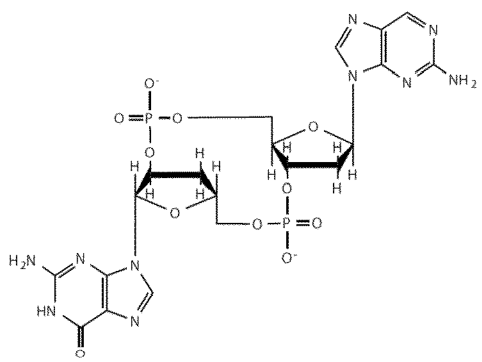

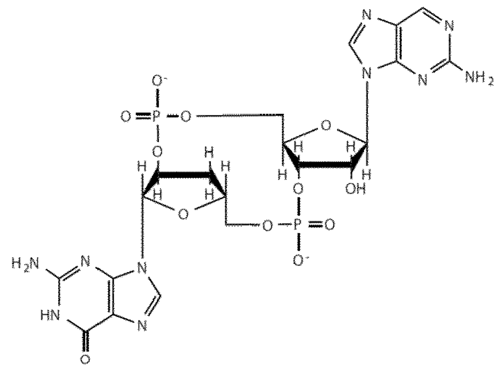

or

.

As can be seen from all of the above, the 2'3'fCDN of the present invention can advantageously be used for identifying a substance having an ability to bind to STING according to the inventive method set forth herein. Upon complex formation between STING and the cyclic dinucleotides of the present invention, the cyclic dinucleotides can

further be used to label STING.

**Method of identifying a substance having an abitlity to modulate 2'-5' phosphodiesterase activity**

Phosphodiesterases (PDE) are an important group of enzymes with an extensive functional range that are distributed widely and are highly conserved between species. The skilled person can purify any known PDE by commonly known chromatographic fractionation techniques using a cell extract and test the extract on PDE activity by the method of measuring PDE activity according to the invention. Furthermore, the skilled person will be able to identify new PDEs by way of a standard sequence comparison (e.g. BLAST) against a protein and/or nucleic acid sequence data base to identify similarities to already known PDEs.

In humans the ecto-nucleotide pyrophosphatase/phosphodiesterase 1 (ENPP1, UniProtKB Accession P22413; Human Gene Numberin Committee: 3356; Entrez Gene: 5167) is expressed in a wide range of tissues including cartilage, heart, kidney, parathyroid and collateral model, and in cells such as vascular smooth muscle cells (VSMCs), osteoblasts and chondrocytes. ENPP1 specifically cleaves the 2'-5' phosphodiesterase linkage in cyclic dinucleotides and regulates the localization, duration and amplitude of 2'3'CDNs produced by cGAS. The enzyme therefore plays an important role in the control and mediation of immune responses induced via the cGAS/STING axis. Not surprisingly, ENPP1 dysfunction is associated with a variety of human diseases. For example, mutation in the gene encoding for ENPP1 at the found to be associated with a rare type of idiopathic infantile arterial calcification demonstrating the importance of ENPP 1 in maintaining normal tissue function. Furthermore, ENPP 1 mutation have been reported to result in autosomal recessive conditions causing pre-major onset of arterial calcification resulting in stenosis and pseudoxanthoma elasticum characterized by ectopic calcification of soft connective tissue. Finally, ENPP1 mutations are associated with diabetic kidney diseases (Mackenzie, Huesa et al. "New insights into NPP1 function: Lessons from clinical and animal studies". Bone 51, 961-968, 2012; Li, Yin et al. "Hydrolysis of 2'3'-cGAMP by ENPP1 and design of nonhydrolyzable analogs". Nat Chem Biol 10, 1043-1048, 2014; Sortica, Buffon et al. "Association between the ENPP1 K121Q Polymorphism and Risk of Diabetic Kidney Disease: A Systematic Review and Meta-Analysis". PLOS ONE 10, e0118416, 2015).

Beside human PDEs, bacteria have developed several immune evasion strategies specifically targeting the CDN-based immune signaling pathways. For example, M. tuberculosis has developed a mechanism to inhibit STING-dependent type I interferon expression via a multifunctional mycobacterial phosphodiesterase CdnP. This PDE mediates hydrolysis of both bacterial-derived and host-derived CDNs thereby directly interfering with the cGAS/STING axis (Dey, Dey et al. "Inhibition of innate immune cytosolic surveillance by an M. tuberculosis phosphodiesterase". Nat Chem Biol 13, 210-217, 2017).

In view of the essential roles of PDEs in bacterial infections as well as their association with a variety of human diseases, this group of enzymes represents an attractive target for the development of new immune therapeutic approaches as well as in treatment of bacterial infections. The basic idea in the pharmaceutical industry therefore is to modulate the activity of PDE, thereby specifically controlling CDN homeostasis. In particular, it will be of great importance to identify substances having an ability to modulate PDE activity thereby providing potential vaccines against bacterial infections or substances that could be used to treat rare human diseases, such as cancer and arterial calcification.

Thus, in one aspect the present invention relates to a method for identifying a substance having an ability to modulate 2'-5' phosphodiesterase activity, wherein the method comprises the steps of (i) providing an aqueous solution comprising a cyclic dinucleotide and a 2'-5' phosphodiesterase, wherein the cyclic dinucleotide comprises a 3'-5' phosphodiester linkage and a 2'-5' phosphodiester linkage between a first nucleoside monophosphate and a second nucleoside monophosphate, wherein at least one of the first and second nucleoside monophosphates is a fluorescent nucleoside monophosphate; (ii) measuring the fluorescence signal of the aqueous solution; (iii) repeating steps (i) and (ii), wherein upon said repetition the aqueous solution further comprises the substance, and wherein the fluorescence is measured under identical or substantially identical conditions following the provision of the respective aqueous solution; and (iv) comparing the fluorescence signal measured in the presence of the substance with the fluorescence signal measured in the absence of the substance; wherein a measured fluorescence signal which is higher in the presence of the substance than in the absence of the substance indicates that the substance is a 2'-5' phosphodiesterase agonist, while a measured fluorescence signal which is lower in the presence of the substance compared to the absence of the substance indicates that the substance is a 2'-5' phosphodiesterase antagonist.

In one embodiment of the invention, the substance is added to the aqueous solution comprising a 2'-5' phosphodiesterase after the addition of the 2'3'fCDN.

In one embodiment of the present invention, an increase of the measured fluorescence signal in the presence of the substance compared to the fluorescence signal measured in the absence of the substance, indicates that the substance activates (partially or fully) or enhances 2'-5' phosphodiesterase activity, thereby resulting in increased cleavage of the 2'3' fluorescent dinucleotide (2'3'fCDN) of the present invention. Cleavage of the 2'-5' and/or 3'-5' phosphodiester linkages results in the release of the first and the second nucleoside monophosphates. The release of the fluorescent nucleoside monophosphate of the present invention partially or wholly reduces the quenching effect previously caused

by the incorporation of the free fluorescent nucleoside into the 2'3'fCDN of the present invention by cGAS. Thus, an increase of the fluorescent signal measured in the presence of the substance in comparison to the fluorescence signal measured in the absence of the substance directly correlates with an increased cleavage of 2'3'fCDN of the present invention and indicates that the substance has an ability to activate (partially or fully) or enhance 2'-5' phosphodiesterase activity. In this case, the substance is thus a 2'-5' phosphodiesterase agonist.

According to another embodiment of the present invention, a decrease in the fluorescence signal in the presence of the substance compared to the fluorescence signal measured in the absence of the substance indicates that the substance inhibits (partially or fully) 2'-5' phosphodiesterase. A decrease in the measured fluorescent signal in the presence of the substance indicates that less 2'3'fCDN of the present invention is cleaved by the 2'-5' phosphodiesterase, resulting in more fluorescent nucleoside monophosphate kept incorporated in 2'3'fCDN which directly correlates with quenching of the measured fluorescent signal. Thus, a decreased fluorescence signal measured in the presence of the substance compared to the absence of the substance indicates that less fluorescent nucleoside monophosphate is released from 2'3'fCDN due to reduced 2'-5' phosphodiesterase activity. The substance therefore acts as a 2'-5' phosphodiesterase antagonist.

In a further embodiment of the present invention, the fluorescent signal of the fluorescent nucleoside monophosphate in the 2'3'fCDN is measured at a predetermined time-point. This time-point should be determined in preliminary experiments taking into account the dead time of the instrument used as the fluorescence reader, i.e. the shortest time it takes between mixing the reagents and measuring the first data point in a given instrument. Once the correct time point is determined for each experimental set up, measuring of the fluorescent signal at a predetermined time point allows fast and reliable identification of a substance having an ability to modulate 2'-5' phosphodiesterase activity. Measuring over a time interval includes continuous measurement, and the measurement of a fluorescence signal over a series of discrete time points. This allows one to obtain information about the linearity of the reaction and reduces the possibility that the measurement occurs at a time point when the reaction might already have been completed. The measurement over a time interval may therefore reduce the risk that the measured fluorescence signal is misinterpreted.

In another embodiment of the present invention, the fluorescence signal is measured over a predetermined time interval. The advantage is a single readout that does not need to be processed further. In some embodiments of the present invention, the fluorescent signal is measured starting from about 10 seconds from mixing all compounds together up to about 1 hour. The advantage is that measuring over a time interval, the slope is a more precise readout than a single time point and also shows the linearity of the reaction. The time interval of the measurement depends strongly on 2'-5' phosphodiesterase activity in the presence of particular fluorescent 2'3'fCDN as substrate and on the experimental setup, such as buffer condition, temperature and substance concentration. In general, for the low 2'-5' phosphodiesterase activity higher protein and 2'3'fCDN concentrations may be used, compared to the case of high 2'-5' phosphodiesterase activity to perform the measurement within 5 min - 1 h time interval.

In a preferred embodiment according to the method of the present invention, the 2'3'fCDN comprises 2-APMP.

In another preferred embodiment, the 2'3'fCDN has the structure

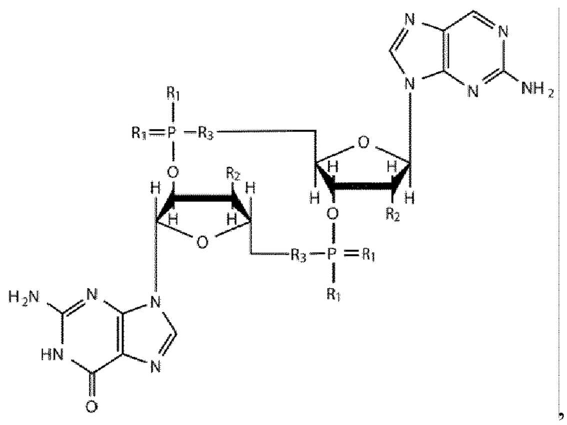

,

wherein

when $R_1$ is bound to P through a single bond, $R_1$ is independently selected from the group consisting of O, S, $BH_3$ and $CH_3$;

when $R_1$ is bound to P through a double bond, $R_1$ is independently selected from the group consisting of O, S and NH; wherein at least one $R_1$ bound to each P is O;

$R_2$ is independently selected from the group consisting of H, OH, methyl, amino-, methoxy-, fluoro-, methoxyethyl-,

-O-propargyl and O-propylamine; and
$R_3$ is O.

Most preferred the 2'3'fCDN has the structure:

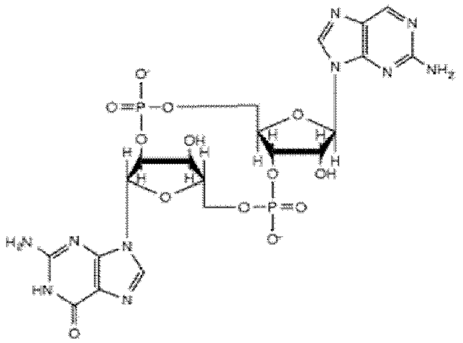

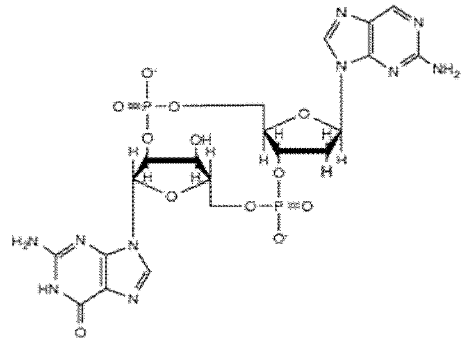

or

or

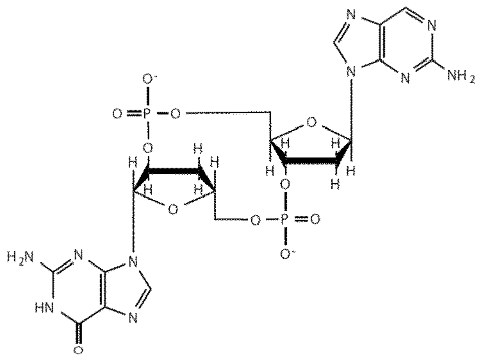

or

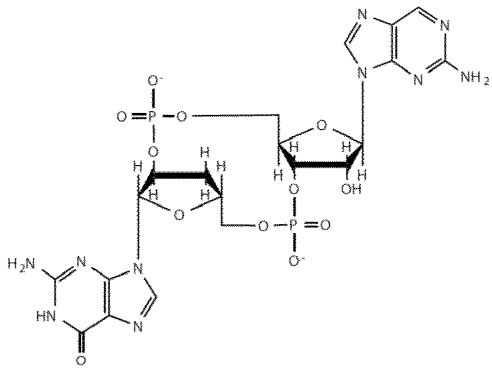

.

In one embodiment of the invention, the reaction is carried out by incubating 2'3'fCDN with a PDE. The change of the fluorescence signal is than measured in a commonly known fluorescence reader. Suitable cofactors (typical metal ions $Mn^{2+}$, $Mg^{2+}$, $Zn^{2+}$ depending on the particular PDE) can be added to the reaction mixture. In case the PDE under investigation is specific for 2'-5' or 3-5' phosphodiesters, an PDE with the other specificity would be added as well, to ensure that the CDN is cleaved into two single nucleoside monophosphates.

In view of all of the above, the 2'3'fCDN of the present invention can be used in a method of identifying a substance having an ability to modulate 2'-5' phosphodiesterase activity.

**Methods of (a) measuring cGAS activity and of (b) identifying a substance having an ability to modulate cGAS activity**

**(a) Method of measuring cGAS activity**

The present invention is based, in part, on the surprising finding that cGAS can incorporate a fluorescent nucleobase analogue from a fluorescent nucleoside triphosphate into a cyclic dinucleotide which comprises a conventional 3'-5' phosphodiester linkage and an unconventional 2'-5' phosphodiester linkage, resulting in 2'3' fluorescent dinucleotides (2'3'fCDN). The inventors have found that the fluorescence signal of the fluorescent nucleobase analogue of the free fluorescent nucleoside triphosphate in aqueous solution is quenched, i.e. reduced, upon incorporation of the fluorescent nucleoside into a 2'3'fCDN of the present invention. Thus, the change in the measured fluorescence signal in aqueous solution over time can be used to measure cGAS activity.

Thus, a further aspect of the present invention relates to a method of measuring cGAS activity, wherein the method comprises the steps of (i) providing an aqueous solution comprising cGAS, a cGAS-activating nucleic acid, a first nucleoside triphosphate, a second nucleoside triphosphate, and one or more divalent cation, wherein at least one of the first and second nucleoside triphosphates is a fluorescent nucleoside triphosphate and wherein one of the first and second nucleoside triphosphates has a free 2' hydroxyl group and the other one has a free 3' hydroxyl group and

(ii) measuring the change of the fluorescence signal of the aqueous solution over time. Measurement may be continuous or discontinuous, i.e. at intervals, over a predetermined time interval.

In one embodiment of the present invention, the method of measuring cGAS activity can be used to study cGAS variants and their efficiency in converting nucleoside triphosphates into 2'3'CDNs of the present invention. For example, the claimed method allows for the identification of engineered cGAS variants having increased or decreased cGAS activity compared to unmodified cGAS. The engineered cGAS variants can be used to study polymorphisms in the gene encoding cGAS that are associated with different diseases. For instance, it may be possible to analyze the enzymatic activity of cGAS encoded by polymorphic genes only found in a particular disease, thereby shedding light on the possible genetic mechanisms underlying said disease. Knowing the genetic mechanism related to cGAS dysfunction in a particular disease could serve as a promising starting point for the development of a therapeutic treatment.

In a further embodiment of the present invention, the method of measuring cGAS activity can be used e.g. to determine any of (a) the start and end points of measuring the fluorescence signal of the aqueous solution in the presence of different cGAS variants, (b) the effect of different nucleic acids on cGAS activation, (c) the optimal time interval required for measuring the change of the fluorescence signal of the aqueous solution, and (d) the optimal starting concentrations of the reaction partners. Furthermore, the method of measuring cGAS activity can be used to determine the standard fluorescence signal of a given fluorescent nucleoside triphosphate in aqueous solution under predetermined conditions. Thus, the method of measuring cGAS activity according to the present invention can be used to identify optimal reaction parameters which could then be used in the methods of the invention described below. The methods of measuring cGAS activity according to the present invention therefore also serve to calibrate and fine tune the experimental reaction setups for the methods of the invention described below.

In one embodiment of the present invention, cGAS activity is calculated based on the time-dependent change of the measured fluorescence signal of the aqueous solution. In particular, cGAS activity may be calculated by continuously measuring the change in the fluorescence signal of the aqueous solution in a plate reader or in a single cuvette. The obtained fluorescence curve may then be inverted into a curve representing cGAS activity (see e.g. Figure 7d). The initial slope of the curve representing cGAS activity may then be used to calculate the reaction velocity, which is one way of defining cGAS activity according to the present invention. This particular embodiment has the advantage of determining the linear portion of the curve representing cGAS activity which is not possible when using commonly known techniques such as mass spectrometry or radioactivity. The determination of the initial slope is a more precise measure of activity than measuring end points, few or single-time points. The slope, due to many more individual data points is statistically more significant and allows the determination of the linear range of the reaction, before substrate depletion or product inhibition slows down the reaction. A single point or few points of measurement are typically not sufficient to determine the linear range of a reaction, therefore increasing the possibility to underestimate the activity. In view of the above, the method of measuring cGAS activity can thus be used to optimize and fine-tune cGAS measurement conditions for other cGAS-related methods of the invention, e.g. for optimizing cGAS measurement conditions in a method of identifying a substance having an ability to modulate cGAS activity as described below in more detail.

**(b) Method of identifying a substance having an ability to modulate cGAS activity**

As discussed above, cGAS is a key sensor for cytosolic DNA in the innate immune system which elicits a signaling cascade that triggers the host response via production by cyclic dinucleotides comprising a conventional 3'-5' phosphodiester linkage and an unconventional 2'-5' phosphodiester linkage between two nucleoside monophosphates (2'3'CDN). The cyclic dinucleotides generated by cGAS bind to STING, thereby triggering the expression of type I interferon production. While under normal conditions the cGAS/STING axis protects the cell from bacterial and viral DNA infections, impairment of this pathway is associated with a variety of different diseases, such as inflammatory diseases and autoimmune disorders. For example, uncontrolled expression of type-I interferon due to constitutively active cGAS/STING is associated with various autoimmune disorders including systemic lupus erythematosus (SLE) and Aicardi-Goutières Syndrome (AGS). Thus, the identification of substances having the ability to act as cGAS/STING antagonists would be of great importance for the development of therapeutic treatments against such diseases.

In tumor cells the innate immune response is usually silenced allowing the tumor to proliferate and spread. The general idea in anti-cancer therapy is therefore to specifically trigger the cGAS/STING pathway in cancer cells, thereby eliciting a type I interferon immune response. Such an immune response could then instruct the immune system not only to attack the cancer cells but also to develop longer lasting immunogenic memory T cells against the tumor. Many cancer types developed strategies to become immunologically invisible and have for instance no T cell infiltrates, rendering anti-cancer immunity unsuccessful.

In view of the above, the identification of substances having an ability to modulate cGAS activity therefore represents an emerging technology for the development of new therapeutic strategies for the treatment of immunological disorders and cancer.

A further aspect of the present invention therefore relates to a method of identifying a substance having an

ability to modulate the activity of cGAS, wherein the method comprises the steps of (i) providing an aqueous solution comprising cGAS, cGAS-activating nucleic acid, a first nucleoside triphosphate, a second nucleoside triphosphate, and one or more divalent cation, wherein at least one of the first and second nucleoside triphosphates is a fluorescent nucleoside triphosphate, and wherein one of the first and second nucleoside triphosphates has a free 2' hydroxyl group and the other one has a free 3' hydroxyl group; (ii) measuring the fluorescence signal of the aqueous solution; (iii) repeating steps (i) and (ii), wherein upon said repetition the aqueous solution further comprises the substance, and wherein the fluorescence signal is measured under identical or substantially identical conditions following the provision of the respective aqueous solution; and (iv) comparing the fluorescence signal measured in the presence of the substance with the fluorescence signal measured in the absence of the substance; wherein a measured fluorescence signal which is higher in the presence of the substance than in the absence of the substance indicates that the substance is a cGAS antagonist, while a measured fluorescence signal which is lower in the presence of the substance than in the absence of the substance indicates that the substance is a cGAS agonist.

In one embodiment of the invention, the substance is added to the aqueous solution comprising cGAS, cGAS-activating nucleic acid and one or more divalent cation after the addition of the first and second nucleoside triphosphates.

In one embodiment of the present invention, an increase of the measured fluorescence signal in the presence of the substance compared to the fluorescence signal measured in the absence of the substance, indicates that less fluorescent nucleoside triphosphate is incorporated into 2'3'fCDN resulting in a higher concentration of free fluorescent nucleoside triphosphate in the aqueous solution. Upon incorporation of the fluorescent nucleobase analogue from the fluorescent nucleotide triphosphate into 2'3'fCDN by cGAS, the fluorescent signal of the fluorescent nucleobase analogue is quenched resulting in a decreased fluorescence of the fluorescent nuceobase analogue from the fluorescent nucleoside triphosphate. Thus, a lower fluorescence signal correlates with increased incorporation of the fluorescent nucleobase analogue from the fluorescent nucleotide triphosphate into 2'3'fCDN, whereas a higher fluorescence signal correlates with less incorporation of the fluorescent nucleobase analogue from the fluorescent nucleotide triphosphate into 2'3'fCDN. The higher comparative amount of the measured fluorescence signal is therefore a direct indication for reduced incorporation of the fluorescent nucleoside triphosphate into 2'3'fCDN. Since the reduced incorporation of the fluorescent nucleoside triphosphate into the 2'3'fCDN is directly associated with reduced cGAS activity in the presence of a substance, the increased fluorescence signal allows for identification of a substance having an inhibitory effect on cGAS activity. An increase in the measured fluorescence signal in the presence of the substance compared to the fluorescence signal measured in the absence of the substance therefore indicates that the substance is a cGAS antagonist.

According to another embodiment of the present invention, a decrease of the measured fluorescence signal in the presence of the substance compared to the fluorescence signal measured in the absence of the substance indicates that more fluorescent nucleoside triphosphate is incorporated into 2'3'fCDN of the present invention which results in a reduced concentration of free fluorescent nucleoside triphosphate in the aqueous solution. Due to its environmental sensitivity, the fluorescence signal of the fluorescent nucleoside triphosphate is quenched upon incorporation of the nucleoside into the 2'3'fCDN of the present invention, which results in a decrease in the measured fluorescence signal of the aqueous solution. Thus, a decrease in the measured fluorescence signal in the presence of the substance compared to the fluorescence signal measured in the absence of the substance indicates that cGAS converts more free fluorescent nucleoside triphosphate into 2'3'fCDN in the presence of the substance than in the absence of the substance. A decrease of the measured fluorescence signal in the presence of the substance therefore indicates that the substance activates (partially or fully) or enhances cGAS enzymatic activity; in this case, the substance is thus a cGAS agonist.

According to the present invention, cGAS is a recombinant protein obtained by conventional cloning and expression systems. A typical set of cloning and expression systems includes *E. coli,* Hi5 or SF9 insect cells and e.g. baculuvirus based expression plasmids or non-viral insect cells expression system (e.g. transient transfection to S2 insect cell line using ExpreS2), Saccharomyces cerevisiae, Pichia pastoris, mammalian cell lines (e.g. HEK293T, CHO). The protein can also be generated using cell free transcription/translation or translation systems (e.g. based on wheat germ). cGAS can be expressed as full-length protein or as a truncated version comprising the catalytic domain. Furthermore, cGAS can be engineered to have alteration in its amino acid sequence. For example, cGAS can be engineered in its active catalytic domain resulting in increased or decreased enzymatic activity of cGAS compared to the activity of unmodified cGAS. Exemplarily techniques to engineer cGAS include but are not limited to site directed mutagenesis using polymerase chain reaction, cassette ligation, ligation-during-amplification.

The protein may further contain affinity tags allowing purification and detection of the expressed protein.

According to the methods of the invention, cGAS is derived from any vertebrate, including mammals such as human, macaque, mouse, sus, bos, and the like, chicken, duck, zebra fish and *Xenopus laevis.*

In a preferred embodiment of the invention, cGAS is derived from human. The nucleotide sequence of full-length human cGAS is defined by SEQ ID NO: 9.

SEQ ID NO: 9

```
ATGCAGCCTTGGCACGGAAAGGCCATGCAGAGAGCTTCCGAGGCCGGAGCCAC
TGCCCCCAAGGCTTCCGCACGGAATGCCAGGGGCGCCCCGATGGATCCCACCG
AGTCTCCGGCTGCCCCCGAGGCCGCCCTGCCTAAGGCGGGAAAGTTCGGCCCC
GCCAGGAAGTCGGGATCCCGGCAGAAAAAGAGCGCCCCGGACACCCAGGAGAG
GCCGCCCGTCCGCGCAACTGGGGCCCGCGCCAAAAAGGCCCCTCAGCGCGCCC
AGGACACGCAGCCGTCTGACGCCACCAGCGCCCCTGGGGCAGAGGGGCTGGAG
CCTCCTGCGGCTCGGGAGCCGGCTCTTTCCAGGGCTGGTTCTTGCCGCCAGAG
GGGCGCGCGCTGCTCCACGAAGCCAAGACCTCCGCCCGGGCCCTGGGACGTGC
CCAGCCCCGGCCTGCCGGTCTCGGCCCCCATTCTCGTACGGAGGGATGCGGCG
CCTGGGGCCTCGAAGCTCCGGGCGGTTTTGGAGAAGTTGAAGCTCAGCCGCGA
TGATATCTCCACGGCGGCGGGGATGGTGAAAGGGGTTGTGGACCACCTGCTGC
TCAGACTGAAGTGCGACTCCGCGTTCAGAGGCGTCGGGCTGCTGAACACCGGG
AGCTACTATGAGCACGTGAAGATTTCTGCACCTAATGAATTTGATGTCATGTT
TAAACTGGAAGTCCCCAGAATTCAACTAGAAGAATATTCCAACACTCGTGCAT
ATTACTTTGTGAAATTTAAAAGAAATCCGAAAGAAAATCCTCTGAGTCAGTTT
TTAGAAGGTGAAATATTATCAGCTTCTAAGATGCTGTCAAAGTTTAGGAAAAT
CATTAAGGAAGAAATTAACGACATTAAAGATACAGATGTCATCATGAAGAGGA
AAAGAGGAGGGAGCCCTGCTGTAACACTTCTTATTAGTGAAAAAATATCTGTG
GATATAACCCTGGCTTTGGAATCAAAAAGTAGCTGGCCTGCTAGCACCCAAGA
AGGCCTGCGCATTCAAAACTGGCTTTCAGCAAAAGTTAGGAAGCAACTACGAC
TAAAGCCATTTTACCTTGTACCCAAGCATGCAAAGGAAGGAAATGGTTTCCAA
GAAGAAACATGGCGGCTATCCTTCTCTCACATCGAAAAGGAAATTTTGAACAA
TCATGGAAAATCTAAAACGTGCTGTGAAAACAAAGAAGAGAAATGTTGCAGGA
AAGATTGTTTAAAACTAATGAAATACCTTTTAGAACAGCTGAAAGAAAGGTTT
AAAGACAAAAAACATCTGGATAAATTCTCTTCTTATCATGTGAAAACTGCCTT
CTTTCACGTATGTACCCAGAACCCTCAAGACAGTCAGTGGGACCGCAAAGACC
TGGGCCTCTGCTTTGATAACTGCGTGACATACTTTCTTCAGTGCCTCAGGACA
GAAAAACTTGAGAATTATTTTATTCCTGAATTCAATCTATTCTCTAGCAACTT
AATTGACAAAAGAAGTAAAGAATTTCTGACAAAGCAAATTGAATATGAAAGAA
ACAATGAGTTTCCAGTTTTTGATGAATTTTGA
```

SEQ IN NO: 9 as shown above includes the terminal TGA stop codon. The stop codon may be useful in expressing the protein as a discrete product. However, there might be instances where the protein is to be expressed together with at least one other protein product, e.g. as a protein fusion. In such instances, it is possible to remove the terminal TGA stop codon, replacing it with a nucleic acid sequence encoding the other protein(s) of interest. The skilled person understands under what circumstances the terminal stop codon TGA is to be retained or dispensed with, and is readily able to reproduce the above sequence in the presence or absence of the terminal stop codon TGA.

In some embodiments of the invention, the identity between cGAS nucleotide sequence and SEQ ID NO: 9 is at least about 50% to about 100% or between about 50% and about 100%. In some embodiments of the invention, the identity between cGAS nucleotide sequence and SEQ ID NO: 9 is at least: about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a percent identity range. Also contemplated within such percent identity ranges are corresponding ranges in which the lower percent identity is included while the upper percent identity is excluded, as well as ranges in which the lower percent identity is excluded while the upper percent identity is included. In a particularly preferred embodiment of the invention, the identity between cGAS nucleotide sequence and SEQ ID NO: 9 is about 100%, more preferably is 100%. In view of the teaching of the present invention, the skilled

person will understand that any cGAS nucleotide sequence having the above recited sequence identities to SEQ ID NO: 9 may be used according to the present invention, provided that such sequence still has the same qualitative or qualitative and quantitative enzymatic activity as cGAS defined by SEQ ID NO: 9. The skilled person can incorporate and express sequences comprising the above recited sequence identities to SEQ ID NO: 9 in recombinant expression systems according to known methods in the art, in order to obtain recombinant cGAS for use in the present invention.

The amino acid sequence of full-length human cGAS is defined by SEQ ID NO: 10.

SEQ ID NO: 10

```
MQPWHGKAMQRASEAGATAPKASARNARGAPMDPTESPAAPEAALPKAGKFGP
ARKSGSRQKKSAPDTQERPPVRATGARAKKAPQRAQDTQPSDATSAPGAEGLE
PPAAREPALSRAGSCRQRGARCSTKPRPPPGPWDVPSPGLPVSAPILVRRDAA
PGASKLRAVLEKLKLSRDDISTAAGMVKGVVDHLLLRLKCDSAFRGVGLLNTG

SYYEHVKISAPNEFDVMFKLEVPRIQLEEYSNTRAYYFVKFKRNPKENPLSQF
LEGEILSASKMLSKFRKIIKEEINDIKDTDVIMKRKRGGSPAVTLLISEKISV
DITLALESKSSWPASTQEGLRIQNWLSAKVRKQLRLKPFYLVPKHAKEGNGFQ
EETWRLSFSHIEKEILNNHGKSKTCCENKEEKCCRKDCLKLMKYLLEQLKERF
KDKKHLDKFSSYHVKTAFFHVCTQNPQDSQWDRKDLGLCFDNCVTYFLQCLRT
EKLENYFIPEFNLFSSNLIDKRSKEFLTKQIEYERNNEFPVFDEF
```

In some embodiments of the invention, the identity between cGAS amino acid sequence and SEQ ID NO: 10 is at least about 36% to about 100% or between about 36% and about 100%. In some embodiments of the invention, the identity between cGAS amino acid sequence and SEQ ID NO: 10 is at least: about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a percent identity range. Also contemplated within such percent identity ranges are corresponding ranges in which the lower percent identity is included while the upper percent identity is excluded, as well as ranges in which the lower percent identity is excluded while the upper percent identity is included. In a particularly preferred embodiment of the invention, the identity between cGAS amino acid sequence and SEQ ID NO: 10 is about 100%, more preferably is 100%. In view of the teaching of the present invention, the skilled person will understand that any cGAS amino acid sequence having the above recited sequence identities to SEQ ID NO: 10 may be used according to the present invention, provided that such sequence still has the same qualitative or qualitative and quantitative enzymatic activity as cGAS defined by SEQ ID NO: 10. The skilled person can incorporate and express sequences comprising the above recited sequence identities to SEQ ID NO: 10 in recombinant expression systems according to known methods in the art, in order to obtain recombinant cGAS for use in the present invention.

As can be seen from Figure 3 human cGAS comprises a highly conserved C-terminal domain (catalytic domain - CD, aa 160-513) containing a Mab21 domain (aa 213 to 513 of human cGAS) and comprising both DNA-binding and enzymatic activity. The N-terminus of human cGAS is unstructured and not well conserved. The inventors found out that truncated human cGAS consisting of the highly conserved C-terminal domain (i.e. aa 155 to 522 of human cGAS) but missing the unstructured N-terminal domain has improved characteristics when used in the herein described methods of the invention. Specifically, truncated cGAS is less prone to proteolytic degradation and aggregation.

Thus, in a further preferred embodiment of the invention cGAS is a truncated version of the human full length protein, wherein the first 154 amino acid sequence encoding for the unstructured N-terminal domain of human cGAS is missing. The nucleotide sequence of truncated human cGAS is defined by SEQ ID NO: 11.

SEQ ID NO: 11

```
CGGAGGGATGCGGCGCCT
GGGGCCTCGAAGCTCCGGGCGGTTTTGGAGAAGTTGAAGCTCAGCCGCGATGA
TATCTCCACGGCGGCGGGGATGGTGAAAGGGGTTGTGGACCACCTGCTGCTCA
GACTGAAGTGCGACTCCGCGTTCAGAGGCGTCGGGCTGCTGAACACCGGGAGC
TACTATGAGCACGTGAAGATTTCTGCACCTAATGAATTTGATGTCATGTTTAA
ACTGGAAGTCCCCAGAATTCAACTAGAAGAATATTCCAACACTCGTGCATATT
ACTTTGTGAAATTTAAAAGAAATCCGAAAGAAAATCCTCTGAGTCAGTTTTTA
GAAGGTGAAATATTATCAGCTTCTAAGATGCTGTCAAAGTTTAGGAAAATCAT
TAAGGAAGAAATTAACGACATTAAAGATACAGATGTCATCATGAAGAGGAAAA
GAGGAGGGAGCCCTGCTGTAACACTTCTTATTAGTGAAAAAATATCTGTGGAT
ATAACCCTGGCTTTGGAATCAAAAAGTAGCTGGCCTGCTAGCACCCAAGAAGG
CCTGCGCATTCAAAACTGGCTTTCAGCAAAAGTTAGGAAGCAACTACGACTAA
AGCCATTTTACCTTGTACCCAAGCATGCAAAGGAAGGAAATGGTTTCCAAGAA
GAAACATGGCGGCTATCCTTCTCTCACATCGAAAAGGAAATTTTGAACAATCA
TGGAAAATCTAAAACGTGCTGTGAAAACAAAGAAGAGAAATGTTGCAGGAAAG
ATTGTTTAAAACTAATGAAATACCTTTTAGAACAGCTGAAAGAAAGGTTTAAA
GACAAAAAACATCTGGATAAATTCTCTTCTTATCATGTGAAAACTGCCTTCTT
TCACGTATGTACCCAGAACCCTCAAGACAGTCAGTGGGACCGCAAAGACCTGG
GCCTCTGCTTTGATAACTGCGTGACATACTTTCTTCAGTGCCTCAGGACAGAA
AAACTTGAGAATTATTTTATTCCTGAATTCAATCTATTCTCTAGCAACTTAAT
TGACAAAAGAAGTAAAGAATTTCTGACAAAGCAAATTGAATATGAAAGAAACA
ATGAGTTTCCAGTTTTTGATGAATTTTGA
```

SEQ IN NO: 11 as shown above includes the terminal TGA stop codon. The stop codon may be useful in expressing the protein as a discrete product. However, there might be instances where the protein is to be expressed together with at least one other protein product, e.g. as a protein fusion. In such instances, it is possible to remove the terminal TGA stop codon, replacing it with a nucleic acid sequence encoding the other protein(s) of interest. The skilled person understands under what circumstances the terminal stop codon TGA is to be retained or dispensed with, and is readily able to reproduce the above sequence in the presence or absence of the terminal stop codon TGA.

In some embodiments of the invention, the identity between truncated cGAS nucleotide sequence and SEQ ID NO: 11 is about 56% to about 100% or between about 56% and about 100%. In some embodiments of the invention, the identity between truncated cGAS nucleotide sequence and SEQ ID NO: 11 is at least: about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form percent identity a range. Also contemplated within such percent identity ranges are corresponding ranges in which the lower percent identity is included while the upper percent identity is excluded, as well as ranges in which the lower percent identity is excluded while the upper percent identity is included. In a particularly preferred embodiment of the invention, the identity between truncated cGAS nucleotide sequence and SEQ ID NO: 11 is about 100%, more preferably is 100%. In view of the teaching of the present invention, the skilled person will understand that any cGAS nucleotide sequence having the above recited sequence identities to SEQ ID NO: 11 may be used according to the present invention, provided that such sequence still has the same qualitative or qualitative and quantitative enzymatic activity as cGAS defined by SEQ ID NO: 11. The skilled person can incorporate and express sequences comprising the above recited sequence identities to SEQ ID NO: 11 in recombinant expression systems according to known methods in the art, in order to obtain recombinant cGAS for use in the present invention.

The amino acid sequence of truncated cGAS is defined by SEQ ID NO: 12.

SEQ ID NO: 12

```
RRDAAPGASKLRAVLEKLKLSRDDISTAAGMVKGVVDHLLLRLKCDSAFRGVG
LLNTGSYYEHVKISAPNEFDVMFKLEVPRIQLEEYSNTRAYYFVKFKRNPKEN
PLSQFLEGEILSASKMLSKFRKIIKEEINDIKDTDVIMKRKRGGSPAVTLLIS
EKISVDITLALESKSSWPASTQEGLRIQNWLSAKVRKQLRLKPFYLVPKHAKE
GNGFQEETWRLSFSHIEKEILNNHGKSKTCCENKEEKCCRKDCLKLMKYLLEQ

LKERFKDKKHLDKFSSYHVKTAFFHVCTQNPQDSQWDRKDLGLCFDNCVTYFL
QCLRTEKLENYFIPEFNLFSSNLIDKRSKEFLTKQIEYERNNEFPVFDEF
```

In some embodiments of the invention, the identity between truncated cGAS amino acid sequence and SEQ ID NO: 12 is about 40% to about 100% or between about 40% and about 100%. In some embodiments of the invention, the identity between truncated cGAS amino acid sequence and SEQ ID NO: 12 is at least: about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, bout 85%, about 86%, about 87%, about 88%, about 89%, about 90%, about 91%, about 92%, about 93%, about 94%, about 95%, about 96%, about 97%, about 98%, about 99%; or is 100%. Any of the above recited percent identities may be combined with one another to form a percent identity range. Also contemplated within such percent identity ranges are corresponding ranges in which the lower percent identity is included while the upper percent identity is excluded, as well as ranges in which the lower percent identity is excluded while the upper percent identity is included. In a particularly preferred embodiment of the invention, the identity between truncated cGAS amino acid sequence and SEQ ID NO: 12 is about 100%, more preferably is 100%. In view of the teaching of the present invention, the skilled person will understand that any cGAS amino acid sequence having the above recited sequence identities to SEQ ID NO: 12 may be used according to the present invention, provided that such sequence still has the same qualitative or qualitative and quantitative enzymatic activity as cGAS defined by SEQ ID NO: 12. The skilled person can incorporate and express sequences comprising the above recited sequence identities to SEQ ID NO: 12 in recombinant expression systems according to known methods in the art, in order to obtain recombinant cGAS for use in the present invention.

In order to convert nucleoside triphosphates into 2'3'CDN cGAS must be activated by a cGAS-activating nucleic acid which induces complex formation of cGAS with the cGAS-activating nucleic acid, thereby activating the enzyme.

According to the methods of the invention, a cGAS-activating nucleic acid comprises double-stranded (ds) and single-stranded (ss) DNA. Furthermore, the cGAS-activating nucleic acid may be an RNA/DNA hybrid, preferably a poly(A):poly(dT) or poly(dA):poly(T) hybrid.

In a preferred embodiment of the present invention, the cGAS-activating nucleic acid, e.g double stranded DNA, may have a length at least 10 base pairs (bp). In a particular embodiment of the present invention, the cGAS-activating nucleic acid, e.g. dsDNA, may have a length selected from the group consisting of at least 10bp, 11bp, 12bp, 13bp, 14bp, 15bp, 16bp, 17bp, 18bp, 19bp, 20bp, 21bp, 22bp, 23bp, 24bp, 25bp, 26bp, 27bp, 28bp, 29bp, 30bp, 31bp, 32bp, 33bp, 34bp, 35bp, 36bp, 37bp, 38bp, 39bp, 40bp, 41bp, 42bp, 43bp, 44bp, 45bp, 46bp, 47bp, 48bp, 49bp, 50bp, 51bp, 52bp, 53bp, 54bp, 55bp, 56bp, 57bp, 58bp, 59bp, 60bp, 61bp, 62bp, 63bp, 64bp, 65bp, 66bp, 67bp, 68bp, 69bp, 70bp, 71bp. 72bp, 73bp, 74bp, 75bp, 76bp, 77bp, 78bp, 79bp, 80bp, 81bp, 82bp, 83bp, 84bp, 85bp, 86bp, 87bp, 88bp, 89bp, 90bp, 91bp, 92bp, 93bp, 94bp, 95bp, 96bp, 97bp, 98bp, 99bp, and 100bp. The double-stranded cGAS-activating nucleic acid may also have a minimal length of at least 200bp, 300bp, 400bp, 500bp, 600bp, 700bp, 800bp, 900bp, 1kbp, 2kbp, 3kbp, 4kbp, 5kbp, 6kbp and the like. Corresponding lengths also apply in the event the cGAS-activating nucleic acid, e.g. DNA, is single-stranded. The double-stranded cGAS-activating nucleic acid may be DNA, for example a plasmid, such as a commonly known expression vector. The cGAS-activating nucleic acid may also be in the form of single-stranded DNA or DNA/RNA hybrids. Such DNA/RNA hybrids may also have the lengths as set out above. Any of the above recited length of base pairs may be combined with one another to form a length of base pair range. Also contemplated within such length of base pair ranges are corresponding ranges in which the lower length of base pairs is included while the upper length of base pairs is excluded, as well as ranges in which the upper length of base pairs is excluded while the upper length of base pairs is included.

In a further embodiment of the invention, the cGAS-activating nucleic acid is a dsDNA molecule in the form of

a plasmid, such as a commonly known expression vector pET, Phil 74, pGEX, pcDNA5, pFBDM, pRS, pETM.

In a preferred embodiment of the invention, the cGAS-activating nucleic acid is a dsDNA molecule having at least 12bp flanked by guanosine-rich (G-rich) ssDNA overhangs. G-rich overhangs significantly enhance cGAS binding to short DNA.

In a further preferred embodiment of the invention, the cGAS-activating nucleic acid is a dsDNA molecule having at least 30bp. Below 30bp dsDNA molecules are less efficient in activating cGAS.

Furthermore, cGAS requires divalent cations as co-factors in order to convert nucleoside triphosphates into 2'3'CDNs. Thus, in a preferred embodiment of the present invention, the one or more divalent cation is selected from the group consisting of $Mg^{2+}$, $Ca^{2+}$, $Zn^{2+}$, $Fe^{2+}$, $Cu^{2+}$, $Ni^{2+}$, and $Mn^{2+}$. In a most preferred embodiment of the present invention, the aqueous solution further comprises $Mg^{2+}$ and/or $Zn^{2+}$.

According to one embodiment, cGAS may be activated by the addition of one or more divalent cations to the aqueous solution already comprising cGAS, a cGAS-activating nucleic acid, first nucleoside triphosphate, and a second nucleoside triphosphate, wherein at least one of the first and second nucleoside triphosphate is a fluorescent nucleoside triphosphate according to the present invention. The conversion of the first and second nucleoside triphosphates into 2'3'fCDN then starts as soon as the first cGAS/cGAS-activating nucleic acid complex is formed. As soon as the first cGAS/cGAS-activating nucleic acid complex is formed, the conversion rate of the first and second nucleoside triphosphates into 2'3'fCDN continuously increases over time as more cGAS molecules become activated. When cGAS activation reaches equilibrium, the conversion rate of the first and second nucleoside triphosphate into 2'3'fCDN reaches a stationary phase. This allows fast and direct measurement of cGAS activity and the identification of a substance having the ability to modulate cGAS activity. Thus, in one embodiment of the present invention, one or more divalent cations are added to the aqueous solution comprising cGAS, cGAS-activating nucleic acid, and a first and second nucleoside triphosphate of the present invention to start the 2'3'fCDN-forming reaction.

According to another embodiment, cGAS may alternatively be activated prior to the addition of a first and a second nucleoside triphosphates of the present invention. This approach allows cGAS activation to achieve equilibrium prior to the addition of its substrates and therefore allows accurate measurement of the initial conversion rates of the nucleoside triphosphates into 2'3'fCDN. Thus, in a further preferred embodiment of the present invention, cGAS is activated prior to the addition of the first and second nucleoside triphosphates in the aqueous solution comprising cGAS, cGAS-activating nucleic acid and one or more divalent cations. After cGAS activation reaches equilibrium, the first and second nucleoside triphosphate according to the present invention are added to the aqueous solution, wherein at least one of the first and second nucleoside triphosphate is a fluorescent nucleoside triphosphate according to the present invention. The skilled person will be able to measure DNA binding equilibrium by commonly known techniques such as EMSA, ITC or fluorescence anisotropy measurements using a fluorophore attached to DNA.

In a further embodiment of the present invention, the fluorescent signal of the aqueous solution is measured at a predetermined time-point. This time-point is determined in preliminary experiments taking into account the dead time of the instrument used as the fluorescence reader, i.e. the shortest time it takes between mixing the reagents and measuring the first data point in a given instrument. It is advantageous to determine cGAS activity in a preceding experiment, e.g. by a method of measuring cGAS activity described above, to avoid choosing a time point of measuring the fluorescence signal when either the conversion of the fluorescent nucleoside triphosphate of the present invention into the 2'3'fCDN has not yet started (this would be the case for cGAS having a very low nucleotidyltransferase activity) or all of the free fluorescent nucleoside triphosphate in the aqueous solution has already been converted into 2'3'fCDN due to very high nucleotidyltransferase activity of cGAS molecules. Once the correct time point is determined for each experimental set up, measuring of the fluorescent signal at a predetermined time point allows fast and reliable measurement of the fluorescent signal in aqueous solution. According to one embodiment of the invention, this allows fast and reliable identification of substances having an agonistic or antagonistic effect on cGAS activity.

In another embodiment of the present invention, the fluorescence signal of the aqueous solution is measured continuously over a predetermined time interval. Measuring over a time interval, the slope can be determined more precisely than using a single time point and the linear interval of the reaction can be determined more accurately. In some embodiments of the present invention, the fluorescent signal is measured starting from about 10 seconds from mixing all compounds together up to about 1 hour. The time interval of the measurement depends strongly on the experimental setup, such as buffer condition, temperature and substance concentration. In general, for the low cGAS activity higher protein and/or activating DNA and/or substance concentrations may be used, compared to the case of high cGAS activity to perform the measurement within 5 min - 1 h time interval.

In a further embodiment of the present invention, the measured fluorescence signal of the aqueous solution is measured at different wavelengths or ranges of wavelengths depending on the fluorescent nucleoside triphosphate or triphosphates used in the methods according to the invention. Specifically, each fluorescent nucleoside triphosphate may have different excitation and emission maxima which influence the wavelengths at which the fluorescence signal is measured. Thus, depending on the fluorescent nucleoside triphosphate or triphosphates used in the methods according to the invention the wavelengths or range of wavelengths at which the fluorescent nucleoside triphosphate is excited

(i.e. the excitation maxima) and the wavelengths or range of wavelengths at which the fluorescence signal is measured (i.e. the emission maxima) need to be adapted for each fluorescent nucleoside triphosphate or triphosphates individually in order to avoid false positives by measuring a fluorescence signal outside the optimal wavelength or range of wavelengths for a given fluorescent nucleoside triphosphate or triphosphates.

In some embodiments of the present invention, the excitation wavelength is in the range of about 295nm to about 365nm, or between about 295nm and 365nm. In some embodiments of the invention, the excitation wavelength is about 295nm, about 296nm, about 297nm, about 298nm, about 299nm, about 300nm, about 301nm, about 302nm, about 303nm, about 304nm, about 305nm, about 306nm, about 307 nm, about 308nm, about 309nm, about 310nm, about 311nm, about 312nm, about 312nm, about 313nm, about 314nm, about 315nm, about 316nm, about 317nm, about 318nm, about 319nm, about 320nm, about 321nm, about 322nm, about 323nm, is about 324nm, about 325nm, about 326nm, about 327nm, about 329nm, about 330nm, about 331nm, about 332nm, about 333nm, about 334nm, about 335nm, about 336nm, about 337nm, about 338nm, about 339nm, about 340nm, about 341nm, about 342nm, about 343nm, about 344nm, about 345nm, about 346nm, about 347nm, about 348nm, about 349nm, about 350nm, about 351nm, about 352nm, about 353nm, about 354nm, about 355nm, about 356nm, about 357nm, about 358nm, about 359nm, about 360nm, about 361nm, about 362nm, about 363nm, about 364nm, or about 365nm. Any of the above recited excitation wavelength may be combined with one another to form an excitation wavelength range. Also contemplated within such excitation wavelength ranges are corresponding ranges in which the lower excitation wavelength is included while the upper excitation wavelength is excluded, as well as ranges in which the lower excitation wavelength is excluded while the upper excitation wavelength is included. In a particularly preferred embodiment of the invention, the excitation wavelength ranges from about 295nm to about 315nm or between about 295nm and 315nm. In a most preferred embodiment the excitation wavelength is 305nm.

In some embodiments of the present invention, the emission wavelength ranges from about 350nm to about 500nm or between about 350nm and 500nm. In some embodiments the excitation wavelength is about 350nm, about 351nm, about 352nm, about 353nm, about 354nm, about 355nm, about 356nm, about 357nm, about 358nm, about 359nm, about 360nm, about 361nm, about 362nm, about 363nm, about 364nm, about 365nm, about 366nm, about 367nm, about 368nm, about 369nm, about 370nm, about 371nm, about 372nm, about 373nm, about 374nm, about 375nm, about 376nm, about 377nm, about 378nm, about 379nm, about 380nm, about 381nm, about 382nm, about 383nm, about 384nm, about 385nm, about 386nm, about 387nm, about 388nm, about 389nm, about 390nm, about 391nm, about 392nm, about 393nm, about 394nm, about 395nm, about 396nm, about 397nm, about 398nm, about 399nm, about 400nm, about 401nm, about 402nm, about 403nm, about 404nm, about 405nm, about 406nm, about 407nm, about 408nm, about 409nm, about 410nm, about 411nm, about 412nm, about 413nm, about 414nm, about 415nm, about 416nm, about 417nm, about 418nm, about 419nm, about 420nm, about 421nm, about 422nm, about 423nm, about 424nm, about 425nm, about 426nm, about 427nm, about 428nm, about 429nm, about 430nm, about 431nm, about 432nm, about 433nm, about 434nm, about 435nm, about 436nm, about 437nm, about 438nm, about 439nm, about 440nm, about 441nm, about 442nm, about 443nm, about 444nm, about 445nm, about 446nm, about 447nm, about 448nm, about 449nm, about 450nm, about 451nm, about 452nm, about 453nm, about 454nm, about 455nm, about 456nm, about 457nm, about 458nm, about 459nm, about 460nm, about 461nm, about 462nm, about 463nm, about 464nm, about 465nm, about 466nm, about 467nm, about 468nm, about 469nm, about 470nm, about 471nm, about 472nm, about 473nm, about 474nm, about 475nm, about 476nm, about 477nm, about 478nm, about 479nm, about 480nm, about 481nm, about 482nm, about 483nm, about 484nm, about 485nm, about 486nm, about 487nm, about 488nm, about 489nm, about 490nm, about 491nm, about 492nm, about 493nm, about 494nm, about 495nm, about 496nm, about 497nm, about 498nm, about 499nm or about 500nm. Any of the above recited emission wavelengths may be combined with one another to form an emission wavelength range. Also contemplated within such emission wavelength ranges are corresponding ranges in which the lower emission wavelength is included while the upper emission wavelength is excluded, as well as ranges in which the lower emission wavelength is excluded while the upper emission wavelength is included. In a particularly preferred embodiment of the invention, the emission wavelength ranges from about 350nm to about 380nm, or between about 350nm and 380nm. In a most preferred embodiment of the invention the emission wavelength is 363nm.

Furthermore, the quantum yield of a fluorescent nucleoside triphosphate may also differ depending on the experimental conditions. Thus, for each fluorescent nucleoside triphosphate the experimental conditions need to be modified in order to optimize the quantum yield of the fluorescent molecule. Experimental conditions that might influence the quantum yield of a given fluorescent nucleoside triphosphate of include but are not limited to pH, temperature, ionic strength, buffer composition

The change of the fluorescence signal can be measured by any known fluorescence read out technique using a conventional plate reader or a single-cuvette photometer. In a preferred embodiment of the present invention, the fluorescence signal of the aqueous solution may be measured by a Tecan infinite M1000 using 96-well black non-binding PS plates (Greiner Bio-One), however a FluoroMax-P spectrofluorometer (Horiba Scientific) or similar instrument can be used.

According to the invention, the fluorescent nucleoside triphosphate is a fluorescent and purine nucleoside triphosphate. In one embodiment of the present invention, the fluorescent nucleoside triphosphate can be selected from the group consisting of a 2-aminopurine nucleoside triphosphate (2-APTP), a 3-methyl-isoxanthopterin nucleoside triphosphate (3-MITP), a 6-methyl isoxanthopterin nucleoside triphosphate (6-MITP), 4-amino-6-methyl-8-(2-deoxy-beta-d-ribofuranosyl)-7(8H)-pteridone nucleoside triphosphate (6-MAPTP), a 4-amino-2,6-dimethyl-8-(2'-deoxy-beta-d-ribofuranosyl)-7(8H)-pteridone nucleoside triphosphate (DMAPTP), a pyrrolocytosine nuceloside triphosphate (pyrrolo-CTP), a 6-phenylpyrrolocytosine nucleoside triphosphate (PhpCTP), a (aminoethoxy)phenylpyrrolocytosine nucleoside triphosphate (moPhpCTP), a [bis-o-(aminoethoxy)phenyl]pyrrolocytosine nucleoside triphosphate (boPhpCTP), a hydropyrimidopyrimidine nucleoside triphosphate ($C^{hpp}TP$), a pyrrolopyrimidopyrimidine nucleoside triphosphate ($C^{ppp}TP$), a pyrimidopyrimidoindole nucleoside triphosphate ($C^{ppi}TP$), a benzopyridopyrimidine nucleoside triphosphate (BPPTP), a naphtopyridopyrimidine nucleoside triphosphate (NPPTP), a methoxybenzodeazaadenine nucleoside triphosphate ($^{MD}ATP$), a methoxybenzodeazainosine nucleoside triphosphate ($^{MD}ITP$), a naphtodeazaadenine nucleoside triphosphate ($^{ND}ATP$), a furan-modified pyrimidine nucleoside states, a thieno[3,2]pyrimidine nucleoside triphosphate, a thieno[3,4]pyrimidine triphosphate, a 5-methoxy-quinazoline-2,4-(1H,3H) dione nucleoside triphosphate, a 5-methylpyrimidine-2-one nucleoside triphosphate, a 7-deazapurine nucleoside triphosphate, a 5-alkyluridine nucleoside triphosphate, a benzoquinalozines nucleoside triphosphate, a triazoleadenosine nucleoside triphosphate, and a 1,$N^6$-ethenoadenosine nucleoside triphosphate, wherein the nucleoside triphosphate is a deoxyribonucleoside triphosphate or a ribonucleoside triphosphate. In a most preferred embodiment, the fluorescent nucleoside triphosphate is 2-amino purine ribonucleoside triphosphate (2-APTP) comprising the fluorescent nucleobase analogue 2-aminopurine (2-AP). The inventors surprisingly found out that the high fluorescence quantum yield of 2-APTP in aqueous solution (about 0.68) is considerably reduced when incorporated into 2'3'fCDN. The high sensitivity of 2-AP to the microenvironment can thus be exploited to measure cGAS activity in the presence or absence of a substance. In particular, the inventors found out that cGAs can utilize 2-APTP instead of adenine nucleoside triphosphate (ATP) to efficiently generate 2'3'fCDN in the presence of a second nucleoside triphosphate. The quenching of the fluorescence signal upon incorporation of 2-APTP into 2'3'fCDN allows the measurement of cGAS activity in a continuous assay using a conventional fluorescence plate reader or single cuvette photometer. The finding that 2-APTP is utilized by cGAS for incorporation into 2'3'fCDN in conjunction with a significant quenching of the fluorescence signal allows the determination of quantitative steady state activity rates of cGAS in an easily scalable and high-throughput manner. Based on the unexpected properties of 2-APTP in the methods of the present invention, the inventors were thus able to develop a fast and reliable method of measuring cGAS activity and a method to screen a large range of substances for their ability to modulate cGAS activity in a fast and reliable manner.

In a preferred embodiment of the present invention, the first nucleoside triphosphate is 2-APTP.

As evident from all of the above, the fluorescent nucleoside triphosphate can be used for measuring cGAS activity or for identifying a substance having an ability to modulate cGAS activity. The fluorescent nucleoside triphosphate therefore represents a powerful tool to measure cGAS activity and to identify substances having an ability to modulate cGAS activity in a fast and reliable method according to the invention.

In a further embodiment of the present invention, the second nucleoside triphosphate is a purine ribonucleoside triphosphate. The ribofuranosyl backbone of the second nucleoside triphosphate contains a hydroxyl group at position 2 of the ribose ring, thereby allowing the formation of the 2'5'-phosphodiester linkage between the first and the second nucleoside triphosphate. It is essential that one of the first and second nucleoside triphosphates has a free 2' hydroxyl group and the other one has a free 3' hydroxyl group in order to enable the formation of the 2'-5' and 3'-5' phosphodiester linkages in the 2'3'CDN.

In a most preferred embodiment of the present invention, the second nucleoside triphosphate is guanosine triphosphate (GTP) having a free 2' hydroxyl group, whereas the first nucleoside triphosphate is 2-APTP having a free 3' hydroxyl group.

According to the methods of the present invention, the skilled person will realize that the fluorescent nucleoside triphosphates set forth herein can be used for measuring cGAS activity and for identifying a substance having an ability to modulate cGAS activity.

## Method of preparing a fluorescent cyclic dinucleotide

In a further aspect, the present invention relates to a method of preparing a fluorescent cyclic dinucleotide (2'3'fCDN) of the present invention. The method comprises the steps of (i) providing an aqueous solution comprising cGAS, a cGAS-activating nucleic acid, a first nucleoside triphosphate, a second nucleoside triphosphate, and one or more divalent cation, wherein at least one of the first and second nucleoside triphosphate is a fluorescent nucleoside triphosphate of the present invention, and wherein one of the first and second nucleoside triphosphate has a free 2' hydroxyl group and the other one has a free 3' hydroxyl group, thereby preparing the cyclic dinucleotide of the present invention. The method is based on the finding that activated cGAS generates cyclic dinucleotides by utilizing fluorescent

nucleoside triphosphates of the present invention instead of natural occurring nucleoside triphosphates with only slightly reduced efficiency.

In a preferred embodiment of the present invention, the fluorescent cyclic dinucleotide of the present invention is further purified. For example, the reaction mixture of the method of preparation can be subjected to commonly known purification techniques such as reversed-phase HPLC, ion exchange chromatography, solid phase extraction, and the like.

In a further embodiment of the invention, the method of preparing a 2'3'fCDN further comprises additional steps of incubating the aqueous solution for a specified time period and under predetermined conditions sufficient to allow cGAS to convert the first and the second nucleoside triphosphate into a CDN of the invention. In particular, about 50 nM to about 50 $\mu$M, most preferably about 10 $\mu$M, cGAS is incubated with about 2.6 to 650 ng/$\mu$l, most preferably about 195 ng/$\mu$l, DNA in the aqueous solution comprising about 40 mM Tris, about 100 mM NaCl, about pH 7.5, about 5 mM $MgCl_2$, about 50 $\mu$M to about 5 mM of a first nucleoside triphosphate (i.e. a fluorescent nucleoside triphosphate), and about 50 $\mu$M to about 5 mM of a second nucleoside triphosphate. The reaction is performed at about 32°C to about 37°C for about 30 minutes to about 3 hours. Most preferably, the reaction mixture is incubated at about 37°C for about 60 minutes.

As evident from the overall teaching of the present invention, the 2'3'fCDN of the present invention can advantageously be used to label cGAS. Upon complex formation between cGAS and the 2'3'fCDN of the present invention, the cyclic dinucleotides can further be used to label cGAS due to its (at least one) fluorescent nucleotide monophosphate.

The following examples, including the experiments conducted and the results achieved, are provided for illustrative purposes only and are not construed as limiting the present invention.

**Example 1: Cell line and reagents**

The following cell lines and reagents were used in the herein described experiments. All DNA oligonucleotides were purchased from Metabion.

The following cell lines were used: HEK293T STING KI (Ablasser, Schmid-Burgk et al. "Cell intrinsic immunity spreads to bystander cells via the intercellular transfer of cGAMP". Nature 503, 530-534, 2013), BLaER1(Rapino, Robles et al. "C/EBP$\alpha$ Induces Highly Efficient Macrophage Transdifferentiation of B Lymphoma and Leukemia Cell Lines and Impairs Their Tumorigenicity". Cell Reports 3, 1153-1163, 2013) and BLaER1 cGAS-KO (cGAS deficient cell line, a general gift from M. Gaidt, group of V. Hornung, Gene Center, Ludwig-Maximilians-Universitat München, Center for Integrated Protein Science Munich, Germany).

**Example 2: Constructs and cloning**

The following constructs were used in the below described experiments:

The plasmid encoding full-length (aa 1-522) and truncated (catalytic domain - $^{cd}$) *Homo sapiens* (h) (aa 155-522), *Mus musculus* (m) (aa 141-507) and *Sus scrofa* (s) (aa 135-495) cGAS for N-terminal $His_6$-MBP fusion protein expression were obtained from Dr. F. Civril (Civril, Deimling et al. "Structural mechanism of cytosolic DNA sensing by cGAS". Nature 498, 332-337, 2013). The plasmid encoding hSTING cytosolic domain ($^{cd}$, aa 139-379, R220H+H232R variant) for N-terminal $His_6$-SUMO1 fusion protein expression was obtained from Dr. T. Deimling (Cavlar, Deimling et al. "Species-specific detection of the antiviral small-molecule compound CMA by STING". The EMBO Jourhal 32, 1440-1450, 2013). These plasmids were used to transform *E.coli* Rosetta (DE3) protein expression strain cells (Novagen).

**Example 3: Cell lines and cell culture**

HEK293T STING KI were cultured in DMEM (Thermo Fisher Scientific or Sigma-Aldrich, respectively) supplemented with 10% heat inactivated FBS (Thermo Fisher Scientific or Biochrom, respectively) and incubated at 37°C with 5% $CO_2$.

BLaER1 and BLaER1 cGAS-KO cells were cultivated in RPMI medium containing heat-inactivated 10% FCS, Penicillin (100 U/ml), Streptomycin (100 $\mu$g/ml) (Gibco, Life Technologies) and 1 mM sodium pyruvate (Thermo Fisher Scientific). For trans-differentiation, $5 \times 10^4$ cells were seeded per well of a flat bottom 96 well plate and cultivated in the presence of ß-estradiol (100 nM, Sigma-Aldrich), hr-IL-3 (10 ng/ml) and M-CSF (10 ng/ml) (both PeproTech) for five days prior to the experiment (Gaidt, Ebert et al. "Human Monocytes Engage an Alternative Inflammasome Pathway". Immunity 44, 833-846, 2016).

**Example 4: Protein expression and purification**

All cGAS protein constructs (e.g. hcGAS$^{cd}$, mcGAS$^{cd}$, scGAS$^{cd}$) were overexpressed in *E. coli* Rosetta (DE3) for 16-18h at 18°C after induction with 0.2 mM IPTG. Cells were lysed by sonication in 50 mM Tris, 500 mM NaCl, 5 mM $MgCl_2$, 10 mM imidazole, 10% Glycerol, pH 7.5, supplemented with 2 mM β-mercaptoethanol and protease inhibitor cocktail (Sigma-Aldrich) and purified with Ni-NTA agarose resin (Qiagen). For truncated cGAS proteins $His_6$-MBP tag was removed with TEV protease (1:50 mass ratio) during 16 h dialysis against 30 mM Tris, 100 mM NaCl, 2 mM DTT, pH 7.0. cGAS proteins were further purified by cation-exchange chromatography (30 mM Tris, 100 mM/l M NaCl, 2 mM DTT, pH 7.0) on HiTrap SP HP columns (GE Healthcare) followed by size-exclusion chromatography on HiLoad S200 16/60 column (GE Healthcare) equilibrated with 20 mM Tris, 100 mM NaCl, pH 7.5. Full-length hcGAS with N-terminal $His_6$-MBP tag (hcGAS) and mcGAS proteins were concentrated to 8-12 mg $ml^{-1}$. Truncated hcGAS was concentrated to 4 mg $ml^{-1}$. All proteins were flash-frozen in liquid nitrogen and stored at -80°C.

mTfam and mHMGB1 (both DNA bending proteins) were purified as described for full-length hcGAS, except the cation-exchange chromatography step was skipped and after dialysis against 20 mM Tris, 300mM NaCl, pH 7.5 size exclusion chromatography on HiLoad S75 16/60 (GE Healthcare) column was performed. Proteins were concentrated to 10-13 mg $ml^{-1}$.

1HU (another DNA bending protein) was purified as described for full-length hcGAS, except instead of cation-exchange chromatography affinity chromatography on HiTrap Heparin HP (GE Healthcare) column was performed (20 mM Tris, 100 mM/l M NaCl, 2 mM DTT, pH 7.5). HiLoad S75 16/60 (GE Healthcare) equilibrated with 20 mM Tris, 100 mM NaCl, pH 7.5 was used for size exclusion chromatography. Protein was concentrated to 7 mg $ml^{-1}$.

hSTING$^{cd}$ construct was expressed and purified as follows. *E. coli* Rosetta (DE3) cells were grown in 31 of LB media supplemented with Kanamycin (50mg/l) and Chloramphenicol (34mg/l) at 37°C for 3 hours, to $OD_{600}$=0.8. Expression of N-terminal $His_6$-SUMO1-tagged STING was induced by adding IPTG (Roth) to a final concentration of 0.2mM. Expression was done overnight at 18°C. Cells were collected by centrifugation and were resuspended in lysis buffer (50mM Tris, 500mM NaCl, 10mM imidazole, 5% glycerol, 2mM β-mercaptoethanol, pH7.5) and lyzed by sonification. The soluble $His_6$-SUMO1-STING was purified by Ni-affinity chromatography. The $His_6$-SUMO1-tag was removed by proteolytic cleavage with SenP2 protease during dialysis over night (20mM Tris, 150mM NaCl, 3% glycerol, 2mM β-mercaptoethanol, pH7.5) and a second Ni-affinity chromatography step. To remove additional contaminants, a HiTrap Q FF (GE Healthcare) purification was applied. Finally, the STING containing flow-through was used in a HiLoad 16/60 Superdex 75 prep grade (GE Healthcare) size-exclusion chromatography step (20 mM Tris, 100 mM NaCl, pH 7.5). Purified STING was concentrated (9-15mg/ml) with a 10kDA cut-off centrifugal concentrator device (Millipore) and was flash frozen in liquid nitrogen for storage (-80°C).

**Example 5: Characterization of cGAS activating nucleic acids**

cGAS is activated by cytosolic DNA. In order to study the effect of different DNA constructs on cGAS activation, DNA of different length was tested in an enzyme-linked immunoabsorbent assay as follows (ELISA). This assay provided information about efficient cGAS-activating nucleic acids that could be used to induce robust cGAS activity in the herein described methods of the invention.

$5x10^4$ trans-differentiated BLaER1 cells expressing cGAS were transfected with 20, 40 or 60 ng DNA of different length (20-100 bp in 5 bp intervals) and herring testis (HT) DNA using 0.5 μl Lipofectamine 2000 Transfection Reagent (Thermo Fischer Scientific) in 50 μl Opti-MEM Reduced Serum medium (Thermo Fischer Scientific). CXCL 10 expression in the supernatants was quantified 8 h after transfection using ELISA (BD OptEIA™, human IP-10 ELISA Set). Cells stimulated with transfection reagent only and unstimulated cells served as control.

As can be seen from Figure 5a, the data indicate a concentration and DNA length-dependent activation of cGAS measuring CXCL10 production as a surrogate parameter of cGAS activity. Long herring testes (HT) DNA robustly activated cGAS at all DNA concentrations, while shorter DNA molecules required increasing concentrations of DNA. The data show a clear preference for long DNA at low DNA concentrations, while short DNA is more effective at higher DNA amounts.

The same effect was also observed in fluorescence-based cGAS activity assays described in Example 9. The data clearly indicate that at a constant concentration of both human full-length cGAS and human truncated cGAS (hcGAS$^{cd}$) a DNA length-dependent activation was observed which was independent of the presence or absence of cGAS N-terminal domain (Figure 5b). The data further indicate that robust activation in vitro proceeds most readily when DNA > 40bp (Figure 5b). The length-dependence was furthermore not species-specific, since truncated mouse cGAS (mcGAS$^{cd}$) showed a comparable length dependent activation, with a gradual increase in activity until about 75bp (Figure 5c). Plasmid DNA was an even more potent activator (Figure 5c).

Overall, the data indicate that cGAS is activated in a manner dependent on the length of the nucleic acid used, wherein cGAS activity directly correlates with the length of the tested DNA. Furthermore, the data indicate that the

nucleotide sequence does not influence cGAS activation and that the observed length-dependence was not species-specific. In summary, an increase in DNA length directly correlates with an increase in cGAS activity.

**Example 6: Radiolabeled cGAS activity assays**

The above results were confirmed by radiolabeled cGAS assays. In this assay 13 ng $\mu l^{-1}$ DNA was mixed with 2 $\mu$M truncated mouse cGAS ($mcGAS^{cd}$) and reaction was started by adding 50 $\mu$M ATP, 500 $\mu$M GTP, 5 mM $MgCl_2$ in 40 mM Tris pH 7.5 and 100 mM NaCl containing 1:800 [$\alpha^{32}$P]ATP (3000 Ci mmol-1, Hartman Analytic). Samples were incubated at 35°C and the reaction was stopped by plotting on PEI-Cellulose F plates (Merck) and analyzed by thin layer chromatography (TLC) with 1M $(NH_4)_2SO_4$/1.5M $KH_2PO_4$ as running buffer. The radiolabeled products were visualized with Typhoon FLA 9000 phosphor imaging system.

The results clearly confirmed the length-dependent cGAS activation already described in Example 5 above (see Figure 5d).

For testing of 2-APTP incorporation into cGAS enzymatic activity product 1 $\mu$M $mcGAS^{cd}$ was mixed with 13 ng $\mu l^{-1}$ ($\approx$1 $\mu$M binding sites) 55 bp, 500 $\mu$M 2-APTP/ATP, 500 $\mu$M GTP, 5 mM $MgCl_2$ and 1:160 [$\alpha^{32}$P]ATP or [$\alpha^{32}$P]GTP (3000 Ci mmol-1, Hartman Analytic) in the same condition.

The data demonstrate that cGAS is capable of incorporating 2-APTP together with GTP into 2'3'fGAMP (see Figure 6a).

**Example 7: Analysis of chemical properties of 2'3'fGAMP**

To analyze the physical and chemical properties of 2'3'fGAMP, the fluorescent cyclic dinucleotide was analyzed by anion-exchange chromatography. 10 $\mu$M truncated mouse cGAS ($mcGAS^{cd}$), truncated porcine cGAS ($scGAS^{cd}$) or truncated human cGAS ($hcGAS^{cd}$) were incubated with 195 ng $\mu l^{-1}$ ($\approx$15 $\mu$M binding sites) plasmid DNA at 35°C for 2 h in buffer containing 40 mM Tris pH 7.5, 100 mM NaCl, 10 mM $MgCl_2$, 2 mM 2-APTP and 2 mM GTP. Reaction mixtures were centrifuged for 10 min, 16100 rcf, and the supernatant was separated from cGAS by ultrafiltration (30 kDa, Amicon). Resulting flow through was diluted in 50 mM Tris pH 9.0 and loaded on Mono Q 5/50 GL (GE Healthcare) for anion exchange chromatography (50 mM Tris, 0 M/1 M NaCl, pH 9.0). The fractions containing cGAS product fGAMP were collected, lyophilized and stored at -20°C. Control runs with 2-APTP, GTP, 2'3'- and 3'3'cGAMP were made analogously to validate the resulting peaks of cGAS reaction products.

As can be seen from the chromatograms in Figures 6b and 6c, 2'3'fGAMP generated by truncated mouse ($mcGAS^{cd}$) or human cGAS ($hcGAS^{cd}$) was detected in the same elution volume as natural 2'3'cGAMP at approximately 10 mL, indicating that the fluorescent 2'3'fGAMP has similar chemical and physical properties as the natural 2'3'cGAMP.

**Example 8: Analysis of enzymatically synthesized fGAMP by mass spectrometry.**

To further determine the exact chemical composition of fGAMP produced by cGAS from 2-APTP and GTP, the resulting fGAMP was analyzed with mass spectrometry as follows. 10 $\mu$M truncated mouse cGAS ($mcGAS^{cd}$) was incubated with 195 ng $\mu l^{-1}$ ($\approx$15 $\mu$M binding sites) plasmid DNA at 35°C for 2 h in buffer containing 40 mM Tris pH 7.5, 100 mM NaCl, 10 mM $MgCl_2$, 2 mM 2-APTP and 2 mM GTP. Reaction mixtures were centrifuged for 10 min, 16100 rcf, and the supernatant was separated from cGAS by ultrafiltration (30 kDa, Amicon). Resulting flow through was diluted in 50 mM Tris pH 9.0 and loaded on Mono Q 5/50 GL (GE Healthcare) for anion exchange chromatography (50 mM Tris, 0 M/l M NaCl, pH 9.0). The fractions containing cGAS product fGAMP were collected, lyophilized and stored at -20°C.

For further purification reverse phase high-performance liquid chromatography (HPLC) was used with solvent A (triethylammonium acetate:acetonitrile:$H_2O$ [0.1 M:3%:97%], w:v:v) and solvent B (methanol:acetonitrile:$H_2O$ [45%:45%:10%], v:v:v). Lyophilized fGAMP was diluted in solvent A and subjected to a C18 column. Two step linear gradient 0%-10% B (2 CV), 10%-50% B (2 CV) was used as described in (Gao, Ascano et al. "Cyclic [G(2',5')pA(3',5')p] Is the Metazoan Second Messenger Produced by DNA-Activated Cyclic GMP-AMP Synthase". Cell 153,1094-1107,2013).

The sample was lyophilized and diluted in 100 $\mu$L $H_2O$ for ESI LC/MS analysis.

Flow injection analysis with 10 $\mu$L fGAMP sample was performed with a Surveyor MS pump with a flow rate of 100 $\mu$L/min 20-80% ($H_2O$:acetonitrile) using an injection filter. ESI-LC/MS analysis was performed using a Finnigan LTQ FT Ultra Fourier Transform Ion Cyclotron Resonance mass spectrometer (Thermo Fisher Scientific) with an IonMax source with ESI head. The resolution was set to 100.000 at m/z 400 and the spectra were acquired in a mass range from m/z 150 to 1,000. The ion transfer capillary temperature was set to 250 °C, the spray voltage to 4 kV, liquid nitrogen Sheathgasflow to 20 and the Sweepgasflow to 5 units. The acquired spectrum of enzymatically synthesized fGAMP shows ions corresponding to fGAMP-H (m/z 673.09) and fGAMP-2H+$Na^+$ (m/z 695.07) (Figure 6d). In tandem mass spectrometry spectrum (Figure 6e) derived from a parent fGAMP ion (m/z 673.09) ion products corresponding to depurination of the dinucleotide were observed. Depurination of guanine resulted in product with m/z 522.19 and depurination of 2-AP - in product with m/z 538.15. The ratio of these peaks (more depurinated guanines than 2-AP) is characteristic for the linkage between 2'-OH of GTP and 5'-phosphate of 2-APTP within the fGAMP molecule corresponding to 2'3'fGAMP as cGAS product (Ablasser, Goldeck et al. "cGAS produces a 2[prime]-5[prime]-linked cyclic dinucleotide second messenger that activates STING". Nature 498, 380-384, 2013).

**Example 9: Fluorescence-based cGAS activity assay**

To precisely measure initial rates of cGAS activity in high-throughput, the inventors developed a fluorescence-based cGAS activity assay based on the incorporation of the fluorescent nucleoside analog 2-aminopurine nucleoside triphosphate (2-APTP) along with GTP into cyclic dinucleotide denoted 2'3'fGAMP (Figure 7a).In this assay 2-APTP and 6.5 ng $\mu l^{-1}$ DNA of different lengths (20 -100 bp in 5 bp intervals, and pET28M-SUMO1-GFP vector (EMBL) (6.2 kbp, plasmid)) that corresponds to roughly 0.5 $\mu$M 20 bp (approximate length of cGAS binding site) were premixed with 0.5 $\mu$M cGAS in 40 mM Tris pH 7.5 and 100 mM NaCl. Alternatively, 2.6 ng $\mu l^{-1}$ DNA ($\approx$0.2 $\mu$M binding sites) and 1$\mu$M cGAS were used. The reaction was started by adding 5 mM $MgCl_2$ together with 500 $\mu$M GTP and 50 $\mu$M 2-APTP and performed at 32°C. Fluorescence decrease was measured in 96-well black non-binding PS plates (Greiner Bio-One) on Tecan infinite M1000 ($\lambda_{ex}$=305 nm, $\lambda_{em}$=363 nm, gain 100, settle time 10ms, kinetic interval 2 min).

Additionally, 2-APTP fluorescence alone was studied in different conditions, relevant for the assay, as follows. 2.5, 10, 50 or 100 $\mu$M 2-APTP was mixed in ultrapure water, 100mM NaCl + 5mM $MgCl_2$, 40mM Tris pH 7.5, 500 $\mu$M GTP, 40mM Tris pH 7.5 + 100mM NaCl + 5mM $MgCl_2$ or 40mM Tris pH 7.5 + 100mM NaCl + 5mM $MgCl_2$ and fluorescence was measured as described above. The fluorescence intensity of 2-APTP was not influenced by the used components in the aqueous solution and did not differ from that of fluorescence intensity in ultrapure water (Figure 7b).

The data clearly indicate that the fluorescence signal of free 2-APTP is quenched upon the incorporation of 2-APTP into 2'3'fGAMP during the reaction which allows the measurement of the reaction rate by measuring the change in fluorescence intensity (Figure 7a,c). Initial fluorescence read-out curves (Figure 7c) represent the changes in fluorescence during the reaction. For each curve the background fluorescence values were subtracted and inverted to give the positive value ($\Delta$F). The resulting curves were scaled, such that $\Delta$F at time point 0 min equals 0 ($\Delta F - \Delta F_{t=0}$) (Figure 7d). The initial cGAS reaction rates were calculated as a slope of the linear intervals (dashed lines) on the resulting curves and are defined as $\Delta F/\Delta t$ [RFU $min^{-1}$] (Figure 7e).

The data indicate that the new assay allows measurement of cGAS activity in a simple-to-implement high-throughput assay. Furthermore, the data suggest that the assay can be used to identify substances having an ability to modulate cGAS activity as described above.

**Example 10: cGAS incorporates 2-APTP into 2'3'fGAMP in a species-independent manner**

In order to investigate whether 2-APTP is incorporated into 2'3'fGAMP by cGAS in a species-dependent manner, three truncated constructs comprising Mab21 from human ($hcGAS^{cd}$), mouse ($mcGAS^{cd}$) and *Sus scrofa* ($scGAS^{cd}$) and human full length cGAS (hcGAS) were generated as described above.

These constructs were then analyzed using anion exchange chromatography of cGAS products on MonoQ 5/50 GL column (GE Healthcare), as described in Example 7. The data show that the incorporation of 2-APTP into 2'3'fGAMP does not depend on the origin of cGAS as indicated by the calculated cGAS activity rates for the different species (see Figure 8a).

The constructs were further tested in fluorescence-based cGAS activity assays. 0.5 $\mu$M $mcGAS^{cd}$ (Figure 8b), $hcGAS^{cd}$ (Figure 8c) or hcGAS (Figure 8d) were premixed with 6.5 ng $\mu l^{-1}$ DNA of different lengths (20 -100 bp), and pET28M-SUMO1-GFP vector (EMBL) (6.2 kbp, plasmid)) that corresponds to roughly 0.5 $\mu$M 20 bp in 40 mM Tris pH 7.5 and 100 mM NaCl. The reaction was started by adding 5 mM $MgCl_2$ together with 500 $\mu$M GTP and 50 $\mu$M 2-APTP and performed at 32°C. Fluorescence decrease (quenching) was measured in 96-well black non-binding PS plates (Greiner Bio-One) on Tecan infinite M1000 ($\lambda_{ex}$=305 nm, $\lambda_{em}$=363 nm, gain 100, settle time 10ms, kinetic interval 2 min). cGAS activity was calculated as described in Example 9 above. All tested constructs showed the same increase of cGAS activity with DNA length.

The results further indicate that the described assay is suitable for evaluating the activity of both truncated and full length cGAS constructs, as well as cGAS constructs originating from different species.

**Example 11: DNA-bending proteins enhance cGAS activity**

In order to investigate the effect of DNA-bending proteins on cGAS activity, a fluorescence-based cGAS activity assay as described above was performed in the presence several DNA stress associated factors. The experiment was performed as follows:

13 ng $\mu l^{-1}$ DNA was premixed with DNA-bending proteins mTFAM, mHMGB1 or 1HU in concentrations 0-5 $\mu$M, after which 100 nM cGAS in 40 mM Tris pH 7.5 and 100 mM NaCl was added. The reaction was started by adding 5 mM $MgCl_2$ together with 500 $\mu$M GTP and 50 $\mu$M 2-APTP and carried out at 32°C for $mcGAS^{cd}$ or 37°C for hcGAS. Fluorescence was measured in 96-well black non-binding PS plates (Greiner Bio-One) on Tecan infinite M1000 ($\lambda_{ex}$=305 nm, $\lambda_{em}$=363 nm, gain 100, settle time 10ms, kinetic interval 2 min). Based on the measured fluorescence cGAS activity was calculated as described in Example 9 above.

The data indicate that with low concentrations of cGAS only background activity is observed in the absence of DNA-bending proteins, whereas adding increasing amounts of mTFAM, mHMGB1 and 1Hu robustly activated truncated mouse cGAS ($mcGAS^{cd}$) in vitro up to ~25 fold (Figure 9a-c) A similar activation was also seen for hTFAM and full-length human cGAS (hcGAS) (Figure 9d). Each DNA-bending protein tested had an optimal concentration at which maximal stimulation of cGAS activity was observed. Above such concentration cGAS activity was sharply abolished.

The data indicate that the above described assay is suitable for identifying a substance, illustratively represented by one or more DNA bending proteins, which has an ability to modulate, e.g. enhance, cGAS activity.

**Example 12: STING binding assays**

In order to investigate whether 2'3'fCDN is able to bind to STING, luciferase activity assays were performed in which HEK293T cells stably expressing STING (HEK293T STING KI) (Ablasser, Schmid-Burgk et al. "Cell intrinsic immunity spreads to bystander cells via the intercellular transfer of cGAMP". Nature 503, 530-534, 2013) were induced with 2'3'fGAMP.

Briefly, $1x10^5$ HEK293T STING KI cells were seeded on 96-well plates and transfected with 100 ng p-125Luc, 10 ng pGL4.74 (Promega) plasmids and fGAMP diluted in DPBS (Gibco, Thermo Scientific) per well using Lipofectamine 2000 (Invitrogen, Thermo Fisher Scientific) as transfection reagent according to the vendor's protocol. After 14h cells were lysed in 50 $\mu$L passive lysis buffer (Promega, Madison, WI, USA). Immunoactivity experiments using the Dual-Glo luciferase assay system (Promega, Madison, WI, USA) were performed according to manufacturer's instructions. The luciferase activity was determined in a 96-well plate reader.

Interferon-$\beta$ response was measured as a proportion of firefly (FF) luciferase activity to Renilla (Ren) lucifearse activity. All ratios were normalized to 2'3'fGAMP buffer control. The data clearly show that IFN-$\beta$ response increases with 2'3'fGAMP concentration (see Figure 10a) indicating that is capable to bind and activate STING in a cellular system.

**Example 13: STING-dependent 2'3'fCDN quenching assay**

To investigate the effect of STING binding on the fluorescene signal of 2'3'fCDN, STING-dependent 2'3'fGAMP assays were performed as follows:

Lyophilized was dissolved in water, diluted for the experiment to the final absorption 2.34 at 250 nm ($A_{250}$=2.34) and mixed with 0-250 $\mu$M human truncated STING ($hSTING^{cd}$). The samples were incubated for 15 min at room temperature and measured in 96-well black non-binding PS plates (Greiner Bio-One) on Tecan infinite M1000 ($\lambda_{ex}$=305 nm, $\lambda_{em}$=363 nm, gain 140, settle time 10ms) at 25°C.

The data indicate that fluorescence considerably decreases with increasing concentrations of STING, suggesting that STING-binding induces fluorescence quenching in 2'3'fCDN (see Figure 10b). Thus, while incorporation of 2-AP into 2'3'fGAMP quenches the fluorescence signal of 2-APTP as observed in free solution (see e.g. Example 9), the binding of 2'3'fGAMP to STING quenches the fluorescence signal of 2-AP even further. The results shown in Figure 10b therefore indicate that 2'3'fCDN can be used in a method to study 2'3'fCDN binding to STING either in the presence of a substance or in the absence of a substance.

The quenching effect of STING on 2'3'fGAMP can be further used to search and compare STING-binding compounds. The competition assays with 2'3'-fGAMP-bound STING were performed as follows:

Lyophilized was dissolved in water, diluted for the experiment to the final absorption 2.34 at 250 nm ($A_{250}$=2.34) and mixed with 200 $\mu$M human truncated STING ($hSTING^{cd}$). The samples were incubated for 15 min at room temperature after which 0-150 $\mu$M cGAMP 2'3' or c-di-GMP were added. After second round of incubation for 15 min the samples were measured in 96-well black non-binding PS plates (Greiner Bio-One) on Tecan infinite M1000 ($\lambda_{ex}$=305 nm, $\lambda_{em}$=363 nm, gain 140, settle time 10ms) at 25°C.

The data show that adding a STING-binding molecule such as 2'3'cGAMP leads to an increase of observed fluorescent signal (Figure 10c). Such molecules compete for STING binding, leading to the release of 2'3'fGAMP from

the STING-2'3'fGAMP complex. So the quenched fluorescence of 2'3'fGAMP can be regained. Comparison of two STING-binding molecules with different affinities - 2'3'cGAMP and c-di-GMP - shows different efficiency of STING binding. In particular, if comparing the increase in fluorescence by increasing concentrations of STING-binding molecules, those with higher affinity towards STING (e.g. 2'3'cGAMP) will show a more rapid fluorescence increase than those with lower affinity (e.g. c-di-GMP) (compare s1 and s2 from Figure 10d).

Thus, the STING-dependent 2'3'fCDN quenching assay provides a robust tool for the identification of substances having an ability to bind to STING as discussed above.

## References

Ablasser, A., M. Goldeck, et al. (2013). "cGAS produces a 2[prime]-5[prime]-linked cyclic dinucleotide second messenger that activates STING." Nature 498(7454): 380-384.

Ablasser, A., J. L. Schmid-Burgk, et al. (2013). "Cell intrinsic immunity spreads to bystander cells via the intercellular transfer of cGAMP." Nature 503(7477): 530-534.

Ahn, J., P. Ruiz, et al. (2014). "Intrinsic Self-DNA Triggers Inflammatory Disease Dependent on STING." The Journal of Immunology.

Cavlar, T., T. Deimling, et al. (2013). "Species-specific detection of the antiviral small□molecule compound CMA by STING." The EMBO Journal 32(10): 1440-1450.

Civril, F., T. Deimling, et al. (2013). "Structural mechanism of cytosolic DNA sensing by cGAS." Nature 498(7454): 332-337.

Crow, Y. J., D. S. Chase, et al. (2015). "Characterization of Human Disease Phenotypes Associated with Mutations in TREX1, RNASEH2A, RNASEH2B, RNASEH2C, SAMHD1, ADAR, and IFIH1." American journal of medical genetics. Part A 0(2): 296-312.

Crow, Y. J., B. E. Hayward, et al. (2006). "Mutations in the gene encoding the 3[prime]-5[prime] DNA exonuclease TREX1 cause Aicardi-Goutieres syndrome at the AGS1 locus." Nat Genet 38(8): 917-920.

Dey, R. J., B. Dey, et al. (2017). "Inhibition of innate immune cytosolic surveillance by an M. tuberculosis phosphodiesterase." Nat Chem Biol 13(2): 210-217.

Gaidt, M. M., T. S. Ebert, et al. (2016). "Human Monocytes Engage an Alternative Inflammasome Pathway." Immunity 44(4): 833-846.

Gall, A., P. Treuting, et al. (2012). "Autoimmunity Initiates in Nonhematopoietic Cells and Progresses via Lymphocytes in an Interferon-Dependent Autoimmune Disease." Immunity 36(1): 120-131.

Gao, D., T. Li, et al. (2015). "Activation of cyclic GMP-AMP synthase by self-DNA causes autoimmune diseases." Proceedings of the National Academy of Sciences 112(42): E5699-E5705.

Gao, P., M. Ascano, et al. (2013). "Structure-Function Analysis of STING Activation by c[G(2',5')pA(3',5')p] and Targeting by Antiviral DMXAA." Cell 154(4): 748-762.

Gao, P., T. Zillinger, et al. (2014). "Binding-Pocket and Lid-Region Substitutions Render Human STING Sensitive to the Species-Specific Drug DMXAA." Cell Reports 8(6): 1668-1676.

Hughes, R. A., A. E. Miklos, et al. (2011). Chapter twelve - Gene Synthesis: Methods and Applications. Methods in Enzymology. V. Christopher, Academic Press. Volume 498: 277-309.

Lakowicz, J. R. (2006). "Principles of Fluorescence Spectroscopy." Springer 3th edition.

Lee-Kirsch, M. A., M. Gong, et al. (2007). "Mutations in the gene encoding the 3[prime]-5[prime] DNA exonuclease TREX1 are associated with systemic lupus erythematosus." Nat Genet 39(9): 1065-1067.

Li, L., Q. Yin, et al. (2014). "Hydrolysis of 2'3'-cGAMP by ENPP1 and design of nonhydrolyzable analogs." Nat Chem Biol 10(12): 1043-1048.

Li, X., C. Shu, et al. "Cyclic GMP-AMP Synthase Is Activated by Double-Stranded DNA-Induced Oligomerization." Immunity 39(6): 1019-1031.

Mackenzie, N. C. W., C. Huesa, et al. (2012). "New insights into NPP1 function: Lessons from clinical and animal studies." Bone 51(5): 961-968.

Nowarski, R., N. Gagliani, et al. (2013). "Innate Immune Cells in Inflammation and Cancer." Cancer Immunology Research 1(2): 77-84.

Rapino, F., Eloy F. Robles, et al. (2013). "C/EBPα Induces Highly Efficient Macrophage Transdifferentiation of B Lymphoma and Leukemia Cell Lines and Impairs Their Tumorigenicity." Cell Reports 3(4): 1153-1163.

Roembke, B. T., J. Zhou, et al. (2014). "A cyclic dinucleotide containing 2-aminopurine is a general fluorescent sensor for c-di-GMP and 3',3'-cGAMP." Molecular BioSystems 10(6): 1568-1575.

Russell, J. F. S. a. D. W. (2012). "Molecular Cloning, a laboratory manual." Cold Spring Harbor Laboratory Press 1(4th edition).

Sortica, D. A., M. P. Buffon, et al. (2015). "Association between the ENPP1 K121Q Polymorphism and Risk of Diabetic Kidney Disease: A Systematic Review and Meta-Analysis." PLOS ONE 10(3): e0118416.
Tang, E. D. and C.-Y. Wang (2015). "Single Amino Acid Change in STING Leads to Constitutive Active Signaling." PLOS ONE 10(3): e0120090.
Throop, A. L. and J. LaBaer (2015). "Recombinational Cloning Using Gateway and In-Fusion Cloning Schemes." Current protocols in molecular biology / edited by Frederick M. Ausubel ... [et a1.] 110: 3.20.21-23.20.23.
Woo, S.-R., Mercedes B. Fuertes, et al. (2014). "STING-Dependent Cytosolic DNA Sensing Mediates Innate Immune Recognition of Immunogenic Tumors." Immunity 41(5): 830-842.

## Claims

1. A cyclic dinucleotide comprising a 3'-5' phosphodiester linkage and a 2'-5' phosphodiester linkage between a first nucleoside monophosphate and a second nucleoside monophosphate,
wherein at least one of the first and second nucleoside monophosphates is a fluorescent nucleoside monophosphate.

2. The cyclic dinucleotide according to claim 1, wherein

   • the 3'-5' phosphodiester linkage is between the 3'-oxygen of the first nucleoside monophosphate and the 5'-oxygen of the second nucleoside monophosphate, and
   • the 2'-5' phosphodiester linkage is between the 5'-oxygen of the first nucleoside monophosphate and the 2'-oxygen of the second nucleoside monophosphate.

3. The cyclic dinucleotide according to claims 1 or 2, wherein the fluorescent nucleoside monophosphate is a fluorescent purine nucleoside monophosphate.

4. The cyclic dinucleotide according to claims 1 or 2, wherein the fluorescent nucleoside monophosphate is selected from the group consisting of a 2-aminopurine nucleoside monophosphate (2-APMP), a 3-methyl-isoxanthopterin nucleoside monophosphate (3-MIMP), a 6-methyl isoxanthopterin nucleoside monophosphate (6-MIMP), 4-amino-6-methyl-8-(2-deoxy-beta-d-ribofuranosyl)-7(8H)-pteridone nucleoside monophosphate (6-MAPMP), a 4-amino-2,6-dimethyl-8-(2'-deoxy-beta-d-ribofuranosyl)-7(8H)-pteridone nucleoside monophosphate (DMAPMP), a pyrrolocytosine nuceloside monophosphate (pyrrolo-CMP), a 6-phenylpyrrolocytosine nucleoside monophosphate (PhpCMP), a (aminoethoxy)phenylpyrrolocytosine nucleoside monophosphate (moPhpCMP), a [bis-o-(aminoethoxy)phenyl]pyrrolocytosine nucleoside monophosphate (boPhpCMP), a hydropyrimidopyrimidine nucleoside monophosphate ($C^{hpp}$MP), a pyrrolopyrimidopyrimidine nucleoside monophosphate ($C^{ppp}$MP), a pyrimidopyrimidoindole nucleoside monophosphate ($C^{ppi}$MP), a benzopyridopyrimidine nucleoside monophosphate (BPPMP), a naphthopyridopyrimidine nucleoside monophosphate (NPPMP), a methoxybenzodeazaadenine nucleoside monophosphate ($^{MD}$AMP), a methoxybenzodeazainosine nucleoside monophosphate ($^{MD}$IMP), a naphthodeazaadenine nucleoside monophosphate ($^{ND}$AMP), a furan-modified pyrimidine nucleoside mohophosphate, a thieno[3,2]pyrimidine nucleoside monophosphate, a thieno[3,4]pyrimidine nucleoside monophosphate, a 5-methoxy-quinazoline-2,4-(1H,3H) dione nucleoside monophosphate, a 5-methylpyrimidine-2-one nucleoside monophosphate, a 7-deazapurine nucleoside monophosphate, a 5-alkyluridine nucleoside monophosphate, a benzoquinalozines nucleoside monophosphate, a triazoleadenosine nucleoside monophosphate, and a 1,$N^6$-ethenoadenosine nucleoside monophosphate.

5. The cyclic dinucleotide according to claim 4, wherein the 2-APMP is the first nucleoside monophosphate.

6. The cyclic dinucleotide according to claim any one of claims 1 to 5, having the following formula:

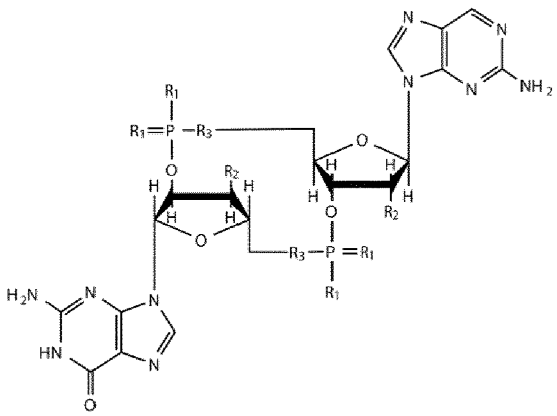
,

wherein

when $R_1$ is bound to P through a single bond, $R_1$ is independently selected from the group consisting of O, S, $BH_3$ and $CH_3$;
when $R_1$ is bound to P through a double bond, $R_1$ is independently selected from the group consisting of O, S and NH;
wherein at least one $R_1$ bound to each P is O;
$R_2$ is independently selected from the group consisting of H, OH, methyl, amino-, methoxy-, fluoro-, methoxyethyl-, -O-propargyl and O-propylamine; and
$R_3$ is O.

**7.** The cyclic dinucleotide according to any one of claims 1 to 6 having the following formula:

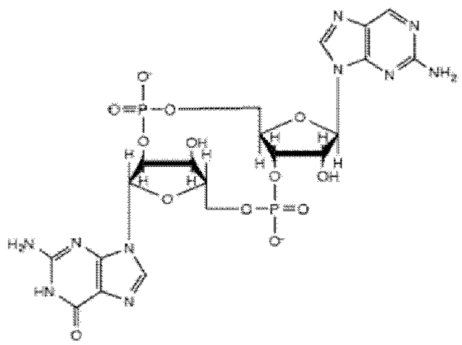
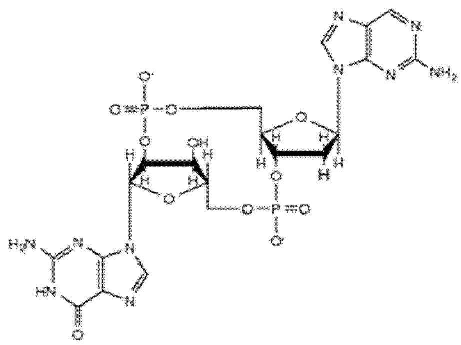
or

or

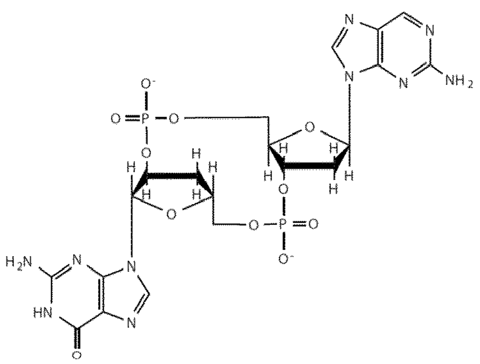
or
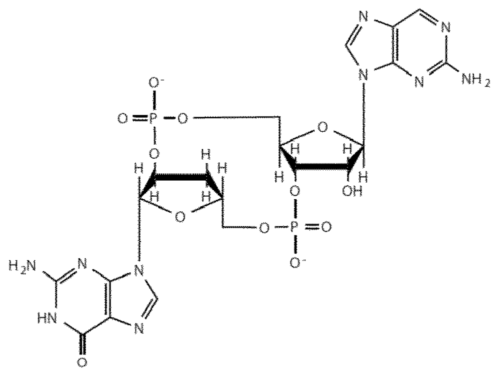
.

**8.** A method of measuring cGAS activity, the method comprising:

(i) providing an aqueous solution comprising cGAS, cGAS-activating nucleic acid, a first nucleoside triphosphate, a second nucleoside triphosphate, and one or more divalent cation, wherein at least one of the first and second nucleoside triphosphates is a fluorescent nucleoside triphosphate, and wherein one of the first and second nucleoside triphosphates has a free 2' hydroxyl group and the other one has a free 3' hydroxyl group; and
(ii) measuring the change of the fluorescence signal of the aqueous solution over time.

**9.** A method of identifying a substance having an ability to modulate the activity of cyclic GMP-AMP synthase (cGAS), the method comprising:

(i) providing an aqueous solution comprising cGAS, cGAS-activating nucleic acid, a first nucleoside triphosphate, a second nucleoside triphosphate, and one or more divalent cation, wherein at least one of the first and second nucleoside triphosphates is a fluorescent nucleoside triphosphate, and wherein one of the first and second nucleoside triphosphates has a free 2' hydroxyl group and the other one has a free 3' hydroxyl group;
(ii) measuring the fluorescence signal of the aqueous solution;
(iii) repeating steps (i) and (ii), wherein upon said repetition the aqueous solution further comprises the substance, and wherein the fluorescence signal is measured under identical or substantially identical conditions following the provision of the respective aqueous solution; and
(iv) comparing the fluorescence signal measured in the presence of the substance with the fluorescence signal measured in the absence of the substance;

wherein a measured fluorescence signal which is higher in the presence of the substance than in the absence of the substance indicates that the substance is a cGAS antagonist, while a measured fluorescence signal which is lower in the presence of the substance than in the absence of the substance indicates that the substance is a cGAS agonist.

**10.** A method of preparing a cyclic dinucleotide according to any one of claims 1 to 7, the method comprising:

(i) providing an aqueous solution comprising cGAS, cGAS activating nucleic acid, a first nucleoside triphosphate, a second nucleoside triphosphate, and one or more divalent cation, wherein at least one of the first and second nucleoside triphosphates is a fluorescent nucleoside triphosphate, and wherein one of the first and second nucleoside triphosphates has a free 2' hydroxyl group and the other one has a free 3' hydroxyl group, thereby preparing the cyclic dinucleotide; and optionally
(ii) purifying the cyclic dinucleotide.

**11.** The method according to any one of claims 8 to 10, wherein the fluorescent nucleoside triphosphate is 2-aminopurine nucleoside triphosphate (2-APTP).

**12.** A method of identifying a substance having an ability to bind to stimulator of interferon genes (STING), the method comprising:

(i) providing an aqueous solution comprising a cyclic dinucleotide and STING, wherein the cyclic dinucleotide comprises a 3'-5' phosphodiester linkage and a 2'-5' phosphodiester linkage between a first nucleoside monophosphate and a second nucleoside monophosphate, wherein at least one of the first and second nucleoside monophosphates is a fluorescent nucleoside monophosphate;
(ii) measuring the fluorescence signal of the aqueous solution;
(iii) repeating steps (i) and (ii), wherein upon said repetition the aqueous solution further comprises the substance, and wherein the fluorescence is measured under identical or substantially identical conditions following the provision of the respective aqueous solution; and
(iv) comparing the fluorescence signal measured in the presence of the substance with the fluorescence signal measured in the absence of the substance;

wherein a measured fluorescence signal which is higher in the presence of the substance than in the absence of the substance indicates that the substance has an ability to bind to STING.

**13.** A method of identifying a substance having an ability to modulate the activity of a 2'-5' phosphodiesterase, the method comprising

(i) providing an aqueous solution comprising a cyclic dinucleotide and a 2'-5' phosphodiesterase, wherein the

cyclic dinucleotide comprises a 3'-5' phosphodiester linkage and a 2'-5' phosphodiester linkage between a first nucleoside monophosphate and a second nucleoside monophosphate, wherein at least one of the first and second nucleoside monophosphates is a fluorescent nucleoside monophosphate;
(ii) measuring the fluorescence signal of the aqueous solution;
(iii) repeating steps (i) and (ii), wherein upon said repetition the aqueous solution further comprises the substance, and wherein the fluorescence is measured under identical or substantially identical conditions following the provision of the respective aqueous solution; and
(iv) comparing the fluorescence signal measured in the presence of the substance with the fluorescence signal measured in the absence of the substance;

wherein a measured fluorescence signal which is higher in the presence of the substance than in the absence of the substance indicates that the substance is a 2'-5' phosphodiesterase agonist, while a measured fluorescence signal which is lower in the presence of the substance compared to the absence of the substance indicates that the substance is a 2'-5' phosphodiesterase antagonist.

**14.** The method according to claim 12 or 13, wherein the 3'-5' phosphodiester linkage of the cyclic dinucleotide is between the 3'-oxygen of the first nucleoside monophosphate and the 5'-oxygen of the second nucleoside monophosphate, and
wherein the 2'-5' phosphodiester linkage of the cyclic dinucleotide is between the 5'-oxygen of the first nucleoside monophosphate and the 2'-oxygen of the second nucleoside monophosphate.

**15.** The method according to any one of claims 12 to 14, wherein the fluorescent nucleoside monophosphate is a fluorescent purine nucleoside monophosphate.

**16.** The method according to any one of claims 13 to 15, wherein the first nucleoside monophosphate is 2-aminopurine nucleoside monophosphate (2-APMP).

**17.** The method according to any one of claims 12 to 16, having the following formula:

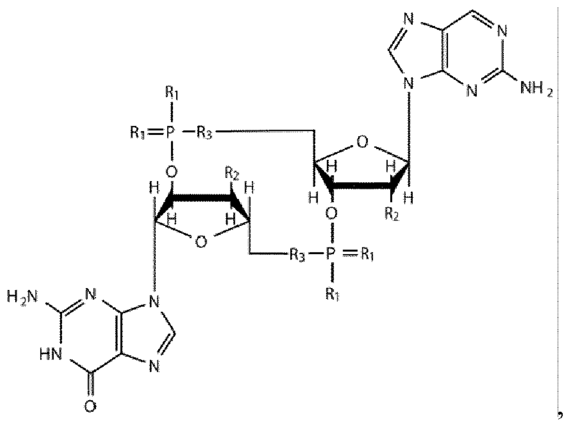

,

wherein

when $R_1$ is bound to P through a single bond, $R_1$ is independently selected from the group consisting of O, S, $BH_3$ and $CH_3$;
when $R_1$ is bound to P through a double bond, $R_1$ is independently selected from the group consisting of O, S and NH;
wherein at least one $R_1$ bound to each P is O;
$R_2$ is independently selected from the group consisting of H, OH, methyl, amino-, methoxy-, fluoro-, methoxyethyl-, -O-propargyl and O-propylamine; and
$R_3$ is O.

**18.** The method according to any one of claims 12 to 17, wherein the cyclic dinucleotide has the following formula:

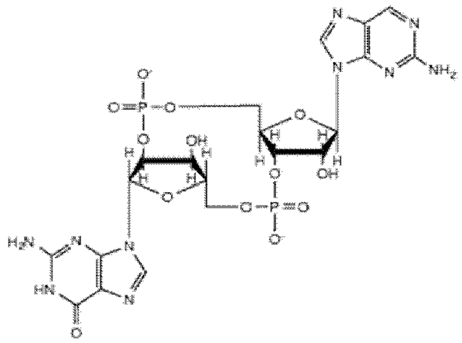

or

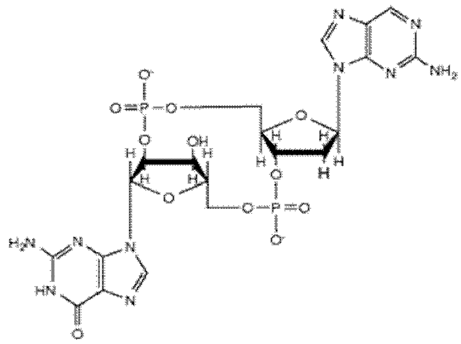

or

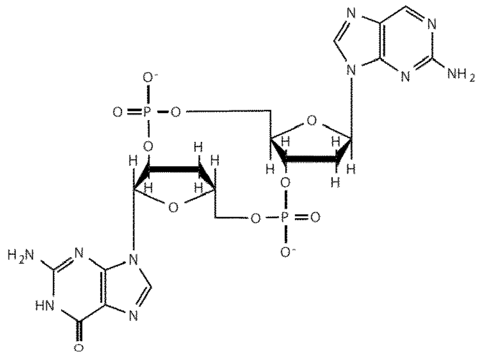

or

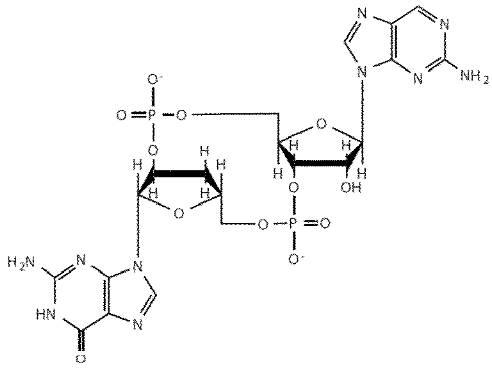

.

**19.** Use of a fluorescent nucleoside triphosphate for measuring cGAS activity or for identifying a substance having an ability to modulate cGAS activity.

**20.** Use of a cyclic dinucleotide according to any one of claims 1 to 7 for identifying a substance with the ability to bind to STING.

**21.** Use of a cyclic dinucleotide according to any one of claims 1 to 7 for labeling cGAS or STING.

**22.** A complex comprising STING and a cyclic dinucleotide according to any one of claims 1-7.

Figure 1

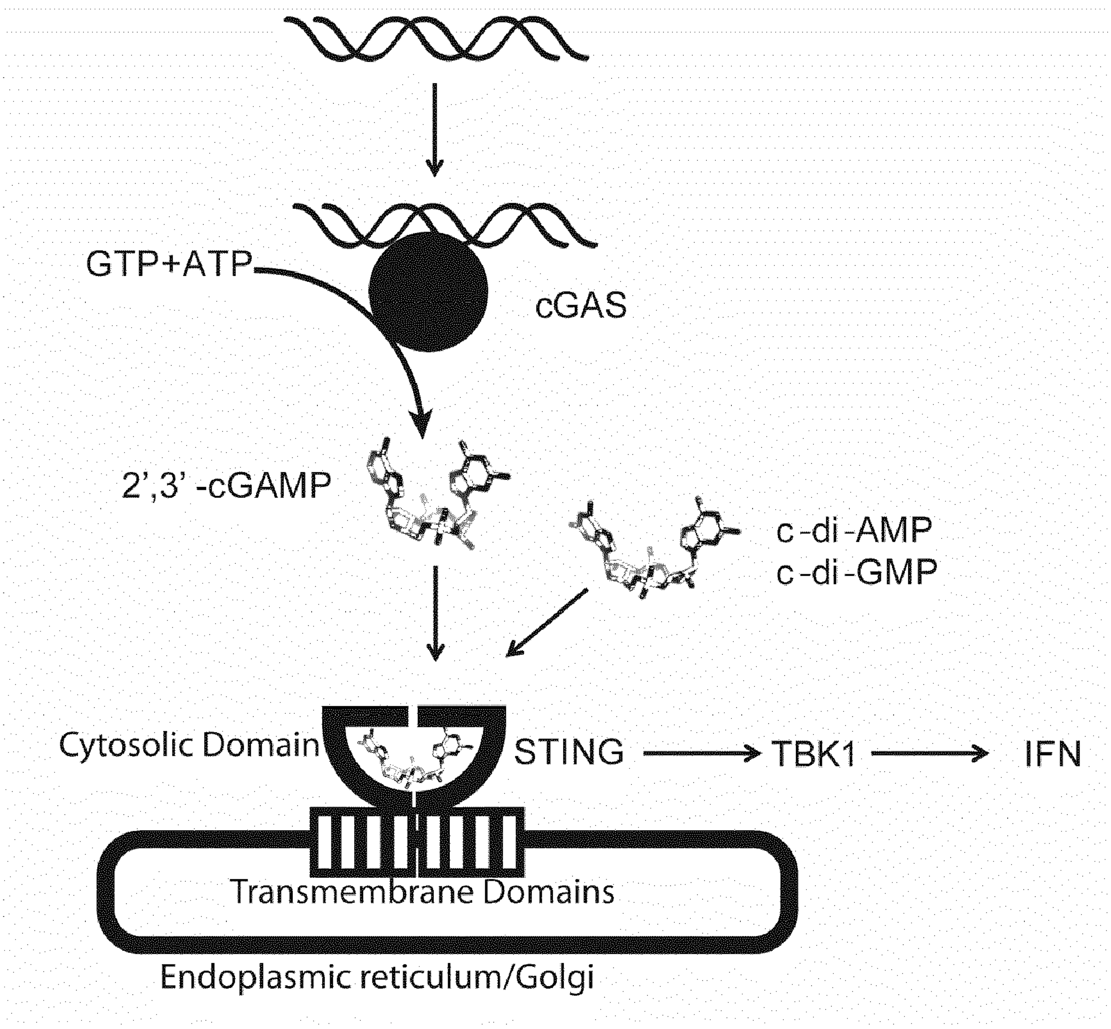
GTP+ATP
cGAS
2',3' -cGAMP
c-di-AMP
c-di-GMP
Cytosolic Domain
STING
TBK1
IFN
Transmembrane Domains
Endoplasmic reticulum/Golgi

Figure 2

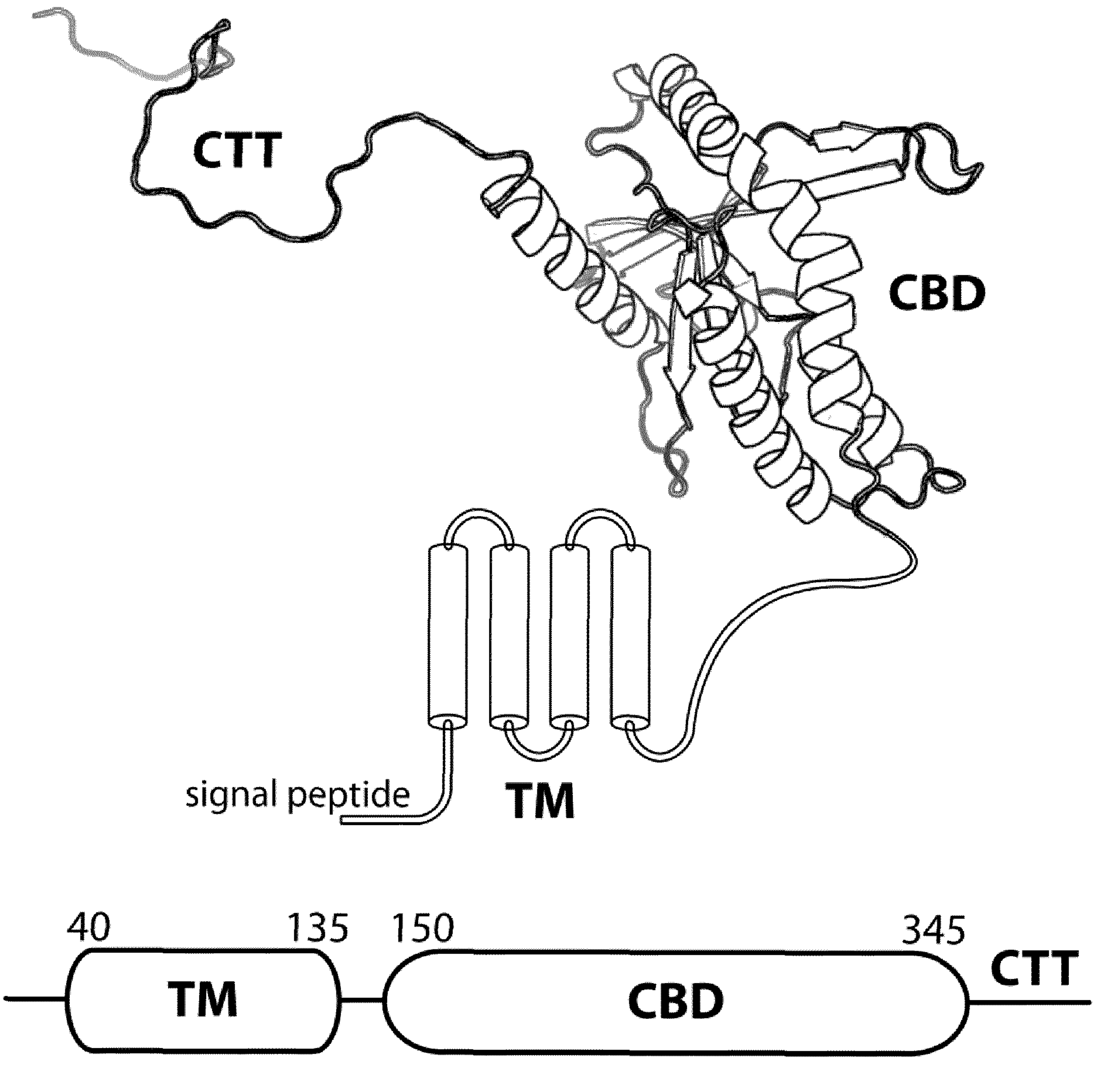

Figure 3

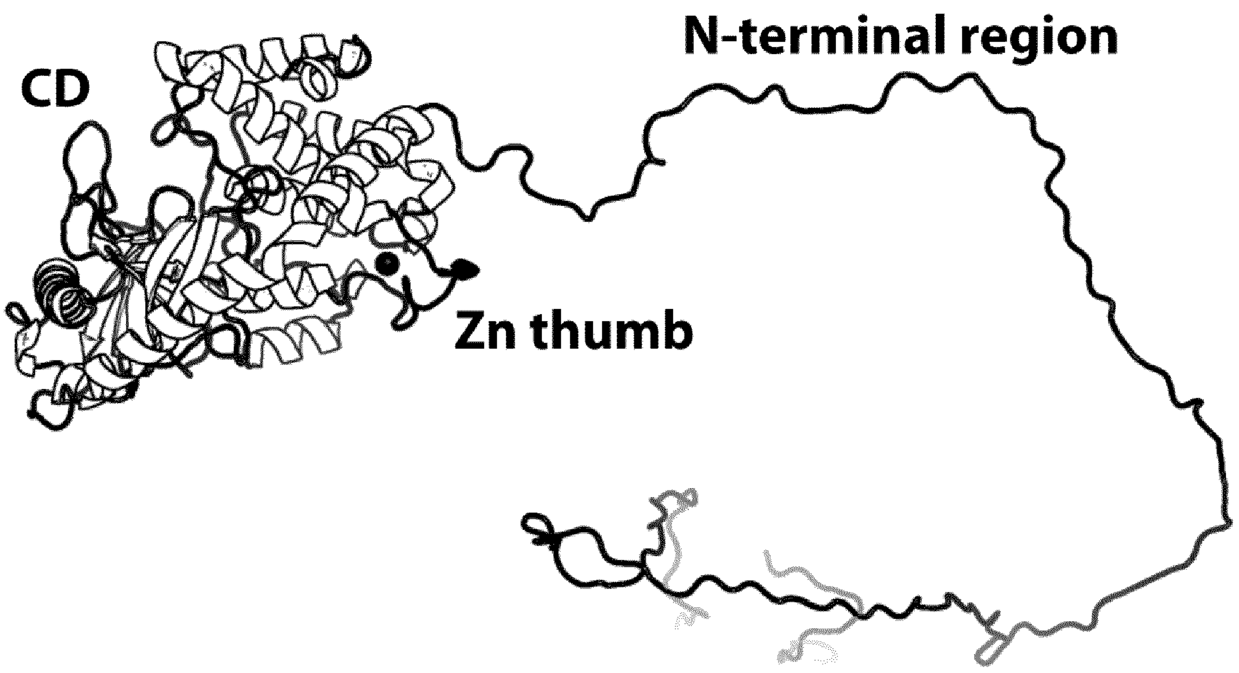


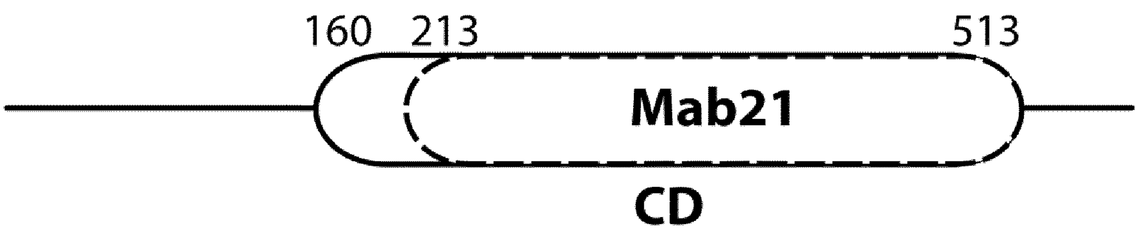

Figure 4

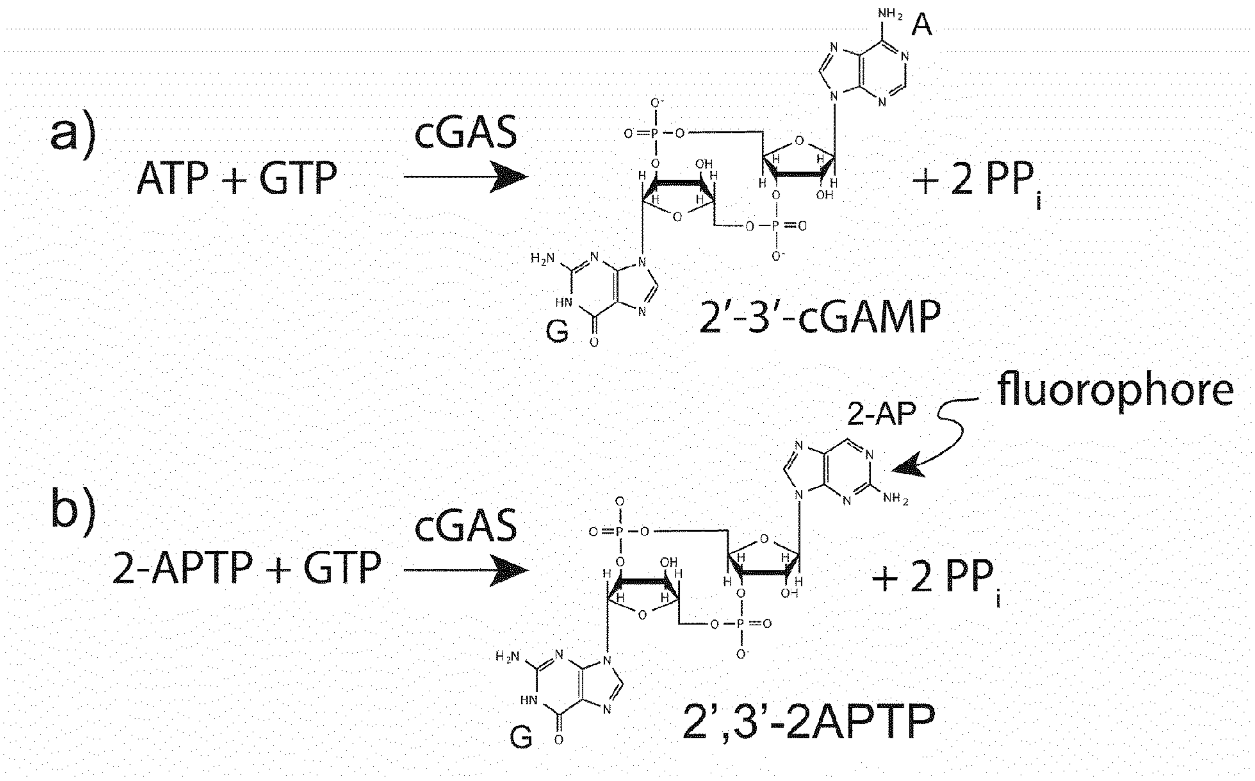
a)
ATP + GTP
cGAS
+ 2 PP$_i$
2'-3'-cGAMP
b)
2-APTP + GTP
cGAS
2-AP
fluorophore
+ 2 PP$_i$
2',3'-2APTP

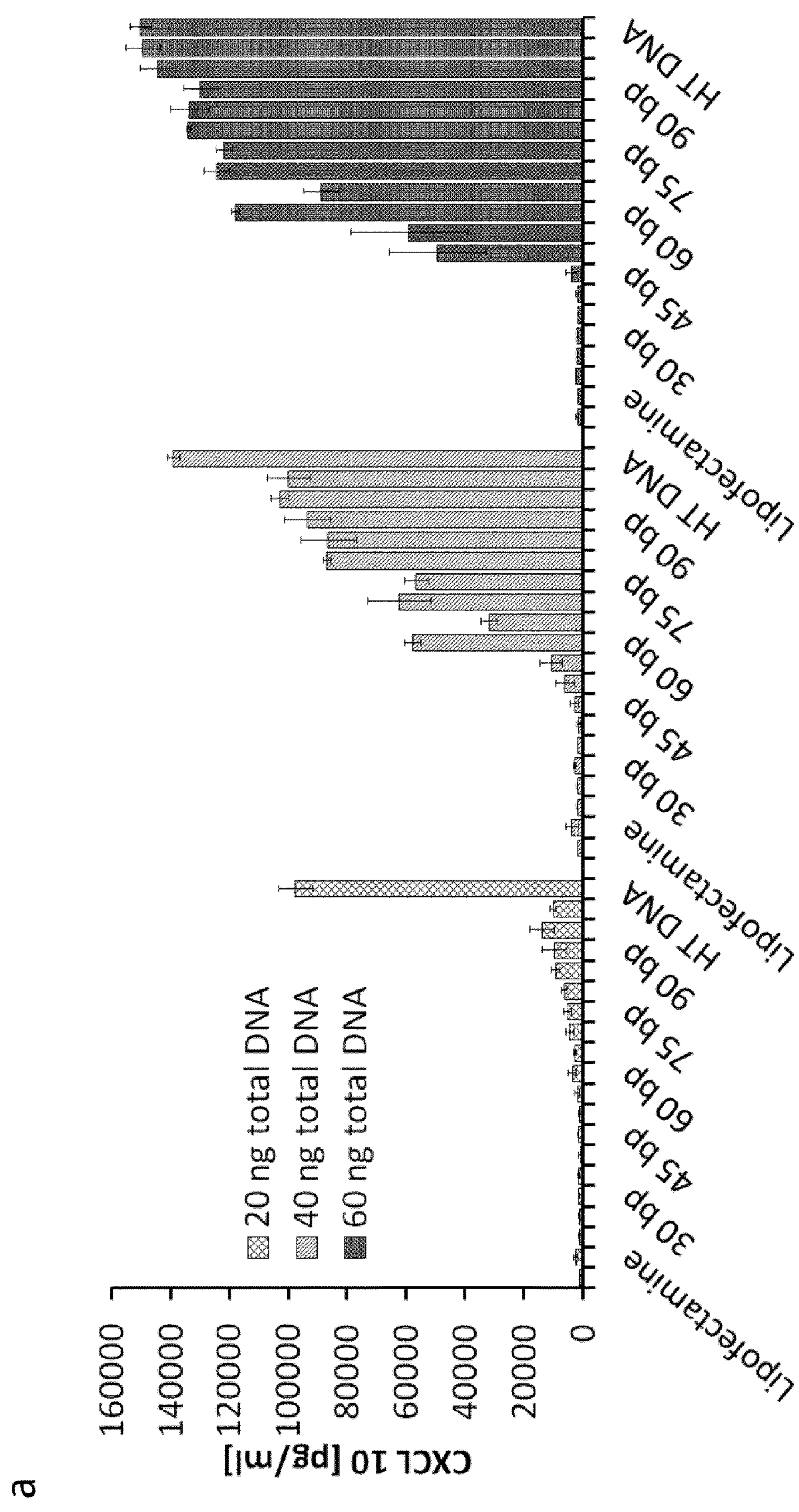
a
CXCL 10 [pg/ml]
0
20000
40000
60000
80000
100000
120000
140000
160000
20 ng total DNA
40 ng total DNA
60 ng total DNA
Lipofectamine
30 bp
45 bp
60 bp
75 bp
90 bp
HT DNA

Figure 5a

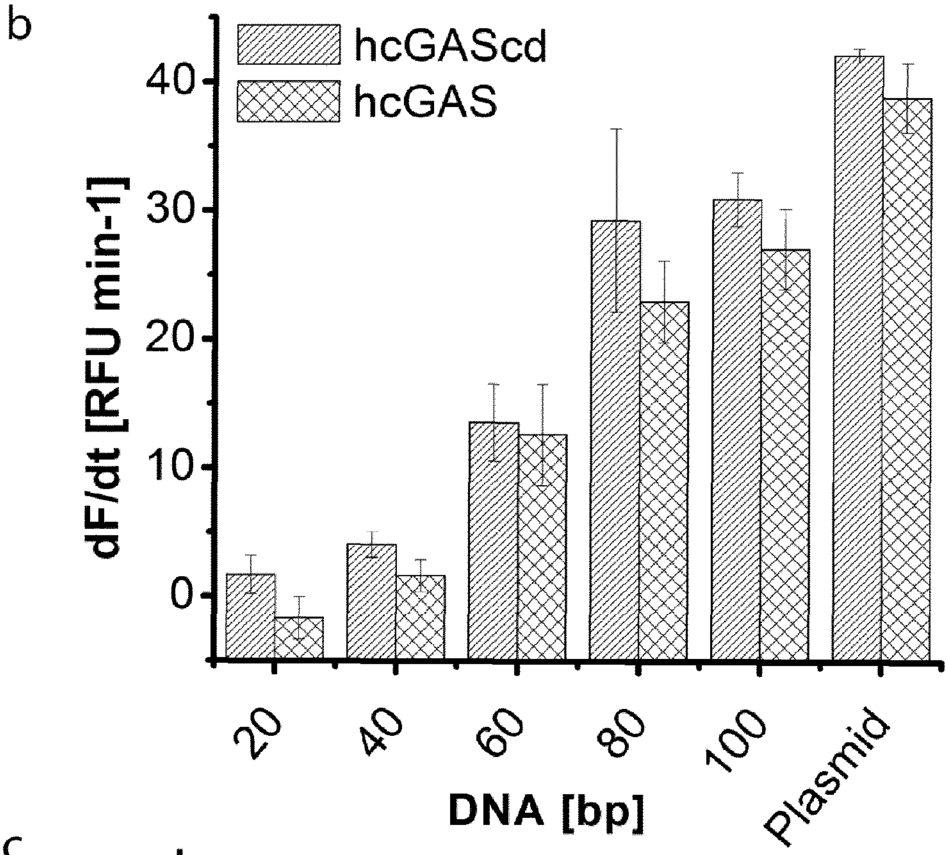
b
hcGAScd
hcGAS
dF/dt [RFU min-1]
0
10
20
30
40
20
40
60
80
100
Plasmid
DNA [bp]

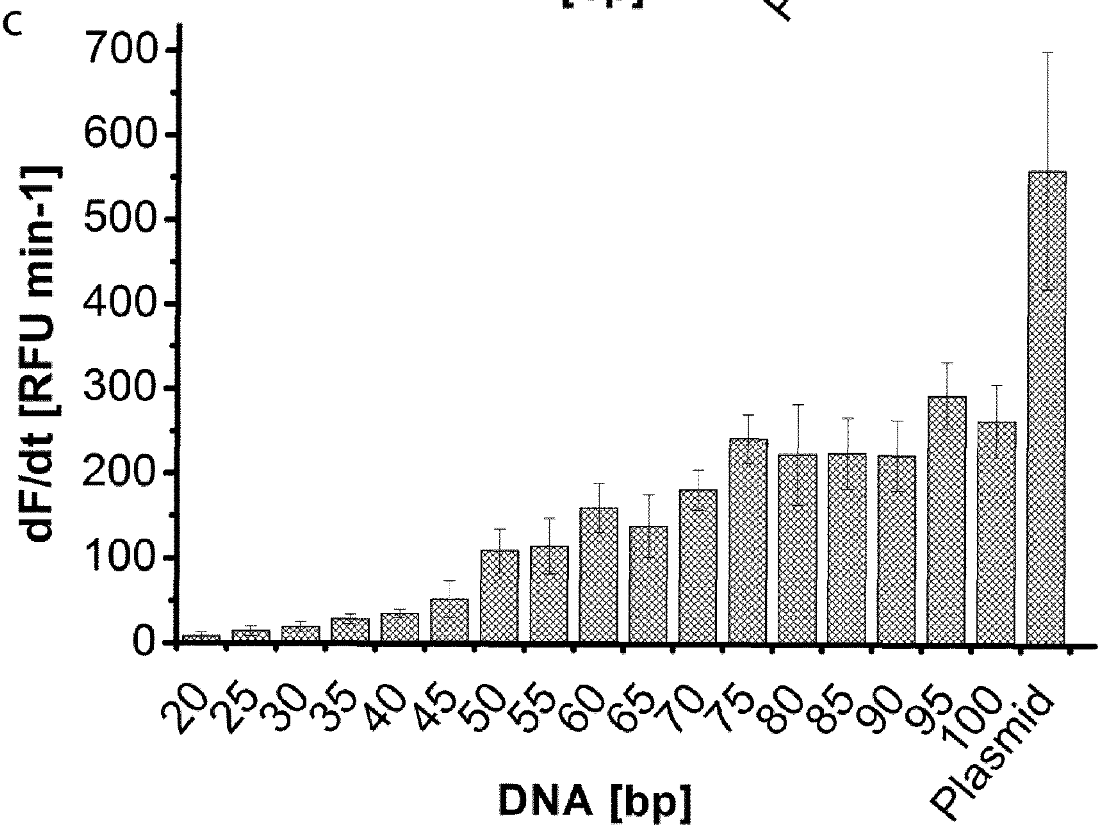
c
dF/dt [RFU min-1]
0
100
200
300
400
500
600
700
20
25
30
35
40
45
50
55
60
65
70
75
80
85
90
95
100
Plasmid
DNA [bp]

Figure 5b_c

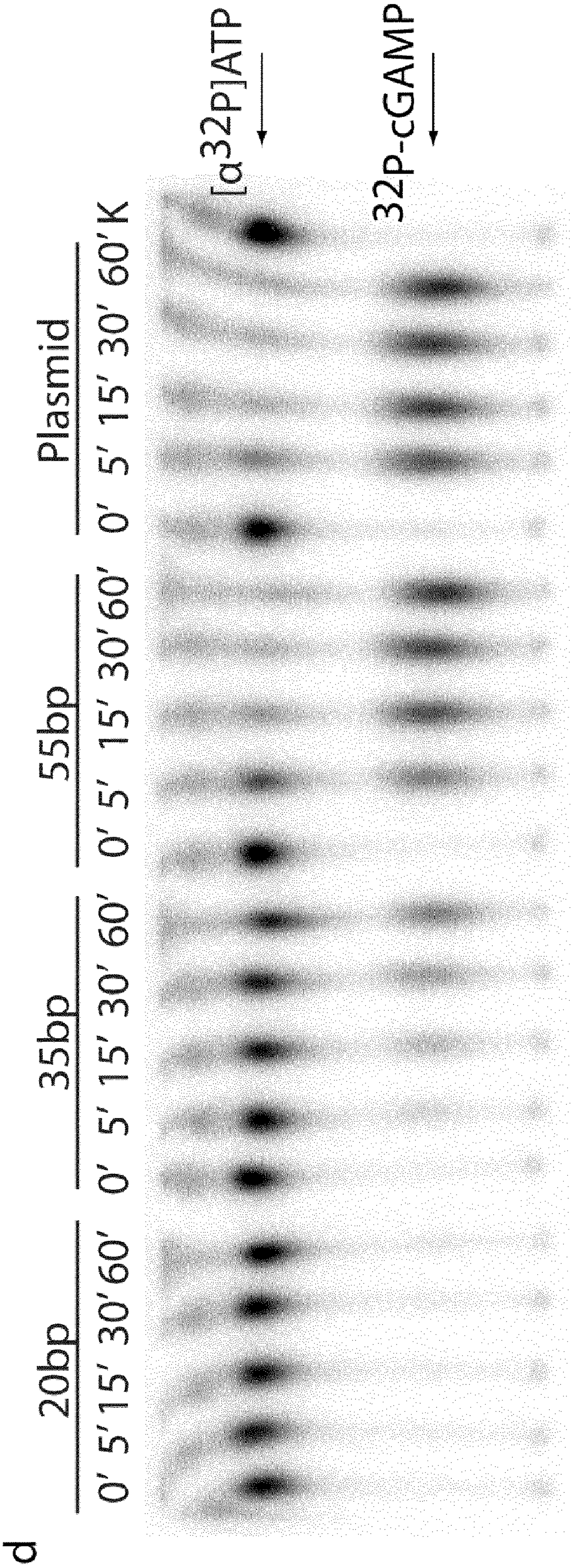
d
20bp
0' 5' 15' 30' 60'
35bp
0' 5' 15' 30' 60'
55bp
0' 5' 15' 30' 60'
Plasmid
0' 5' 15' 30' 60' K
[α32P]ATP
32P-cGAMP

**Figure 5d**

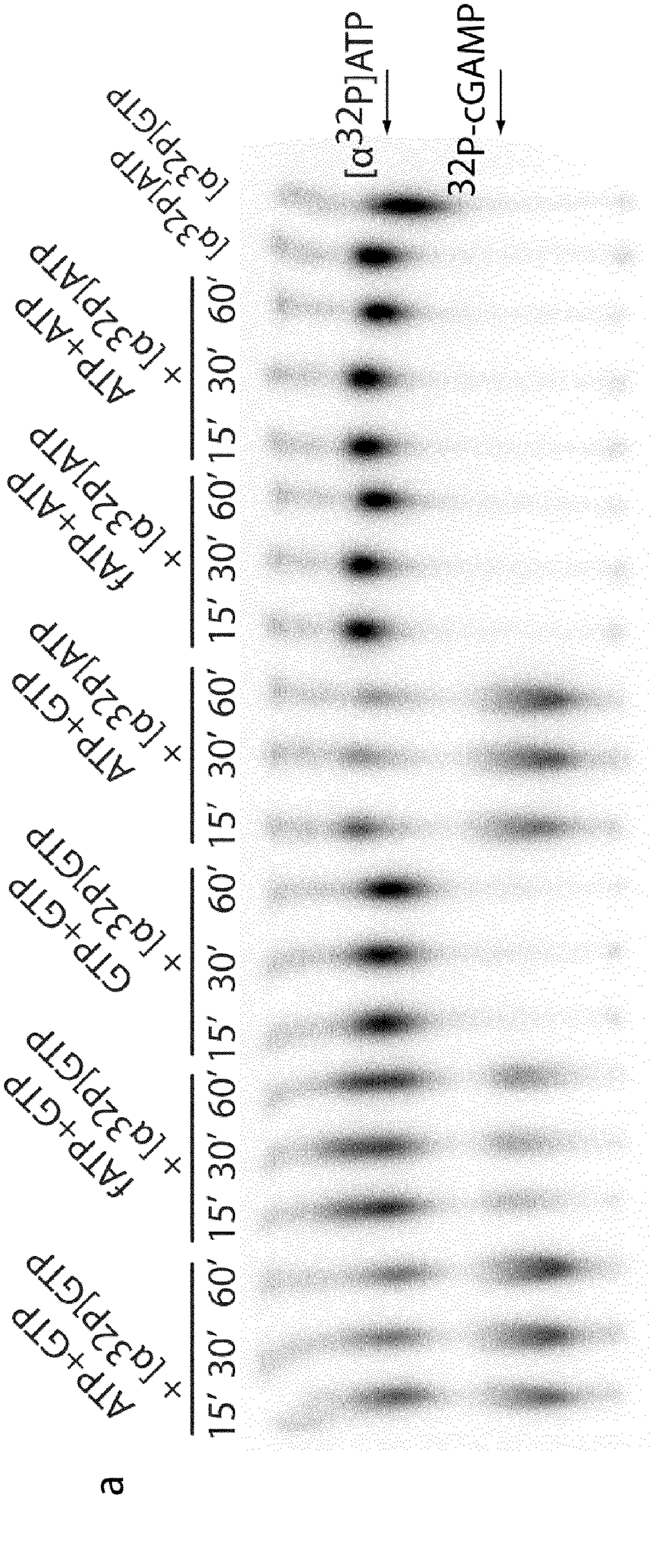
[α32P]ATP
32P-cGAMP
[α32P]GTP
[α32P]ATP
ATP+ATP × [α32P]ATP
15′ 30′ 60′
fATP+ATP × [α32P]ATP
15′ 30′ 60′
ATP+GTP × [α32P]ATP
15′ 30′ 60′
GTP+GTP × [α32P]GTP
15′ 30′ 60′
fATP+GTP × [α32P]GTP
15′ 30′ 60′
ATP+GTP × [α32P]GTP
15′ 30′ 60′
a

Figure 6a

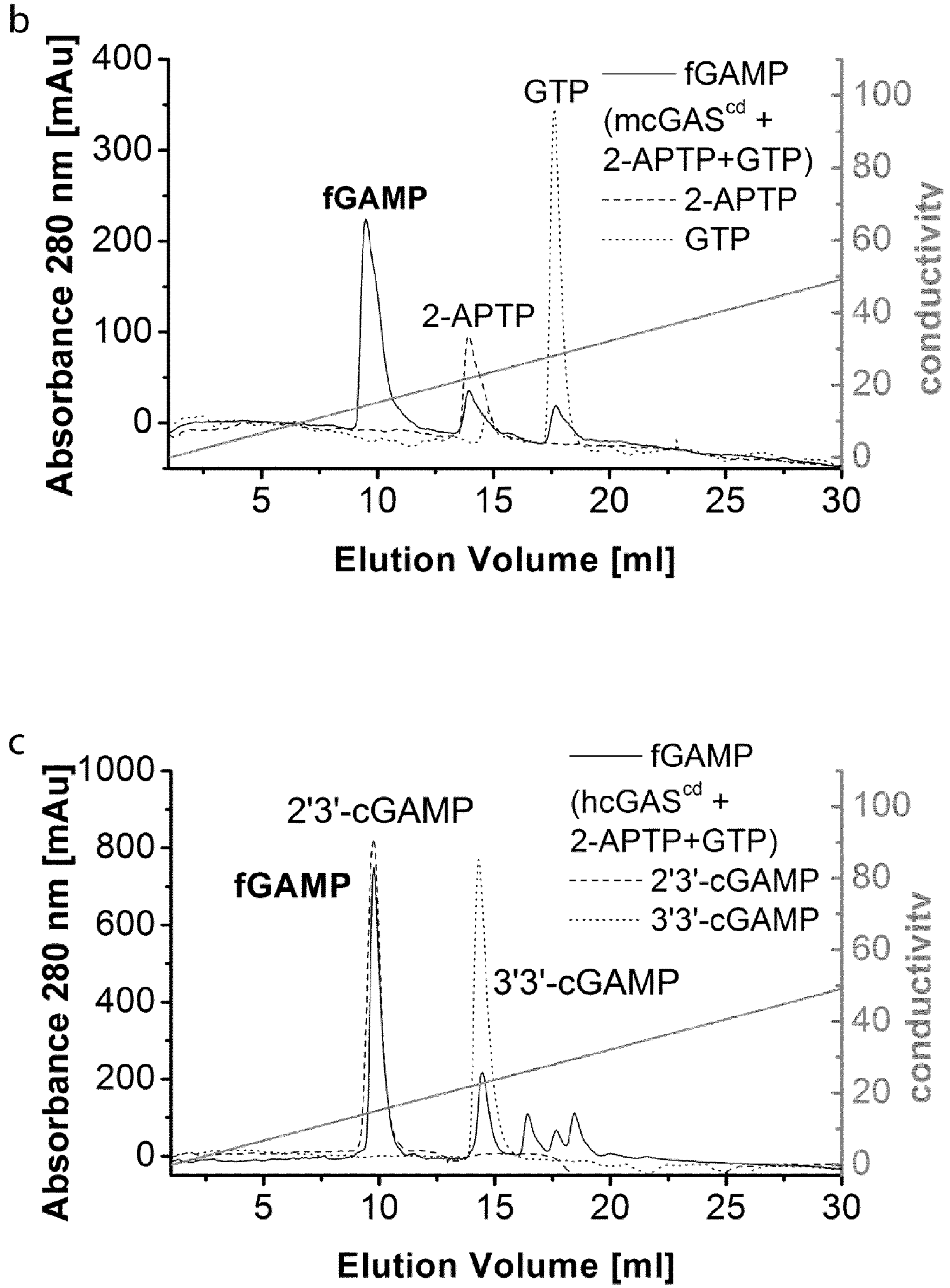
b
fGAMP (mcGAS^cd + 2-APTP+GTP)
2-APTP
GTP
GTP
fGAMP
2-APTP
Absorbance 280 nm [mAu]
conductivity
Elution Volume [ml]
c
fGAMP (hcGAS^cd + 2-APTP+GTP)
2'3'-cGAMP
3'3'-cGAMP
2'3'-cGAMP
fGAMP
3'3'-cGAMP
Absorbance 280 nm [mAu]
conductivity
Elution Volume [ml]

Figure 6bc

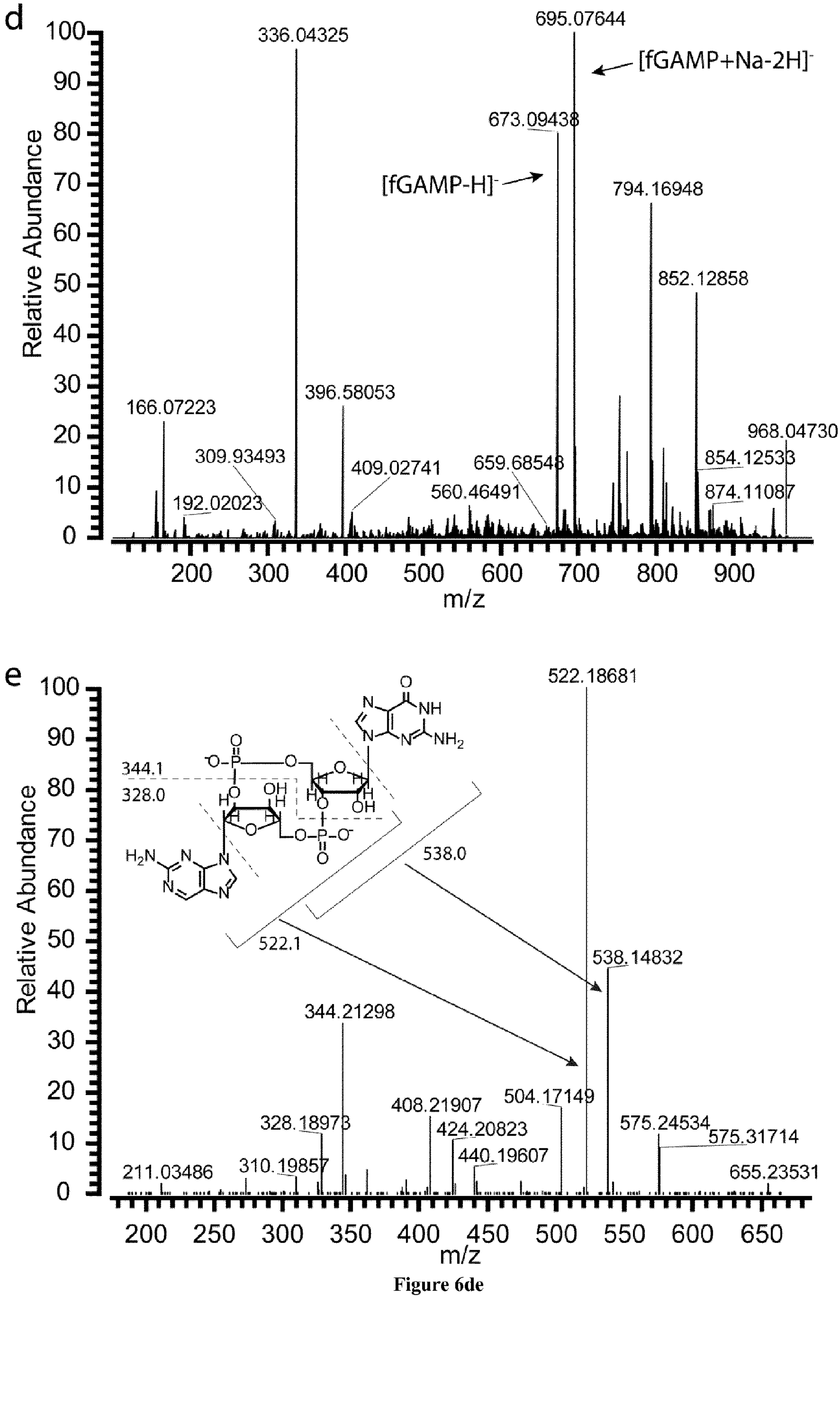
d
Relative Abundance
m/z
336.04325
695.07644
[fGAMP+Na-2H]-
673.09438
[fGAMP-H]-
794.16948
852.12858
396.58053
166.07223
968.04730
309.93493
409.02741
659.68548
854.12533
560.46491
874.11087
192.02023
e
Relative Abundance
m/z
522.18681
344.1
328.0
538.0
522.1
538.14832
344.21298
504.17149
408.21907
328.18973
424.20823
575.24534
575.31714
440.19607
211.03486
310.19857
655.23531

Figure 6de

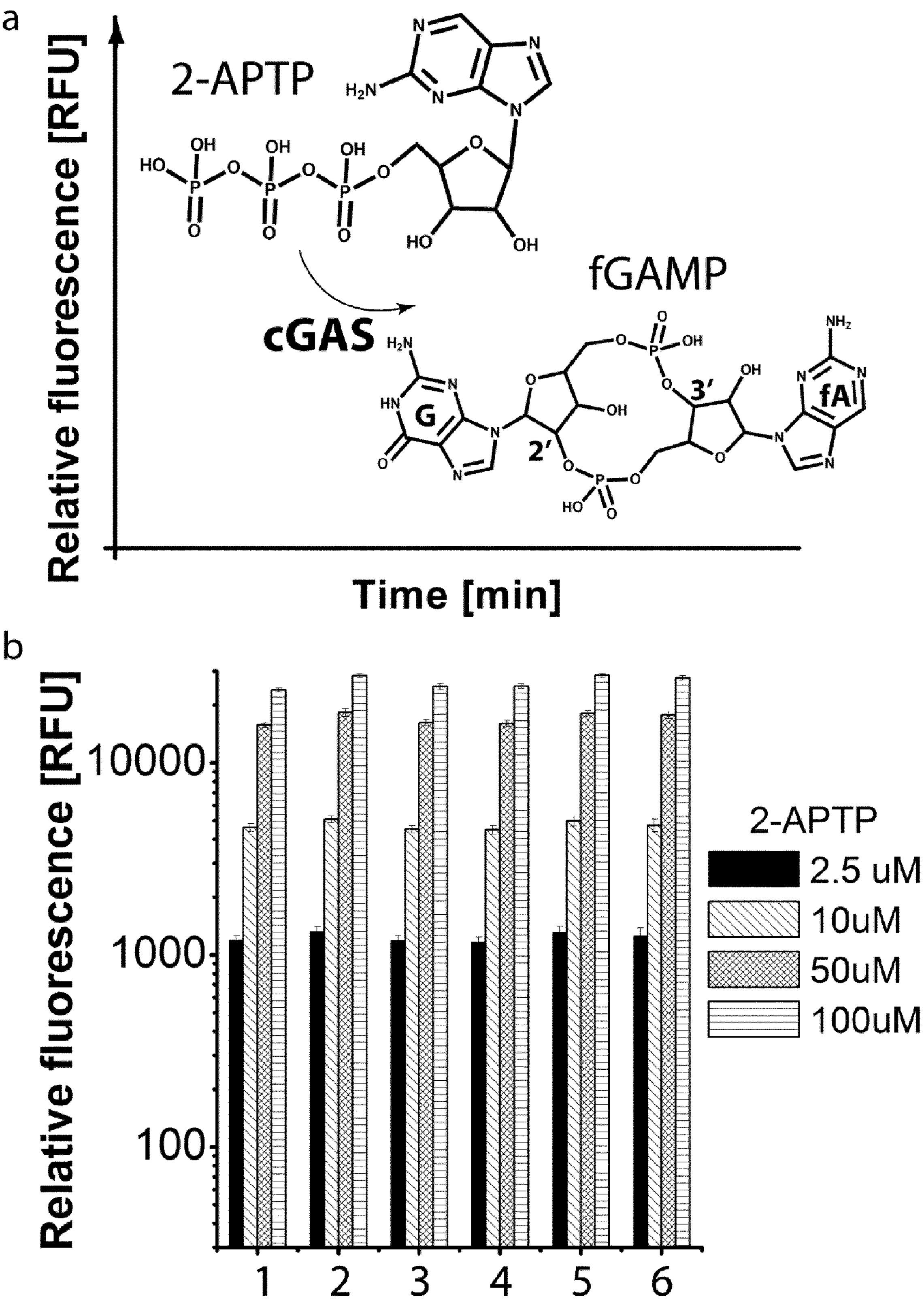
a
Relative fluorescence [RFU]
2-APTP
cGAS
fGAMP
Time [min]
b
Relative fluorescence [RFU]
10000
1000
100
1
2
3
4
5
6
2-APTP
2.5 uM
10uM
50uM
100uM

Figure 7ab

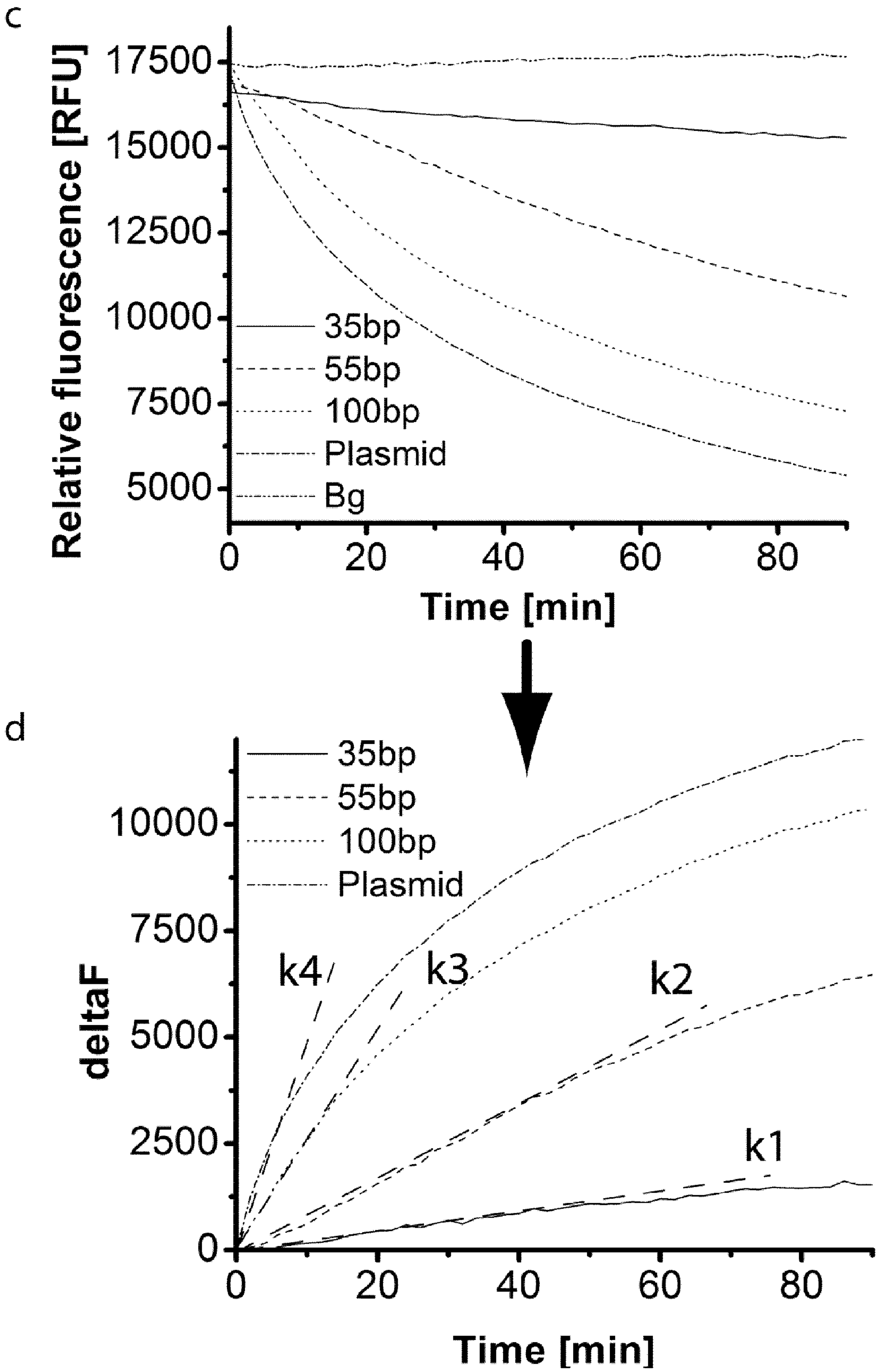
c
Relative fluorescence [RFU]
17500
15000
12500
10000
7500
5000
35bp
55bp
100bp
Plasmid
Bg
0
20
40
60
80
Time [min]
d
deltaF
10000
7500
5000
2500
0
35bp
55bp
100bp
Plasmid
k4
k3
k2
k1
0
20
40
60
80
Time [min]

Figure 7cd

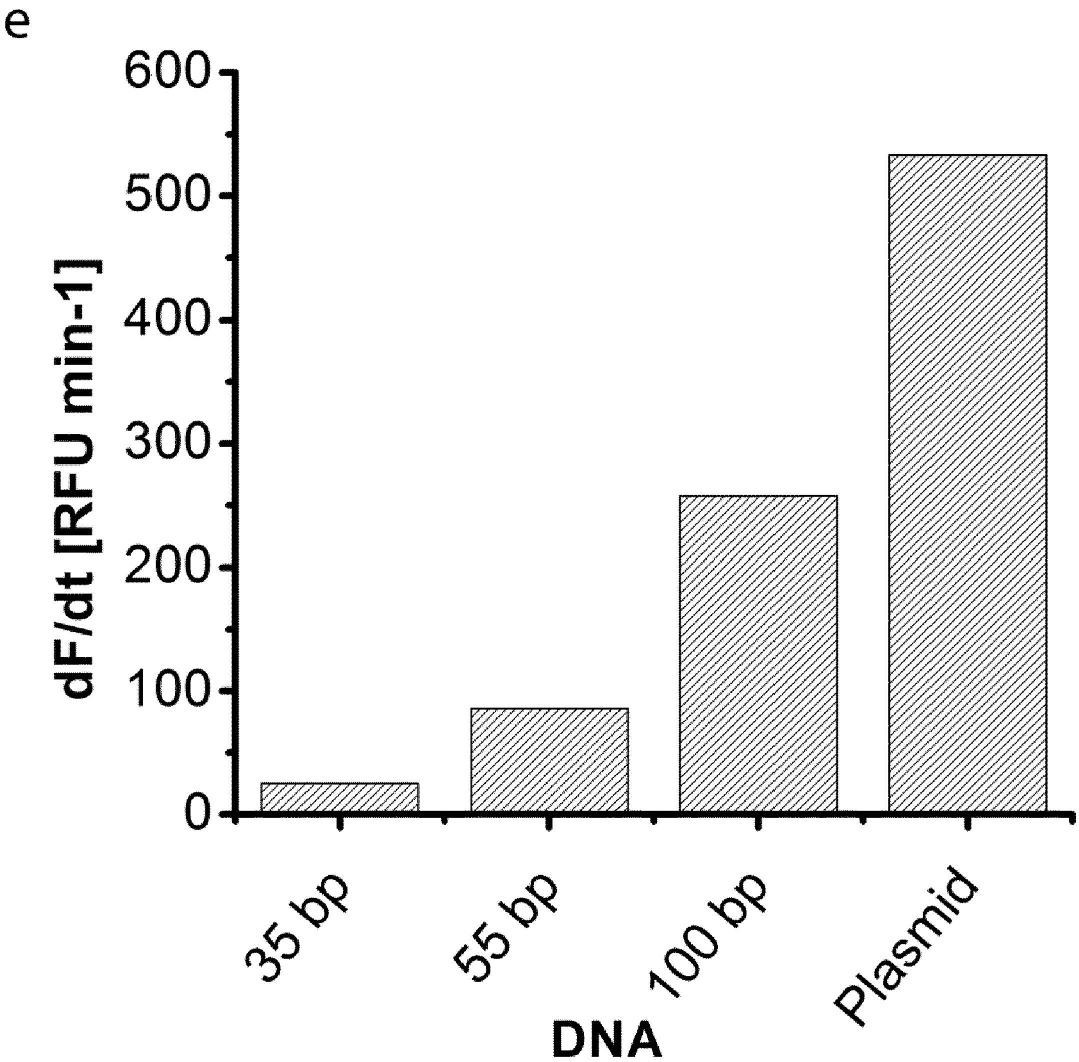
e
600
500
400
300
200
100
0
dF/dt [RFU min-1]
35 bp
55 bp
100 bp
Plasmid
DNA

Figure 7e

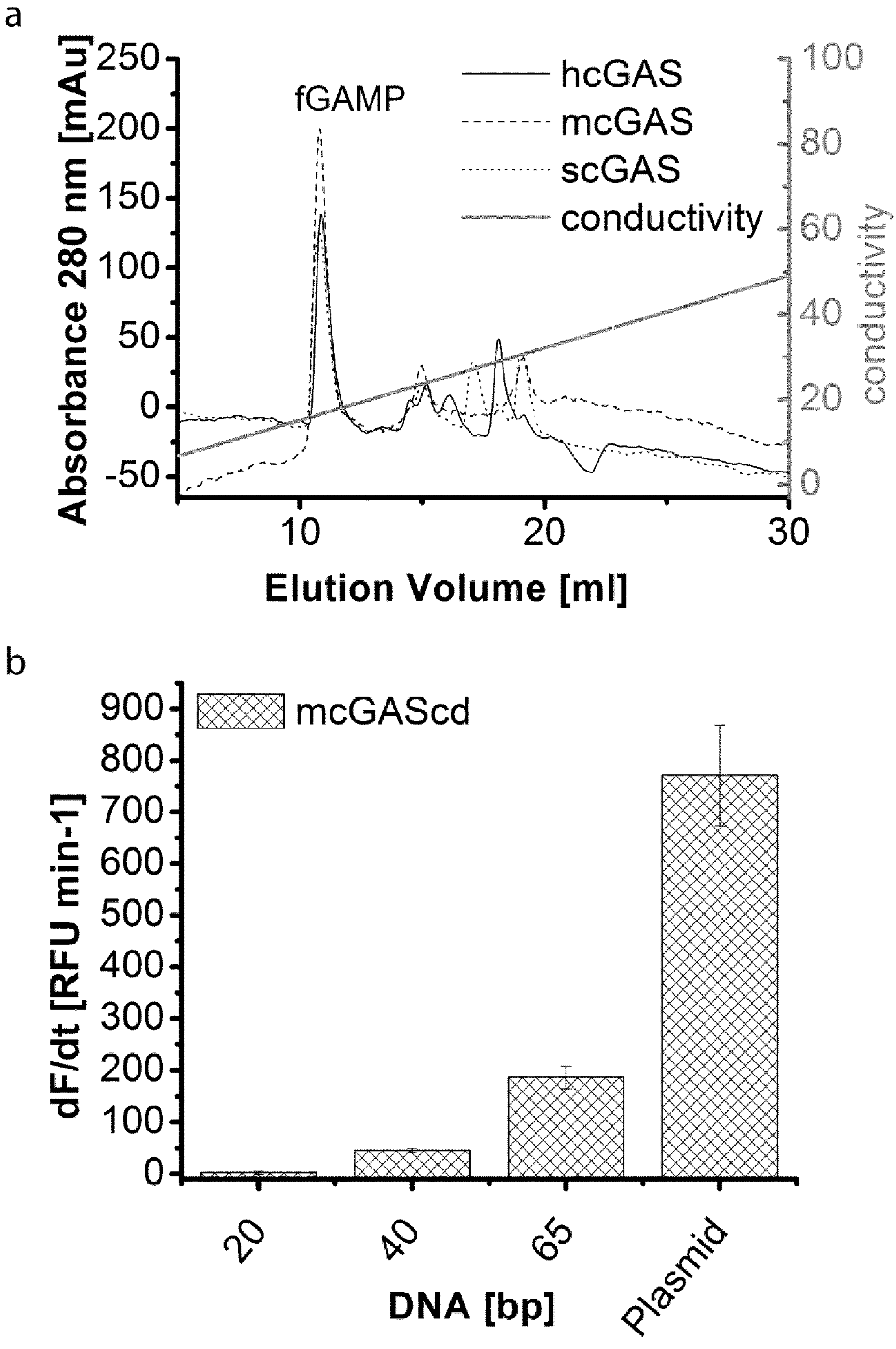
a
fGAMP
hcGAS
mcGAS
scGAS
conductivity
Absorbance 280 nm [mAu]
250
200
150
100
50
0
-50
100
80
60
40
20
0
conductivity
10
20
30
Elution Volume [ml]
b
mcGAScd
dF/dt [RFU min-1]
900
800
700
600
500
400
300
200
100
0
20
40
65
Plasmid
DNA [bp]

Figure 8_ab

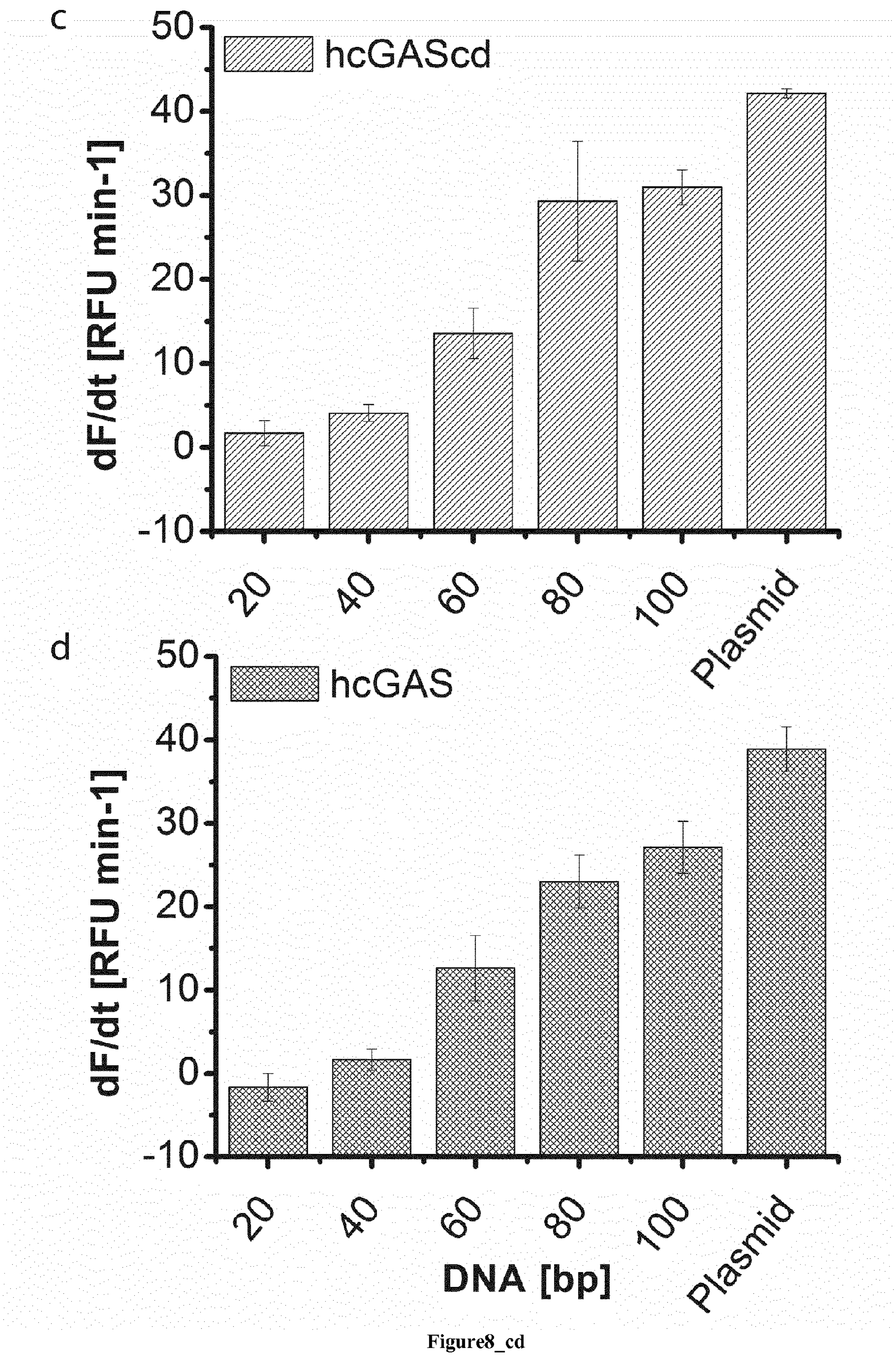
c
hcGAScd
dF/dt [RFU min-1]
50
40
30
20
10
0
-10
20
40
60
80
100
Plasmid
d
hcGAS
dF/dt [RFU min-1]
50
40
30
20
10
0
-10
20
40
60
80
100
Plasmid
DNA [bp]

Figure8_cd

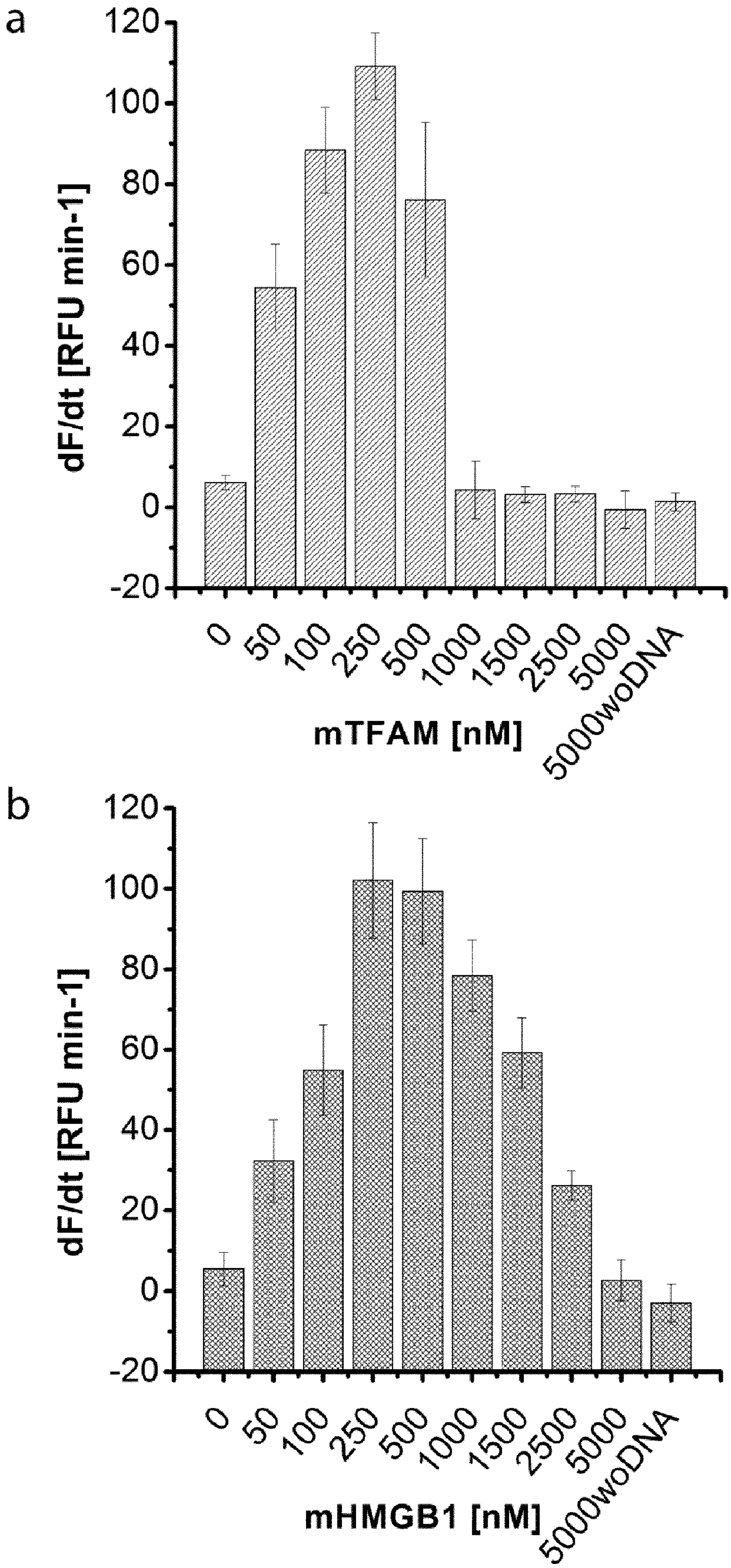
a
120
100
80
60
40
20
0
-20
dF/dt [RFU min-1]
0
50
100
250
500
1000
1500
2500
5000
5000woDNA
mTFAM [nM]
b
120
100
80
60
40
20
0
-20
dF/dt [RFU min-1]
0
50
100
250
500
1000
1500
2500
5000
5000woDNA
mHMGB1 [nM]

Figure 9ab

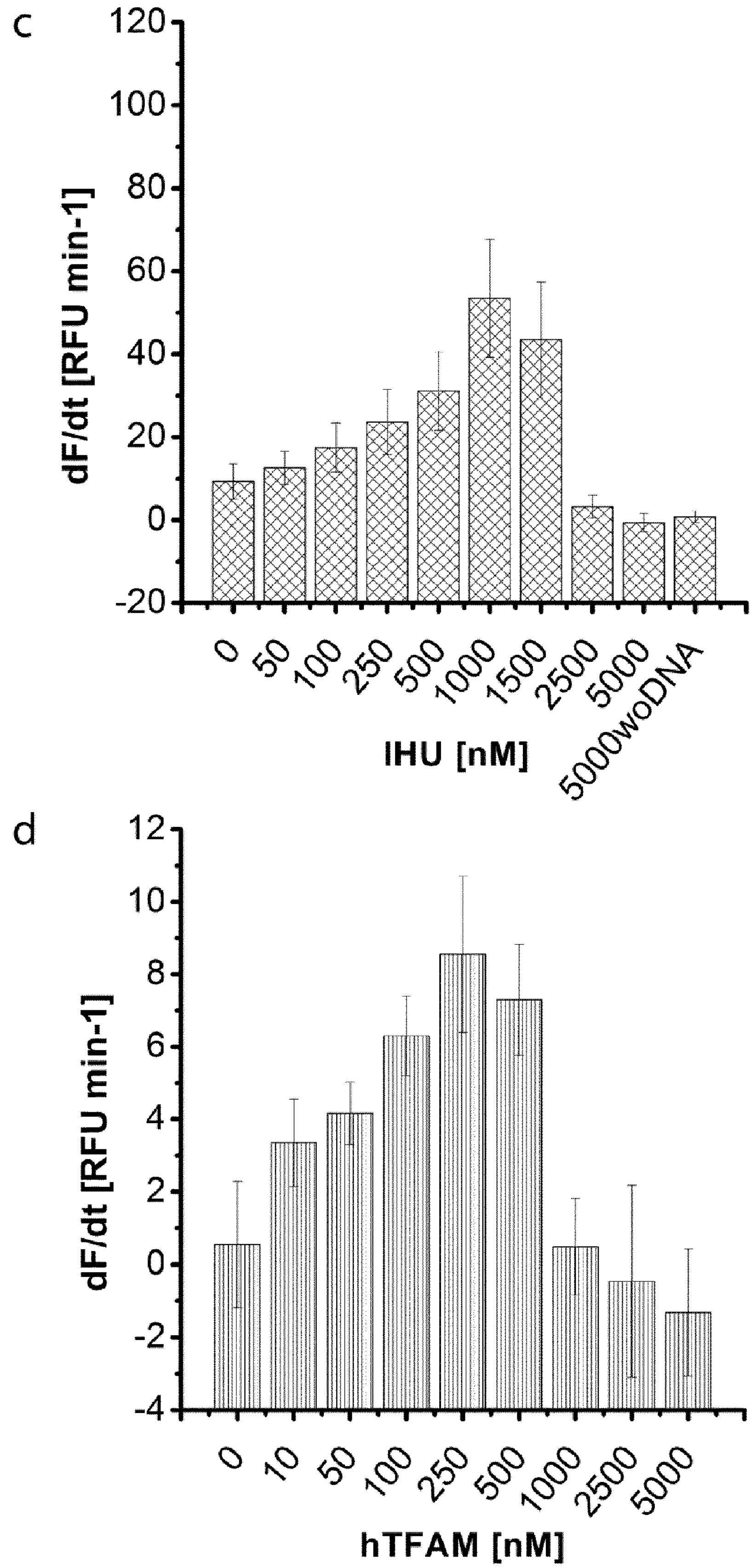
c
dF/dt [RFU min-1]
120
100
80
60
40
20
0
-20
0
50
100
250
500
1000
1500
2500
5000
5000woDNA
IHU [nM]
d
dF/dt [RFU min-1]
12
10
8
6
4
2
0
-2
-4
0
10
50
100
250
500
1000
2500
5000
hTFAM [nM]

Figure9cd

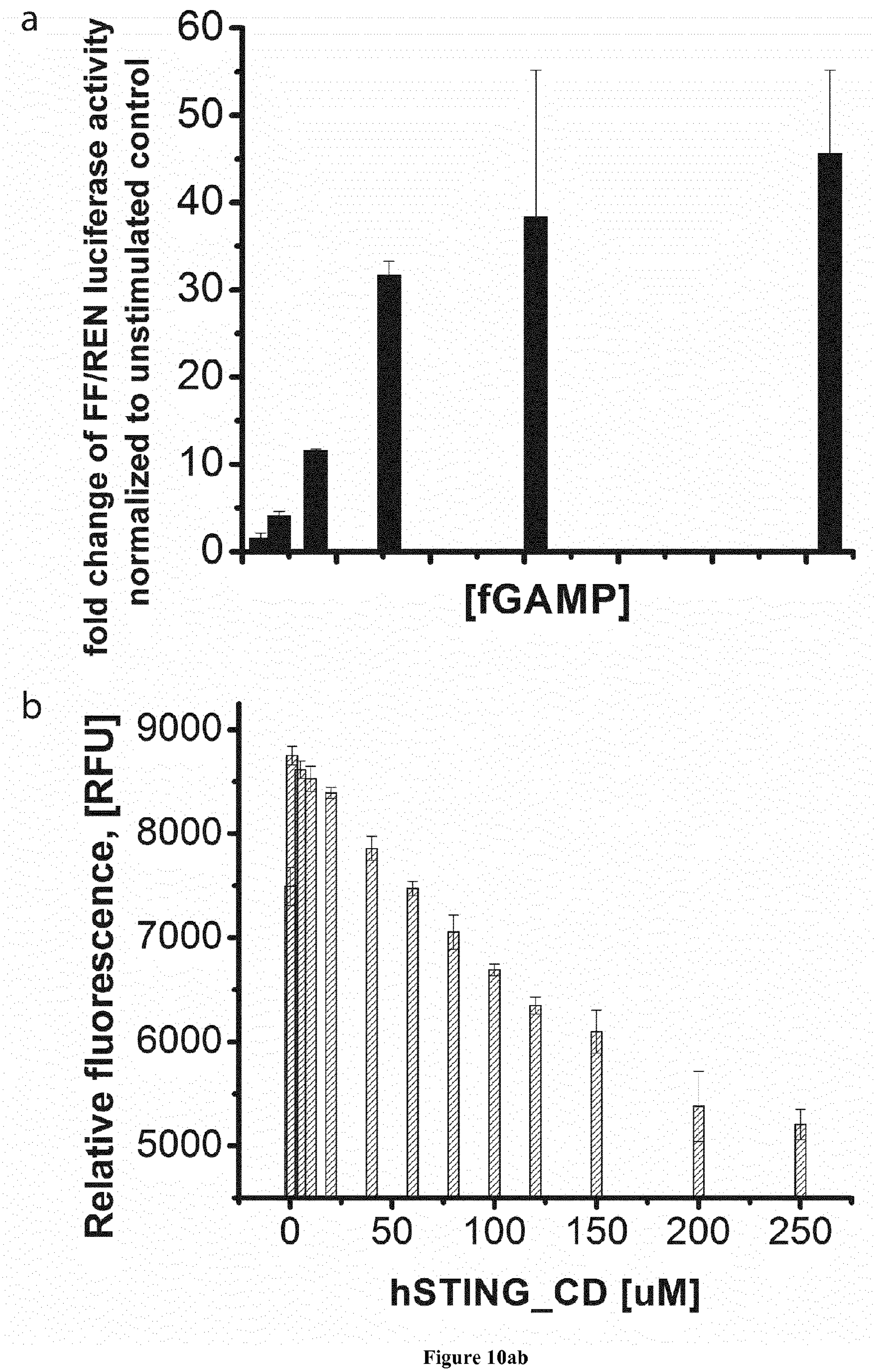
a
60
50
40
30
20
10
0
fold change of FF/REN luciferase activity normalized to unstimulated control
[fGAMP]
b
9000
8000
7000
6000
5000
Relative fluorescence, [RFU]
0
50
100
150
200
250
hSTING_CD [uM]

Figure 10ab

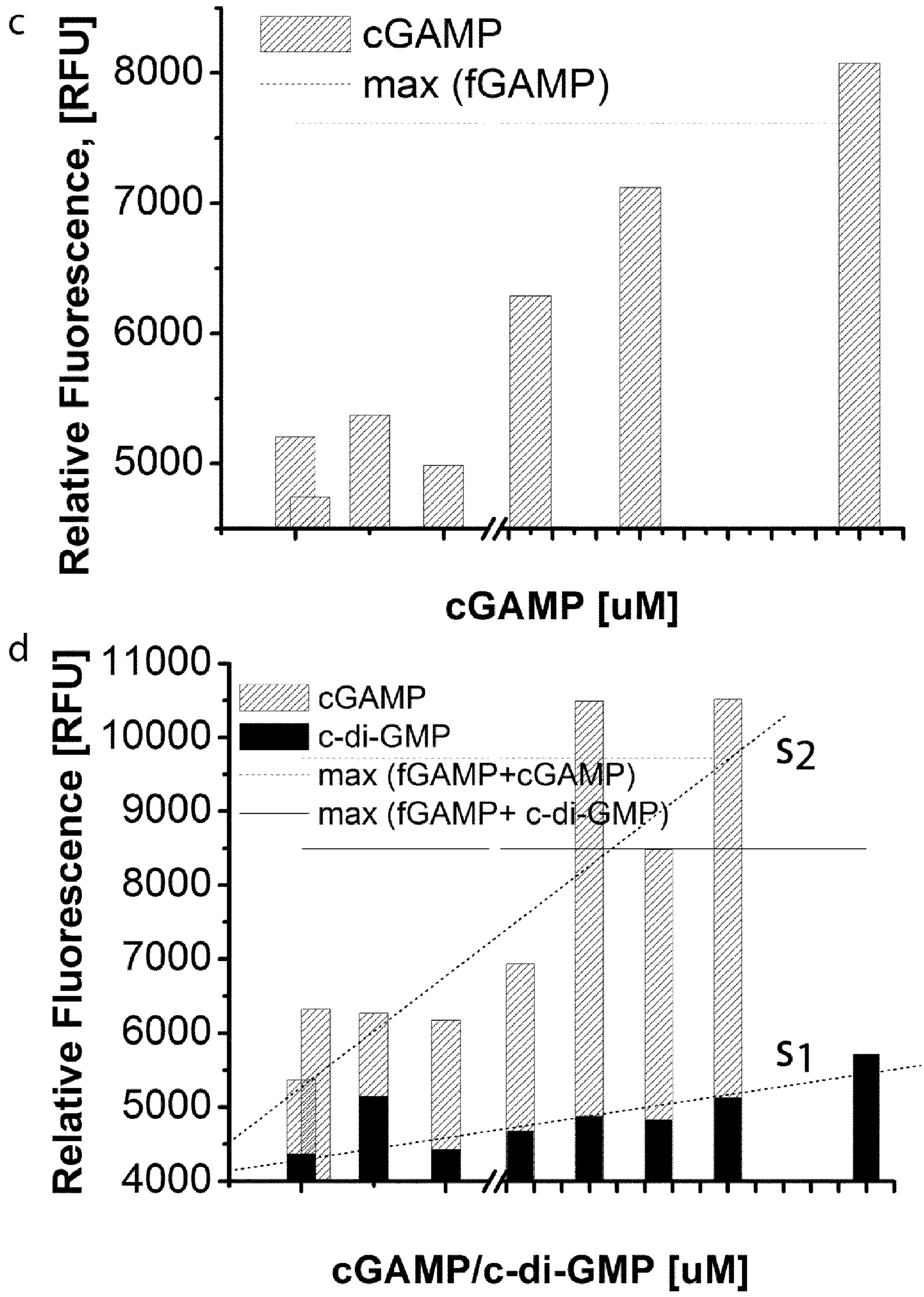
c
cGAMP
max (fGAMP)
Relative Fluorescence, [RFU]
8000
7000
6000
5000
cGAMP [uM]
d
cGAMP
c-di-GMP
max (fGAMP+cGAMP)
max (fGAMP+ c-di-GMP)
S2
S1
Relative Fluorescence [RFU]
11000
10000
9000
8000
7000
6000
5000
4000
cGAMP/c-di-GMP [uM]

Figure10_cd

Europäisches Patentamt
European Patent Office
Office européen des brevets

# EUROPEAN SEARCH REPORT

Application Number
EP 17 18 2689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/027646 A1 (MERCK SHARP & DOHME CORP., USA) 16 February 2017 (2017-02-16)<br>* the whole document in particular the examples *<br>----- | 1-7,11, 20-22 | INV.<br>C07H21/02<br>C07H21/04<br>G01N33/52 |
| X | WO 2017/093933 A1 (GLAXOSMITHKLINE IP DEV LTD [GB]) 8 June 2017 (2017-06-08)<br>* the whole document in particular the examples *<br>----- | 1-3, 20-22 | |
| X | WO 2015/077354 A1 (UNIV CHICAGO [US]) 28 May 2015 (2015-05-28)<br>* the whole document in particular claim 23 and the examples *<br>----- | 1-3, 20-22 | |
| X | WO 2014/189805 A1 (AURO BIOTECH, INC., USA; UNIVERSITY OF CALIFORNIA) 27 November 2014 (2014-11-27)<br>* the whole document in particular Figures 4-6 *<br>----- | 1-3, 20-22 | TECHNICAL FIELDS SEARCHED (IPC) |
| X | WO 2017/027645 A1 (MERCK SHARP & DOHME [US]; ALTMAN MICHAEL D [US]; ANDRESEN BRIAN [US];) 16 February 2017 (2017-02-16)<br>* the whole document in particular the examples *<br>----- | 1-3, 20-22 | C07H<br>G01N |
| X | WO 2017/075477 A1 (ADURO BIOTECH INC [US]; NOVARTIS AG [CH]) 4 May 2017 (2017-05-04)<br>* the whole document *<br>-----<br>-/-- | 1-3, 20-22 | |

~~The present search report has been drawn up for all claims~~

| Place of search | Date of completion of the search | Examiner |
|---|---|---|
| Munich | 11 April 2018 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 17 18 2689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HIROAKI SAWAI ET AL: "Preparation and properties of oligocytidylates with 2'-5' internucleotide linkage.", BULLETIN OF THE CHEMICAL SOCIETY OF JAPAN, vol. 58, no. 1, 1 January 1985 (1985-01-01), pages 361-366, XP055293995, JP ISSN: 0009-2673, DOI: 10.1246/bcsj.58.361 * table 1 * ----- | 1,2 | |
| X | HIROAKI SAWAI ET AL: "Synthesis of 2'-5' Linked Oligouridylates in Aqueous Medium using the Pd2+ Ion", CHEM. PHARM.BULL, vol. 29, no. 8, 1 January 1981 (1981-01-01), pages 2237-2245, XP055322566, * table 1 * ----- | 1,2 | |
| T | D. ONIDAS ET AL: "Fluorescence Properties of DNA Nucleosides and Nucleotides: A Refined Steady-State and Femtosecond Investigation", THE JOURNAL OF PHYSICAL CHEMISTRY B, vol. 106, no. 43, 1 October 2002 (2002-10-01), pages 11367-11374, XP55068275, ISSN: 1520-6106, DOI: 10.1021/jp026063g * the whole document * ----- | | TECHNICAL FIELDS SEARCHED (IPC) |

-/--

~~The present search report has been drawn up for all claims~~

| Place of search | Date of completion of the search | Examiner |
|---|---|---|
| Munich | 11 April 2018 | Klein, Didier |

5

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 18 2689

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | BENJAMIN T. ROEMBKE ET AL: "A cyclic dinucleotide containing 2-aminopurine is a general fluorescent sensor for c-di-GMP and 3',3'-cGAMP", MOLECULAR BIOSYSTEMS, vol. 10, no. 6, 1 January 2014 (2014-01-01), page 1568, XP055172186, ISSN: 1742-206X, DOI: 10.1039/c3mb70518h<br>* the whole document *<br>----- | 12,14-18 | |
| A | US 2013/039933 A1 (BARBER GLEN N [US]) 14 February 2013 (2013-02-14)<br>* the whole document *<br>----- | 12,14-18 | |
| | | | TECHNICAL FIELDS SEARCHED (IPC) |

~~The present search report has been drawn up for all claims~~

5

| Place of search | Date of completion of the search | Examiner |
|---|---|---|
| Munich | 11 April 2018 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 17 18 2689

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [x] Only part of the further search fees have been paid within the fixed time limit. The present European search report has been drawn up for those parts of the European patent application which relate to the inventions in respect of which search fees have been paid, namely claims:

  12, 15-17(completely); 14, 18(partially)

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 17 18 2689

The Search Division considers that the present European patent application does not comply with the requirements of unity of invention and relates to several inventions or groups of inventions, namely:

1. claims: 1-7, 10, 20-22(completely); 11(partially)

   Compounds comprising a 2'-5' and 3'-5'-dinucleotide wherein at least one of the nucleotide is fluorescent and uses thereof.

   ---

2. claims: 8, 9, 19(completely); 11(partially)

   A method of measuring CGAS activity

   ---

3. claims: 12(completely); 14-18(partially)

   A method of identifying a substance having the ability to bind to stimulators of STING

   ---

4. claims: 13(completely); 14-18(partially)

   A method of identifying a substance having the ability to modulate the activity of 2',5'-phosphodiesterase

   ---

## ANNEX TO THE EUROPEAN SEARCH REPORT ON EUROPEAN PATENT APPLICATION NO. EP 17 18 2689

This annex lists the patent family members relating to the patent documents cited in the above-mentioned European search report. The members are as contained in the European Patent Office EDP file on
The European Patent Office is in no way liable for these particulars which are merely given for the purpose of information.

11-04-2018

| Patent document cited in search report | Publication date | Patent family member(s) | Publication date |
|---|---|---|---|
| WO 2017027646 A1 | 16-02-2017 | AU 2016304899 A1 | 01-02-2018 |
| | | CA 2995365 A1 | 16-02-2017 |
| | | TW 201718619 A | 01-06-2017 |
| | | US 2017044206 A1 | 16-02-2017 |
| | | WO 2017027645 A1 | 16-02-2017 |
| | | WO 2017027646 A1 | 16-02-2017 |
| WO 2017093933 A1 | 08-06-2017 | AU 2016362697 A1 | 04-01-2018 |
| | | CN 107849084 A | 27-03-2018 |
| | | KR 20180009812 A | 29-01-2018 |
| | | TW 201731862 A | 16-09-2017 |
| | | US 2017158724 A1 | 08-06-2017 |
| | | US 2017233430 A1 | 17-08-2017 |
| | | UY 37007 A | 30-06-2017 |
| | | WO 2017093933 A1 | 08-06-2017 |
| WO 2015077354 A1 | 28-05-2015 | EP 3071209 A1 | 28-09-2016 |
| | | JP 2016538344 A | 08-12-2016 |
| | | US 2016287623 A1 | 06-10-2016 |
| | | US 2018028553 A1 | 01-02-2018 |
| | | WO 2015077354 A1 | 28-05-2015 |
| WO 2014189805 A1 | 27-11-2014 | AU 2014268836 A1 | 24-09-2015 |
| | | CA 2904536 A1 | 27-11-2014 |
| | | CL 2015002522 A1 | 30-12-2016 |
| | | CN 105228450 A | 06-01-2016 |
| | | CR 20150616 A | 19-04-2016 |
| | | CU 20150158 A7 | 30-05-2016 |
| | | DO P2015000281 A | 29-02-2016 |
| | | EP 2996473 A1 | 23-03-2016 |
| | | HK 1219024 A1 | 24-03-2017 |
| | | HK 1222512 A1 | 07-07-2017 |
| | | JP 2016520085 A | 11-07-2016 |
| | | KR 20160009039 A | 25-01-2016 |
| | | PE 00802016 A1 | 21-02-2016 |
| | | PH 12015502438 A1 | 28-03-2016 |
| | | SG 11201508273R A | 30-12-2015 |
| | | US 2015056224 A1 | 26-02-2015 |
| | | US 2017333552 A1 | 23-11-2017 |
| | | WO 2014189805 A1 | 27-11-2014 |
| WO 2017027645 A1 | 16-02-2017 | AU 2016304899 A1 | 01-02-2018 |
| | | CA 2995365 A1 | 16-02-2017 |
| | | TW 201718619 A | 01-06-2017 |
| | | US 2017044206 A1 | 16-02-2017 |
| | | WO 2017027645 A1 | 16-02-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 17 18 2689

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2018

| Patent document cited in search report | Publication date | Patent family member(s) | Publication date |
|---|---|---|---|
| | | WO 2017027646 A1 | 16-02-2017 |
| WO 2017075477 A1 | 04-05-2017 | TW 201726700 A | 01-08-2017 |
| | | UY 36969 A | 31-05-2017 |
| | | WO 2017075477 A1 | 04-05-2017 |
| US 2013039933 A1 | 14-02-2013 | US 2013039933 A1 | 14-02-2013 |
| | | US 2018085432 A1 | 29-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CROW, HAYWARD et al.** Mutations in the gene encoding the 3[prime]-5[prime] DNA exonuclease TREX1 cause Aicardi-Goutieres syndrome at the AGS1 locus. *Nat Genet,* 2006, vol. 38, 917-920
- **LEE-KIRSCH, GONG et al.** Mutations in the gene encoding the 3[prime]-5[prime] DNA exonuclease TREX1 are associated with systemic lupus erythematosus. *Nat Genet,* 2007, vol. 39, 1065-1067
- **GALL, TREUTING et al.** Autoimmunity Initiates in Nonhematopoietic Cells and Progresses via Lymphocytes in an Interferon-Dependent Autoimmune Disease. *Immunity,* 2012, vol. 36, 120-131
- **AHN, RUIZ et al.** Intrinsic Self-DNA Triggers Inflammatory Disease Dependent on STING. *The Journal of Immunology,* 2014
- **CROW, CHASE et al.** Characterization of Human Disease Phenotypes Associated with Mutations in TREX1, RNASEH2A, RNASEH2B, RNASEH2C, SAMHD1, ADAR, and IFIH1. *American journal of medical genetics,* 2015, 296-312
- **GAO, LI et al.** Activation of cyclic GMP-AMP synthase by self-DNA causes autoimmune diseases. *Proceedings of the National Academy of Sciences,* 2015, vol. 112, E5699-E5705
- **NOWARSKI, GAGLIANI et al.** Innate Immune Cells in Inflammation and Cancer. *Cancer Immunology Research,* 2013, vol. 1, 77-84
- **WOO, FUERTES et al.** STING-Dependent Cytosolic DNA Sensing Mediates Innate Immune Recognition of Immunogenic Tumors. *Immunity,* 2014, vol. 41, 830-842
- **ROEMBKE, ZHOU et al.** A cyclic dinucleotide containing 2-aminopurine is a general fluorescent sensor for c-di-GMP and 3',3'-cGAMP. *Molecular BioSystems,* 2014, vol. 10, 1568-1575
- **ABLASSER, GOLDECK et al.** cGAS produces a 2[prime]-5[prime]-linked cyclic dinucleotide second messenger that activates STING. *Nature,* 2013, vol. 498, 380-384
- **ABLASSER, SCHMID-BURGK et al.** Cell intrinsic immunity spreads to bystander cells via the intercellular transfer of cGAMP. *Nature,* 2013, vol. 503, 530-534
- **CROW, CHASE et al.** Characterization of Human Disease Phenotypes Associated with Mutations in TREX1, RNASEH2A, RNASEH2B, RNASEH2C, SAMHD1, ADAR, and IFIH1. *American journal of medical genetics,* 296-312
- **LI, SHU et al.** Cyclic GMP-AMP Synthase Is Activated by Double-Stranded DNA-Induced Oligomerization. *Immunity,* vol. 39, 1019-1031
- **RUSSELL.** Molecular Cloning, a laboratory manual. Cold Spring Harbor Laboratory Press, 2012, vol. 1
- **HUGHES, MIKLOS et al.** Gene Synthesis: Methods and Applications. *Methods in Enzymology,* 2011, vol. 498, 277-309
- Recombinational Cloning Using Gateway and In-Fusion Cloning Schemes. **THROOP ; LABAER.** Current protocols in molecular biology. 2015, vol. 110, 3.20.21-23.20.23
- **LAKOWICZ.** Principles of Fluorescence Spectroscopy. Springer, 2006
- **GAO, ASCANO et al.** Structure-Function Analysis of STING Activation by c[G(2',5')pA(3',5')p] and Targeting by Antiviral DMXAA. *Cell,* 2013, vol. 154, 748-762
- **GAO, ZILLINGER et al.** Binding-Pocket and Lid-Region Substitutions Render Human STING Sensitive to the Species-Specific Drug DMXAA. *Cell Reports,* 2014, vol. 8, 1668-1676
- **TANG ; WANG.** Single Amino Acid Change in STING Leads to Constitutive Active Signaling. *PLOS ONE,* 2015, vol. 10, e0120090
- *Expert Opin Drug Discov.,* 2015, vol. 10 (11), 1145-61
- **MACKENZIE, HUESA et al.** New insights into NPP1 function: Lessons from clinical and animal studies. *Bone,* 2012, vol. 51, 961-968
- **LI, YIN et al.** Hydrolysis of 2'3'-cGAMP by ENPP1 and design of nonhydrolyzable analogs. *Nat Chem Biol,* 2014, vol. 10, 1043-1048
- **SORTICA, BUFFON et al.** Association between the ENPP1 K121Q Polymorphism and Risk of Diabetic Kidney Disease: A Systematic Review and Meta-Analysis. *PLOS ONE,* 2015, vol. 10, e0118416
- **DEY, DEY et al.** Inhibition of innate immune cytosolic surveillance by an M. tuberculosis phosphodiesterase. *Nat Chem Biol,* 2017, vol. 13, 210-217
- **RAPINO, ROBLES et al.** C/EBP$\alpha$ Induces Highly Efficient Macrophage Transdifferentiation of B Lymphoma and Leukemia Cell Lines and Impairs Their Tumorigenicity. *Cell Reports,* 2013, vol. 3, 1153-1163

- **M. GAIDT.** Gene Center. Ludwig-Maximilians-Universitat München, Center for Integrated Protein Science Munich
- **CIVRIL, DEIMLING et al.** Structural mechanism of cytosolic DNA sensing by cGAS. *Nature,* 2013, vol. 498, 332-337
- **CAVLAR, DEIMLING et al.** Species-specific detection of the antiviral small-molecule compound CMA by STING. *The EMBO Jourhal,* 2013, vol. 32, 1440-1450
- **GAIDT, EBERT et al.** Human Monocytes Engage an Alternative Inflammasome Pathway. *Immunity,* 2016, vol. 44, 833-846
- **GAO, ASCANO et al.** Cyclic [G(2',5')pA(3',5')p] Is the Metazoan Second Messenger Produced by DNA-Activated Cyclic GMP-AMP Synthase. *Cell,* 2013, vol. 153, 1094-1107
- **GOLDECK et al.** cGAS produces a 2[prime]-5[prime]-linked cyclic dinucleotide second messenger that activates STING. *Nature,* 2013, vol. 498, 380-384
- **ABLASSER, A., M. GOLDECK et al.** cGAS produces a 2[prime]-5[prime]-linked cyclic dinucleotide second messenger that activates STING. *Nature,* 2013, vol. 498 (7454), 380-384
- **ABLASSER, A. ; J. L. SCHMID-BURGK et al.** Cell intrinsic immunity spreads to bystander cells via the intercellular transfer of cGAMP. *Nature,* 2013, vol. 503 (7477), 530-534
- **AHN, J. ; P. RUIZ et al.** Intrinsic Self-DNA Triggers Inflammatory Disease Dependent on STING. *The Journal of Immunology,* 2014
- **CAVLAR, T. ; T. DEIMLING et al.** Species-specific detection of the antiviral small□molecule compound CMA by STING. *The EMBO Journal,* 2013, vol. 32 (10), 1440-1450
- **CIVRIL, F. ; T. DEIMLING et al.** Structural mechanism of cytosolic DNA sensing by cGAS. *Nature,* 2013, vol. 498 (7454), 332-337
- **CROW, Y. J. ; D. S. CHASE et al.** Characterization of Human Disease Phenotypes Associated with Mutations in TREX1, RNASEH2A, RNASEH2B, RNASEH2C, SAMHD1, ADAR, and IFIH1. *American journal of medical genetics,* 2015, vol. 0 (2), 296-312
- **CROW, Y. J. ; B. E. HAYWARD et al.** Mutations in the gene encoding the 3[prime]-5[prime] DNA exonuclease TREX1 cause Aicardi-Goutieres syndrome at the AGS1 locus. *Nat Genet,* 2006, vol. 38 (8), 917-920
- **DEY, R. J. ; B. DEY et al.** Inhibition of innate immune cytosolic surveillance by an M. tuberculosis phosphodiesterase. *Nat Chem Biol,* 2017, vol. 13 (2), 210-217
- **GAIDT, M. M. ; T. S. EBERT et al.** Human Monocytes Engage an Alternative Inflammasome Pathway. *Immunity,* 2016, vol. 44 (4), 833-846
- **GALL, A. ; P. TREUTING et al.** Autoimmunity Initiates in Nonhematopoietic Cells and Progresses via Lymphocytes in an Interferon-Dependent Autoimmune Disease. *Immunity,* 2012, vol. 36 (1), 120-131
- **GAO, D. ; T. LI et al.** Activation of cyclic GMP-AMP synthase by self-DNA causes autoimmune diseases. *Proceedings of the National Academy of Sciences,* 2015, vol. 112 (42), E5699-E5705
- **GAO, P. ; M. ASCANO et al.** Structure-Function Analysis of STING Activation by c[G(2',5')pA(3',5')p] and Targeting by Antiviral DMXAA. *Cell,* 2013, vol. 154 (4), 748-762
- **GAO, P. ; T. ZILLINGER et al.** Binding-Pocket and Lid-Region Substitutions Render Human STING Sensitive to the Species-Specific Drug DMXAA. *Cell Reports,* 2014, vol. 8 (6), 1668-1676
- **HUGHES, R. A. ; A. E. MIKLOS et al.** Gene Synthesis: Methods and Applications. Methods in Enzymology. Academic Press, 2011, vol. 498, 277-309
- **LAKOWICZ, J. R.** Principles of Fluorescence Spectroscopy. Springer, 2006
- **LEE-KIRSCH, M. A. ; M. GONG et al.** Mutations in the gene encoding the 3[prime]-5[prime] DNA exonuclease TREX1 are associated with systemic lupus erythematosus. *Nat Genet,* 2007, vol. 39 (9), 1065-1067
- **LI, L. ; Q. YIN et al.** Hydrolysis of 2'3'-cGAMP by ENPP1 and design of nonhydrolyzable analogs. *Nat Chem Biol,* 2014, vol. 10 (12), 1043-1048
- **LI, X. ; C. SHU et al.** Cyclic GMP-AMP Synthase Is Activated by Double-Stranded DNA-Induced Oligomerization. *Immunity,* vol. 39 (6), 1019-1031
- **MACKENZIE, N. C. W. ; C. HUESA et al.** New insights into NPP1 function: Lessons from clinical and animal studies. *Bone,* 2012, vol. 51 (5), 961-968
- **NOWARSKI, R. ; N. GAGLIANI et al.** Innate Immune Cells in Inflammation and Cancer. *Cancer Immunology Research,* 2013, vol. 1 (2), 77-84
- **RAPINO, F. ; ELOY F. ROBLES et al.** C/EBPα Induces Highly Efficient Macrophage Transdifferentiation of B Lymphoma and Leukemia Cell Lines and Impairs Their Tumorigenicity. *Cell Reports,* 2013, vol. 3 (4), 1153-1163
- **ROEMBKE, B. T. ; J. ZHOU et al.** A cyclic dinucleotide containing 2-aminopurine is a general fluorescent sensor for c-di-GMP and 3',3'-cGAMP. *Molecular BioSystems,* 2014, vol. 10 (6), 1568-1575
- **RUSSELL, J. F. S. A. D. W.** Molecular Cloning, a laboratory manual. Cold Spring Harbor Laboratory Press, 2012, vol. 1
- **SORTICA, D. A. ; M. P. BUFFON et al.** Association between the ENPP1 K121Q Polymorphism and Risk of Diabetic Kidney Disease: A Systematic Review and Meta-Analysis. *PLOS ONE,* 2015, vol. 10 (3), e0118416

- **TANG, E. D. ; C.-Y. WANG.** Single Amino Acid Change in STING Leads to Constitutive Active Signaling. *PLOS ONE,* 2015, vol. 10 (3), e0120090
- Recombinational Cloning Using Gateway and In-Fusion Cloning Schemes. **THROOP, A. L. ; J. LABAER.** Current protocols in molecular biology. 2015, vol. 110, 3.20.21-23.20.23
- **WOO, S.-R. ; MERCEDES B. FUERTES et al.** STING-Dependent Cytosolic DNA Sensing Mediates Innate Immune Recognition of Immunogenic Tumors. *Immunity,* 2014, vol. 41 (5), 830-842